(19) Europäisches Patentamt
European Patent Office
Office européen des brevets

(11) EP 2 674 168 A1

(12) # EUROPEAN PATENT APPLICATION

(43) Date of publication:
**18.12.2013 Bulletin 2013/51**

(51) Int Cl.:
***A61K 39/00*** *(2006.01)*

(21) Application number: **12075059.1**

(22) Date of filing: **14.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **PLS-Design GmbH**
  **20255 Hamburg (DE)**
- **Klinikum rechts der Isar der Technischen Universität München**
  **81675 München (DE)**
- **Helmholtz Zentrum München Deutsches Forschungszentrum für Gesundheit und Umwelt GmbH**
  **85764 Neuherberg (DE)**

(72) Inventors:
- **Bredehorst, Reinhard**
  **20255 Hamburg (DE)**
- **Grunwald, Thomas**
  **22459 Hamburg (DE)**
- **Ollert, Markus**
  **82131 Gauting (DE)**
- **Schmidt-Weber, Carsten**
  **81249 München (DE)**
- **Spillner, Edzard**
  **22767 Hamburg (DE)**

(74) Representative: **Jungblut, Bernhard Jakob et al**
**Jungblut & Seuss**
**Patentanwälte**
**Max-Dohrn-Strasse 10**
**10589 Berlin (DE)**

(54) **Modulation of effector T cell responses by local depletion of complement component C3**

(57) The invention relates to a pharmaceutical composition for the modulation of effector T cell responses made of one or more preparations and comprising a therapeutically effective dose of at least one recombinant human C3-derivative and of at least one antigen or allergen.



Printed by Jouve, 75001 PARIS (FR)

## Description

The complement system is one of the principal components of both the innate and adaptive immunity. In addition to functioning regulatory and as an effector arm of antibody-initiated processes, complement has a central role in the induction and regulation of CD4+ and CD8+ T cell responses. The modulatory impact of activated complement components on T cell responses is mediated by inducing specific signaling events in the T cell itself and indirectly through alterations of immunomodulatory cells, particularly antigen-presenting cells (APCs) including macrophages, B cells, dendritic cells (DCs) and follicular dendritic cells (FDCs) in lymphoid follicles. These APCs express a remarkable repertoire of complement receptors (CRs) and regulators on their surface and, thereby, are capable to recognize and interact with antigens that have been opsonized by complement. The engagement of CRs and regulators on APCs modulates their maturation status and their chemokine and cytokine expression profiles, which in turn influences the T cell response (for reviews, see Caroll, 2004; Kemper and Atkinson, 2007; Heeger and Kemper, 2012).

*Complement activation.*

The activation of the complement system can be achieved by three different pathways, the classical antibody-dependent activation pathway, the alternative activation pathway and the lectin activation pathway. The central step of the complement cascade resides in the formation of a C3-convertase, which cleaves C3 to C3b and the anaphylatoxin C3a. C3b can act as a part of a C5-convertase, which cleaves C5 in C5b and the anaphylatoxin C5a. In the terminal way, initiated by the generation of C5b, the gradual accumulation of C6, C7, C8 and several molecules C9 result in the formation of the membrane attack complex which is capable of forming a pore in the membrane of the target cells thereby effecting lysis of the cells (for a review, see Dunkelberger and Song, 2010).

*Complement component C3.*

Since complement protein C3 is the central component of all activation pathways, C3 is vital for participation of the complement system in both the innate and adaptive immune responses. C3b is one of the main effector molecules of the complement system and by cleavage of C3b between the amino acid $Cys^{988}$ and $Glu^{991}$ (numbering according to mature protein sequence without signal peptide) a highly reactive thioester is released, which enables C3b to bind to cell surfaces via transacetylation. Furthermore, by the cleavage additional binding sites are exposed which allow C3b to interact with several regulatory and/or complementary proteins comprising binding sites for CR1 (CD35) or Factor H, both co-factors for cleavage by the protease Factor I. Factor I cleaves C3b between $Arg^{1281}$ and $Ser^{1282}$ and $Arg^{1298}$ and $Ser^{1299}$, whereby the fragments C3f and C3bi are generated. C3bi is capable to remain on the surface of pathogens, where it is recognized by CR3 (CD11b/CD18), which is expressed on macrophages and killer cells. Subsequently, CR3 mediates the destruction of pathogens. In conjunction with CR1, Factor I can additionally cleave between amino acids $Arg^{932}$ and $Glu^{933}$, thereby forming C3dg and C3c. C3dg is also capable to remain on the surface and is recognized by CR2 (CD21), which is expressed on B-lymphocytes and DCs (for a review, see Kemper and Atkinson, 2007).

*Local extrahepatic synthesis of C3.*

The liver is the primary source for the synthesis of C3. However, many other specialized cells have the capacity to synthesize C3 including activated macrophages, dendritic cells and epithelial cells (Pratt et al., 2002; Verschoor et al., 2003; Caroll, 2004; Peng et al., 2006; Strainic et al., 2008; Raedler et al., 2009). These cells synthesize C3 spontaneously or in response to cytokine stimulation. Extrahepatic synthesis of C3 is thought to serve important physiological and pathological functions, including regulation of the inflammatory response, host defense, and allograft rejection.

Macrophages secrete all components of the complement system and antigens or inflammatory stimuli activate macrophages to produce substantial amounts of C3 Morgan and Gasque, 1997). In particular, pro-inflammatory cytokines such as IL-6, tumor necrosis factor and interferon-$\gamma$ stimulate macrophages to express the individual components. Macrophage-derived C3 has been shown to locally opsonize various particles and pathogens leading to their more efficient phagocytosis (Ezekowitz et al., 1984). Furthermore, locally produced macrophage-derived C3-fragments can modulate the functions of dendritic cells (DCs). For example, C3b bound to dendritic cell receptors has been demonstrated to increase the capacity of DCs in stimulating allogeneic T cells (Sandor et al., 2009).

Dendritic cells also synthesize and secrete C3 (Peng et al., 2006). However, in the absence of inflammation or 'danger' signals DCs appear to produce only a small amount of C3, which is in accordance with the limited ability of DCs to stimulate an immune response under these conditions. In contrast, in some pathological conditions DC synthesis of C3 may be upregulated by microbial factors and by non-specific inflammatory stimuli. Based on the observation that DC synthesis of C3 is essential for full T cell activation (Peng et al., 2006), DC synthesis of C3 appears to be an important characteristic of DC maturation and up-regulation of DC synthesis of C3 during inflammation and infection could enhance the antigen-presenting capacity of DCs.

*T cell activation by dendritic cells (DCs).*

Among antigen-presenting cells (APCs) including B cells, macrophages and dendritic cells, DCs are the most potent APCs. DCs reside in most peripheral tissues, particularly at the sites of interstitial space and interface with environment. Depending on the activation stimuli in the miroenvironment they can mediate several functions. In addition to activating naive T lymphocytes, DCs are able of promoting tolerogenic responses, regulating T cell-mediated immunity by determining the balance between TH1 and TH2 responses, and developing Treg and TH17 cells (for a review, see Zhou, 2012).

For efficient activation of T cells, DCs have to provide three separate signals including the presentation of antigens, expression of costimulatory molecules and production of cytokines (for reviews, see Banchereau et al., 2000; Shortman and Liu, 2002; Reis e Sousa, 2006). In the absence of inflammation, DCs in the peripheral tissues take up apoptotic cells, process self-antigens and migrate constitutively to the draining lymph node. However, DCs do not express costimulatory molecules without danger signals and therefore, do not activate T cells, but turn T cells into an anergic, nonreactive state. T cell activation is induced through the presentation of antigen by mature APCs in lymph nodes or secondary lymphoid structures to the T cell. Maturation of DCs can be induced by various stimuli including microbial products, immune complexes and inflammatory cytokines (for reviews, see Banchereau et al., 2000; Shortman and Liu, 2002).

The vaccination process involves differentiation of yet uncommitted, naive T cells that mature into effector or regulatory T cells. The differentiation process requires prolonged presence of the antigen between 17h and 40h (Avni et al., 2002), in which the epigenetic configuration of T cell subsets is determined. The nature of antigen in combination with the APC and the microenvironment largely determines which type of T cell will predominate (TH1, TH2, TH17 or regulatory T cells) and whether the activated T cell will then migrate to an inflammatory site. The differentiation process may also involve repetitive stimulation, which is known to sustain the memory phenotype (Calabresi et al., 2003). Since IL-4 may dominate during this process over a commitment to other types of T cells such as TH1 cells (Szabo et al., 1995), inhibition of IL-4 is essential not only for prevention of the TH2 commitment, but also for maintenance of the plasticity towards TH1 or Treg commitment. Therefore, sustained presentation of antigen or allergen and adjuvant along with T cell modulating components appear to be critical for efficient generation of memory T cells in the vaccination process.

*C3-dependent maturation of DCs.*

The important role of complement component C3 in the maturation process of DCs has been documentated in various studies (Reis et al., 2007; Zhou et al., 2007; van Kooten et al., 2008). For example, binding of C3bi-opsonized apoptotic cells to CR3 bearing DCs has been shown to induce the generation of tolerogenic type DCs (Ehirchiou et al., 2007). On the other hand, human C3 deficiency has been demonstrated to be associated with impairments in dendritic cell differentiation, memory B cells, and regulatory T cells (Ghannam et al., 2008). Accordingly, macrophages from C3-deficient mice exibit an impaired potency to stimulate alloreactive T cells (Zhou et al., 2006). C3-deficient mouse kidney, when transplanted into allogeneic wild-type mice, survived for at least 100 days versus 14 days for C3-sufficient mouse kidney (Pratt et al., 2002). These observations are in agreement with the results of a previous study demonstrating that upon reconstitution of C3-deficient mice with bone marrow cells from wild-type mice, the local synthesis of C3 in the spleen and lymph nodes and the ability to make an antibody response to exogenous antigens were restored (Fischer et al., 1998).

Collectively, the studies provide compelling evidence that DCs arising in C3 sufficient environment mature to competent stimulators of T cells, while in the absence of C3 their development is totally abrogated (Ghannam et al., 2008).

*Modulation of DC and macrophage function by C3a and C5a.*

Macrophages express both C3a and C5a receptors (C3aR; C5aR, CD88) and several human dendritic cell subsets (e.g., monocyte derived, dermal, Langerhans, myeloid, plasmacytoid) also express C3aR and/or C5aR. Expression of these G-protein coupled receptors on macophages and DCs suggests that C3a and C5a, which are present in the DC environment, can modulate activation and functions of these cells.

Several studies have demonstrated that C3a and C5a can up-regulate TH1 responses by modulating DC activation and function (for a review, see Zhou, 2012). In the absence of C3a-C3aR interactions, the functions of bone marrow-derived DCs were impaired, resulting in a less activated phenotype with reduced capacity for antigen uptake/ presentation and lowered ability to stimulate TH1 responses (i.e. IL-12 production, generation of IFN-γ producing T cells) *in vitro* and *in vivo.* Accordingly, stimulation of C3aR with C3a increased the capacity of DCs for antigen presentation and enhanced their ability to stimulate the allospecific T cell response (Peng et al., 2008; Li et al., 2008). Similar observations have been made for C5a-C5aR interactions (for a review, see Zhou, 2012).

C3aR signalling has also been associated with the inhibition of TH2 responses by promoting IL-12 (a TH1 driving interleukin) production in DCs. Using a mouse model (BALB/c mice) of allergic inflammation induced by epicutaneous sensitization with ovalbumin (OVA), C3a stimulation has been demonstrated to increase IL-12 production by bone marrow-derived DCs, which in turn mediated *in vitro* inhibition of IL-4 production by OVA-TCR transgenic T cells. Accordingly, in C3aR deficient mice the immune response upon epicutaneous introduction of antigen shifted towards TH2 responses, with significantly higher levels of serum IgG1 and lower levels of serum IgG2a, IgG3, and IgA compared with wild-type mice (Kawamoto et al., 2004). In contrast, deficiency of C5 or C5aR inhibited TH2 immune responses in asthma, thus providing protection to antigen challeged mice (Drouin et al., 2006; Köhl et al., 2006).

C3a and C5a can also up-regulate macrophage function to promote TH1 immune responses (for a review, see Zhou, 2012). For example, macrophages derived from C3 deficient mice (which are unable to generate C3a) had lowered surface expression of MHC class II and reduced ability to elicit allospecific TH1 responses *in vitro* and *in vivo* (Zhou et al., 2006). On the other hand, peritoneal macrophages derived from C5 deficient mice produced significantly less IL-12 following stimulation with IFN-$\gamma$ and Staphylococcus aureus (Karp et al., 2000). In addition to TH1 immune responses, recent evidence suggested that C5a can also promote the development/expansion of TH17 cells by the action on macrophages, thus contributing to the pathogenesis of experimental autoimmune diseases (Fang et al., 2009; Hashimoto et al., 2010).

*Role of C3 for the development of regulatory T cells.*

Regulatory T cells are currently divided into two main subsets (for a review, see Kemper and Atkinson, 2007). Natural CD4+CD25+ regulatory T cells ($T_{reg}$) originate in the thymus, are specific for self-antigens and act predominantly through a contact-dependent mechanism that probably involves granzyme A in humans. These natural $T_{reg}$ cells constitutively express the transcription factor forkhead box P3 (FOXP3) and the IL-2 receptor alpha-chain (CD25) and require exogenous IL-2 for their function and expansion.

Inducible T regulatory 1 ($T_R1$) and $T_H3$ cells are generated in the periphery against both self and foreign antigens. They also require exogenous IL-2 and mediate their suppressive effect primarily by the secretion of IL-10 ($T_R1$ cells) or transforming growth factor-$\beta$ (TGF-$\beta$; $T_H3$ cells). Inducible regulatory T cells might or might not express FOXP3 and show variable expression of CD25.

Several studies have indicated that DCs are able to expand antigen-specific CD4+CD25+ regulatory T cells (Yamazaki et al., 2003; Kretschmer et al., 2005). A recent study demonstrated that $C3^{+/+}$ and $C3^{-/-}$ DCs have different abilities to elicit the development of regulatory T cells (Peng et al., 2006). Co-culturing of CD4+ T cells with allogeneic C3 deficient ($C3^{-/-}$) DCs for 9 days yielded a 4-fold higher level in T cells expressing the *foxp3* gene (used as marker for CD4+CD25+ regulatory T cells; Fontenot et al., 2003) as compared to those stimulated with $C3^{+/+}$ DCs.

In a recent study, activation of complement regulator CD46 (membrane co-factor protein; MCP), which binds to C3b and C4b and functions as a co-factor in their proteolytic degradation by serine protease Factor I, has been shown to drive the switch from classical IFN-$\gamma$-producing CD4+ TH1 effector cells toward IL-10-producing CD4+ TH1 cells with immunosuppressive properties (Cardone et al., 2010). CD46-induced IFN-$\gamma^-$ IL-$10^+$ T cells express high amounts of the TH1 signature transcription factor T-bet, do not require IL-10 for their development and do not express IL-4, IL-5 or Il-17. These characteristics suggest that CD46-induced IFN-$\gamma^-$ IL-$10^+$ T cells are not regulatory type 1 cells, but instead are of Th1 origin (Carsten and Köhl, 2010).

*Role of C3 and C5 in allergy and allergic asthma.*

Complement component C3 has been demonstrated to play an important role in both the TH1 and TH2 response to ovalbumin (OVA) *in vivo* (Yalcindag et al., 2006). In order to investigate the role of C3 in inciting allergic skin inflammation and systemic immune responses after epicutaneous or intraperitoneal sensitization with allergens, C3-deficient ($C3^{-/-}$) mice and wild-type mice were subjected to epicutaneous or intraperitoneal immunization with OVA in alum. Complement component C3 is synthesized by keratinocytes and peritoneal macrophages, both of which are activated by the adjuvant alum. The study revealed that splenocytes from C3-deficient mice secreted less TH2-specific interleukins including IL-4, IL-5 and IL-13, and less TH1-specific IFN-$\gamma$ in response to OVA stimulation than splenocytes from wild-type control mice. C3-deficient mice had impaired IgG1, IgG2a, and IgE antibody responses after both epicutaneous and intraperitoneal immunization. It is important to note that the defect was corrected by the addition of purified C3 protein (Yalcindag et al., 2006).

Complement component C3 also has been demonstrated to play an important role in a murine model of pulmonary allergy by using a mixture of *Aspergillus fumigatus* and OVA (Drouin et al., 2001). In C3 deficient mice protection from airway reduction due to reduced airways responsiveness to inhaled methacholine was observed in this study, as well as reduced levels of IL-4, antigen-specific IgE and IgG, and reduced eosinophil infiltration.

In another study, the role of C3a for eosinophil function in allergic asthma was examined in C3aR deficient mice (Humbles et al., 2000). Using the OVA model of antigen-induced airway hyper-responsiveness, near complete protection from the development of hyper-responsiveness to aerosolized methacholine was observed. The same observation was made in a strain of guinea pigs with a natural C3a-receptor defect (Bautsch et al., 2000). Using an OVA sensitization and challenge model of asthma, significant protection from airway bronchoconstriction following antigen challenge (in contrast to airway hyper-responsiveness to methacholine) was noticed. These data suggest, that at least in the guinea pig lung C3a is a direct-acting spasmogen.

There is also evidence for an important role of C5a in the pathogenesis of allergic asthma. For example, strains of mice naturally deficient of C5 (by virtue of promoter deletions) exhibit enhanced airway responsiveness (Karp et al., 2000). According to the authors, absence of C5a in these strains is responsible for this effect. Since C5a has the ability to effect a TH1 response through IL-12 release from monocytes and macrophages, absence of C5a induces a more vigorous TH2 response. However, it should be noted that a different role of C5a in the pathogenesis of allergic asthma was observed in a rat model (Abe et al., 2001). In this study, a synthetic hexapeptide C5a antagonist as well as inhibition of complement activation by a soluble CR1-derivative blocked the inflammatory response and airway hyper-responsiveness to methacholine. Importantly, these contradictory observations point to the fact that inhibition of complement during the effector phase (study of Abe et al., 2001) may result in a totally different phenotype than inhibition of complement during the sensitization phase (study of Karp et al., 2000). Thus, in mice genetically deficient in C5a, allergen sensitization leads to an enhanced TH2 response due to a lacking TH1-driving effect of C5a, whereas under conditions of an established TH2 response, C5a antagonism has the opposite effect.

Collectively, the data provide substantial evidence that C3, C5 and activated fragments thereof play an important role in the development of allergic responses in animals (for reviews, see Gerard and Gerard, 2002; Hawlisch et al., 2004). However, C3a and C5a are also important for the pathogenesis of allergic asthma in man. A clinical study including 15 patients with asthma demonstrated that both C3a and C5a are specifically released in the challenged asthmatic lungs, and that the inflammatory infiltrate of eosinophils and neutrophils correlates highly with the present amount of anaphylatoxins (Krug et al., 2001).

*Role of C3 and C5 in T cell-mediated autoimmunity.*

Immune cell-derived C3, C3a- and C5a-receptors have been demonstrated to be essential for the development of T cell-dependent autoimmune diabetes (type I diabetes) in mice induced by multiple low doses of streptozotocin (Lin et al., 2010). Coincident with the induced elevations of blood glucose levels, alternative pathway complement component gene expression was upregulated within the islets of the diabetic wild-type animals. Using wild-type chimeras bearing C3-deficient bone marrow cells, the authors demonstrated that bone marrow-derived C3, and not serum C3, is involved in the induction of autoimmune diabetes. C3-deficient mice proved to be completely resistant to the induction of diabetes in this model. An important role of C3aR and C5aR signaling for the development of autoimmune diabetes in this model is documentated by the observation that only 1 of 5 mice deficient in both C3aR and C5aR developed diabetes within 30 days after initiation of treatment with multiple low doses of streptozotocin (Lin et al., 2010).

Activation of C3 plays also an important role in other autoimmune diseases. For example, autoimmune focal and segmental glomerulosclerosis could be induced in mice lacking decay accelerating factor in T cells (Bao et al., 2009). Another study has demonstrated that the interaction of serum-derived C5a with immune cell-expressed C5aR is an essential mediator in an IL-17-dependent model of autoimmune arthritis (Hashimoto et al., 2010). Using a murine model of experimental allergic encephalomyelitis (T cell-mediated autoimmunity that mimics aspects of human multiple sclerosis), $DAF^{-/-}$ mice developed more severe paralysis (associated with enhanced IL-17 production) upon immunization with myelin oligodendrocyte glycoprotein than wild-type mice (Liu et al., 2008). The stronger autoreactive T cell response in $DAF^{-/-}$ mice is C3aR-and C5aR-dependent since mice deficient in either or both of these receptors develop weaker autoreactive T cell responses and are resistant to the development of experimental allergic encephalomyelitis, regardless of DAF expression (Strainic et al., 2008).

Collectively, these data demonstrate that activation of C3 and C5 plays also an important role in the regulation of autoreactive T cells in several murine autoimmune diseases (for a review, see Heeger and Kemper, 2012). Accordingly, complement deficiency or blockade effects attenuation of T cell-mediated autoimmunity (for a review, see Kwan et al., 2012).

*Role of C3 and C5 in alloreactive T cell responses following transplantation.*

A recent study has demonstrated that immune cell-derived and/or donor graft-derived complement components, but not serum complement components, regulate expansion of both alloreactive CD4+ and CD8+ T cells following heart transplantation (Pavlov et al., 2008). Locally produced and activated complement components effect activation of antigen-specific CD4+ T cells. Via CD4+ T cell-mediated CD8+ T cell help, locally produced and activated complement components also effect activation of alloreactive CD8+ T cells and induction of effector CD8+ T cell responses to allogeneic vascular endothelial cells (Raedler et al., 2009). Such responses are enhanced by DAF deficiency as demonstrated by accelerated murine T cell-mediated rejection of $DAF^{-/-}$ heart allografts (Pavlov et al., 2008).

Several observations indicate that C5a is most important for allograft rejection. For example, C5aR blockade prolonged kidney transplant survival in rodents (Gueler et al., 2008). Furthermore, administration of an anti-C5 monoclonal antibody that inhibits C5 cleavage synergized with CTLA4-Ig to prolong heart transplant survival in mice (Raedler et al., 2011). In accordance with these observations, genetic deficiency of C5aR in the donor and the recipient has been demonstrated to limit T cell alloreactivity and, thereby, to prolong kidney transplant survival in mice (Li et al., 2010).

*Therapeutic targeting of locally synthesized C3.*

The various effects of complement component C3, C5 and activated fragments thereof on dendritic cells and macrophages and, thereby, on the activation and differentiation of T cells into TH1, TH2, TH17 or regulatory T cells point to the fact that the complement pathway and, in particular, locally synthesized C3 represents an important therapeutic target in pathological conditions such as allergy, allergic asthma, autoimmune diseases, tissue injury, and transplantation. Therapeutic targeting of locally synthesized C3 appears to the optimal approach for inhibition of local complement activation, since the formation of a C3-convertase represents the central step of the complement cascade and, thereby, affects also the activation of C5.

In principle, therapeutic targeting of C3 may be achieved by inhibition of C3 activation via inactivation of generated C3 convertases. However, at this level at least two different efficient mechanisms of regulation are in effect. CD55 (decay accelerating factor, DAF) regulates complement activation by binding to C3 and C5 convertases, accelerating the decay of the enzyme subunits. CD46 (membrane co-factor protein, MCP) binds either C3b or 4Cb, freshly deposited or left from decayed convertases, enabling the plasma protease factor I to cleave C3b or 4Cb, thereby preventing regeneration of the convertases. Since these mechanisms of regulation apparently are not sufficient under pathological conditions, efficient therapeutic targeting of C3 requires a different approach.

Application of anti-C3 antibodies capable of inhibiting C3 activation represents another approach. However, C3 is the most abundant complement protein and quantitative inhibition of C3 activation or inhibition of C3-derived fragments by specific antibodies does not appear to be a feasable approach.

A fundamentally different approach is inhibition of C3 activation via consumption of C3 prior to medical treatment, i.e. prior to injection of allergen for hyposensitization or prior to transplantation surgery. Consumption of complement component C3 can be achieved by injection of cobra venom factor (CVF), a potent complement activator of the venom of the cobra (for a review, see Vogel and Fritzinger, 2010). CVF shares an identity of 51% and a similarity of 70% on the protein level with human C3. Moreover, both proteins have a chain structure of the same kind. This high similarity is also reflected by the fact that CVF - as C3b - can bind to Factor B and forms a convertase by the Factor D-initiated cleavage of B in Bb and Ba. In contrast to C3Bb, the CVF-dependent convertase CVFBb, however, is a C3- and C5-convertase. By the resistence of CVFBb towards Factor H and Factor I, a convertase is formed with a much higher half-life of 7 h under physiological conditions. In comparison, C3bBb has a half-life of 1.5 min. In addition to the increased stability, the CVF-dependent convertase CVFBb cleaves C3 and C5 also in fluid phase, whereas C3-dependent convertase C3bBb is only active when bound to the cell surface. Due to these characteristics CVF effects a permanent activation of the complement system, leading to the depletion of complement components.

Therapeutic applications of CVF, however, pose serious problems due to its strong immunogenic character. CVF contains complex, N-bound oligosaccharide chains with terminal galactosyl residues, which have an enormous immunogenic potential (Taniguchi et al., 1996). Even deglycosylated CVF is likely to be too immunogenic, especially for repeated applications, given the phylogenetic distance between humans and cobras. Therefore, CVF is not suitable for depletion of complement component C3 in humans.

An additional problem for therapeutic applications of CVF is the fact that locally produced C3 is most important for regulation of effector T cell responses and not C3 in the circulation, particularly under local inflammatory conditions inducing a significant up-regulation of local C3 synthesis. DCs reside in the institial space and access to the interstial space is limited for complement components from the circulation (Farrar et al., 2006). The same is true for systemically applied proteinaceous therapeutics. On the other hand, local injection of proteinaceous therapeutics at the site of inflammation or tissue stress poses the problem that the injected therapeutics may diffuse away from the injection site. Under pathological conditions, however, production and secretion of C3 by DCs and macrophages is an ongoing process. Therefore, effective therapeutic targeting of locally synthesized C3 requires an approach that guarentees the local presence of a particular therapeutic at a concentration and for a period of time sufficient for the purpose of the medical treatment.

In summary, therapeutic targeting of C3 produced and secreted by dendritic cells and macrophages poses serious problems. The lack of suitable complement therapeutics for inhibition of local C3 activation clearly shows that there is a need in the field for an alternative approach capable of intervening with the various functions of locally synthesized C3 and C3-derived fragments at sites of inflammation and tissue stress.

## SUMMARY OF THE INVENTION

The modulatory impact of locally produced and activated complement components on dendritic cells and macrophages and, thereby, on the activation and differentiation of T cells into TH1, TH2, TH17 or regulatory T cells is well documented. Since formation of a C3-convertase represents the central step of the complement cascade, locally synthesized C3 represents an important therapeutic target in pathological conditions such as allergy, allergic asthma, autoimmune diseases, tissue injury, and transplantation. Therapeutic targeting of locally produced and secreted C3, however, poses serious problems due to the lack of suitable complement therapeutics for inhibition of local C3 activation.

This problem is solved by the present invention in that methods for local inhibition of C3 activation via local consumption of C3 are provided leading to the depletion of C3. Thereby, local generation of C3 and C5 convertases is greatly reduced or even negligible, resulting in a significantly decreased concentration of activated C3- and C5-fragments at the site of inflammation and tissue stress, which in turn reduces T cell activation. Furthermore, the present invention discloses methods that allow sustained local presence of C3-depleting therapeutics at a concentration and for a period of time suitable for the purpose of a particular medical treatment within the field of the present invention.

In one embodiment, the present invention discloses recombinant human C3-derivatives (rhC3-derivatives) for controlled depletion of complement component C3, which a) are able to form a physico-chemically stable convertase displaying a relatively long half-life of its decay-dissociation similar to that of the CVF,Bb convertase, b) are resistant to the action of Factor H or the combined action of Factors H and I, c) exert only little to negligible C5-cleaving activity thereby minimizing or eliminateing potential C5a-mediated toxicity, and d) exhibit only low to negligible immunogenicity.

In another embodiment, the present invention discloses methods for sustained local delivery of rhC3-derivates from a depot-mediating matrix for a period of time that is sufficient for the purpose of the particular medical treatment. In a preferred embodiment, matrices are used for local delivery of suitable rhC3-derivatives that a) can serve as depot for substantial quantities of a suitable rhC3-derivative, b) allow the release of sufficient quantities of the embedded rhC3-derivative over a prolonged period of time (optimally for a few days), c) are biodegradable, and d) are chemically and physically compatible with the rhC3-derivative and other compounds necessary for modulation of T cell differentiation and effector T cell responses according to the method of the present invention. Most preferred for a sustained release of recombinant human C3 derivatives are injectable in situ-forming gel systems which are biodegradable.

In another embodiment, the present invention discloses methods for sustained local delivery of both rhC3-derivates and IL-4/IL-13 inhibitors from a depot-mediating matrix for a period of time that is sufficient for the purpose of the particular medical treatment. Most preferred depot-mediating matrices provide the same characteristics as those for sustained delivery of rhC3-derivates.

In another embodiment, the present invention discloses methods for sustained local delivery of rhC3-derivates, IL-4/IL-13 inhibitors and one or more allergens or antigens from a depot-mediating matrix for a period of time that is sufficient for the purpose of the particular medical treatment. Most preferred depot-mediating matrices provide the same characteristics as those for sustained delivery of rhC3-derivates.

In another embodiment, the present invention discloses fields of application for local C3 depletion and local IL-4/IL-13 inhibition as adjuvant therapy for the treatment of T cell-mediated diseases, selected from the group consisting of, but not limited to, allergy, allergic asthma, acute respiratory stress (ARDS), type 1 diabetes, systemic lupus erythemadodes, glomerulonephritis, rheumatoid arthritis, multiple sclerosis, dermatomyositis, nephritis, Parkinson's disease, Alzheimer's disease, pemphigoid, sepsis, myocardial ischemia, acute myocardial infarction (AMI), reperfusion, hemodialysis, paroxysmal nocturnal hemoglobinuria (PNH), age-related macula degeneration (AMD), cardiopulmonary bypass (CPB), angioplasty, hyperacute rejection, transplant rejection, stroke, burns, spinal cord injury, and traumatic brain injury (TBI). Most preferred fields of application include allergy, allergic asthma, and type 1 diabetes.

In another embodiment of the present invention, compositions and methods for local C3-depletion at the site of allergen presentation as adjuvant therapy prior and during specific immunotherapy in patients with type 1 allergy and/or allergic asthma are disclosed.

In another embodiment of the present invention, compositions and methods for local C3-depletion and local inhibition of IL-4/IL-13-mediated effects at the site of allergen presentation as adjuvant therapy prior and during specific immunotherapy in patients with type 1 allergy and/or allergic asthma are disclosed.

In another embodiment of the present invention, compositions and methods for reducing the activation of autoreactive CD8+ cytotoxic T cells by local C3-depletion at the site of self-antigen presentation as adjuvant therapy prior and during self-antigen-specific vaccination in patients with type 1 diabetes are disclosed.

In another embodiment of the present invention, compositions and compositions and methods for reducing the activation of autoreactive CD8+ cytotoxic T cells by local C3-depletion and local inhibition of IL-4-mediated effects, at the site of self-antigen presentation as adjuvant therapy prior and during self-antigen-specific vaccination in patients with type 1 diabetes are disclosed.

In another embodiment of the present invention, methods for systemic anti-CD3 therapy in patients with type I diabetes are combined with methods for reducing the activation of autoreactive CD8+ cytotoxic T cells by local C3-depletion at the site of self-antigen presentation as adjuvant therapy prior and during self-antigen-specific vaccination as described above.

In another embodiment of the present invention, methods for systemic anti-CD3 therapy in patients with type I diabetes are combined with methods for reducing the activation of autoreactive CD8+ cytotoxic T cells by local C3-depletion and local inhibition of IL-4-mediated effects, at the site of self-antigen presentation as adjuvant therapy prior and during self-antigen-specific vaccination as described above.

In another embodiment, the present invention discloses routes of administration of the compositions of the present invention by injection or by implantation, including but are not limited to subcutaneous, intradermal, intramuscular, nasal, transbucal, transmucosal, sublingual, rectal, vaginal, intraocular, or topical administration. Preferred examples of routes of administration of the compositions of the present invention include but are not limited to intradermal, subcutaneous, and intramuscular administration.

In still another embodiment, the present invention discloses methods for incorporating the compositions of the present invention into pharmaceutical compositions suitable for administration.

Specific preferred embodiments of the present invention will become evident from the following more detailed description and the claims.

## BRIEF DESCRIPTION OF THE DRAWINGS

Figure 1 is a schematic representation of the rhC3-derivative H5 encoding a hybrid protein of approx. 91% identity with human C3. A: Chain structures of C3 and pro-CVF. B: Structure of the cDNA of H5. The dark part of the nucleic acid sequence from the 5' end up to the BglII cleavage site represents part of the molecule comprising a C3 nucleic acid sequence. the white part downstream of the BglII site comprises a CVF nucleic acid sequence.

Figure 2 shows a solid phase assay with St-H5 (Strep-tag-fusion construct of rhC3-derivative H5), St-CVF (Strep-tag-fusion construct of CVF), and St-C3 (Strep-tag-fusion construct of human C3). The assay was performed as described in Example 1.4. The Figure shows the mean values ± standard deviation of at least three independent experiments.

Figure 3 shows the complement-consuming activity of CVF, St-H5 (Strep-tag-fusion construct of rhC3-derivative H5), and human C3. The assay was performed as described in Example 1.3. The figure shows the mean values ± standard deviation of at least three independent experiments.

Figure 4 shows the stability of CVF,Bb and rhC3-derivative H5,Bb C3 convertase complexes (CVF,Bb: gray triangles; rhC3-derivative H5,Bb: black squares). Analysis of the stability was performed as described in Example 1.9. Shown are mean values ± s.d. obtained from at least three independent experiments.

Figure 5 shows a schematic representation of the rhC3-derivative H6. **A:** Chain structures of human C3 and pro-CVF. **B:** Structure of rhC3-derivative H6 cDNA.

Figure 6 shows a solid phase-assay with St-H6 (Strep-tag-fusion construct of rhC3-derivative H6), St-CVF (Strep-tag-fusion construct of CVF), and St-C3 (Strep-tag-fusion construct of human C3). The assay was performed as described in Example 1.4. The Figure shows the mean values ± standard deviation of at least three independent experiments.

Figure 7 shows a complement-consumption-assay with CVF, His-tag-fusion construct of rhC3-derivative H6 and human C3. The assay was performed as described in Example 1.3. The figure shows the mean values ± standard deviation of at least three independent experiments. The complement-consuming activity was evaluated by linear regression analysis.

Figure 8 shows the *in vitro* release of a model protein (anti-lysozyme antibody) from gelled polymer at 37°C. The polymers were synthesized according to example 20. Two gels at 25% concentration in PBS pH 7.4 were compared. 1 $\mu$l of the antibody were added to 200 $\mu$l of the gel solutions and incubated at 37°C for gellation. Gels were covered with 1.8 ml PBS and incubated at 37°C. At indicated times samples of the supernatant of the gels were taken and analyzed in ELISA for the amount of released antibody protein binding to lysozyme. The amount of protein released is given as percentage of total release in comparison to a sample with 1 $\mu$l antibody in 2 ml PBS without gel.

## DETAILED DESCRIPTION OF THE INVENTION

The modulatory impact of activated complement components on T cell responses is mediated by inducing specific signaling events in the T cell itself and indirectly through alterations of immunomodulatory cells, particularly antigen-presenting cells (APCs) including macrophages, dendritic cells (DCs) and follicular dendritic cells (FDCs) in lymphoid follicles. The various effects of complement components C3 and C5 (and fragments thereof) on dendritic cells and macrophages and, thereby, on the activation and differentiation of T cells into TH1, TH2, TH17 or regulatory T cells point to the fact that the complement pathway and, in particular, locally synthesized C3 represents an important therapeutic target in pathological conditions such as allergy, allergic asthma, autoimmune diseases, tissue injury, and transplantation. Therapeutic targeting of C3 produced and secreted by dendritic cells and macrophages, however, poses serious problems.

Inhibition of C3 activation by antibody-based therapeutics and depletion of complement component C3 by injection of CVF do not represent feasable approaches. Furthermore, systemically applied proteinaceous therapeutics are not suitable for targeting of locally produced C3, since DCs reside in the interstitial space and access to the interstitial space from circulation is limited. Locally synthesized C3, however, appears to be most important for regulation of effector T cell responses, particularly under local inflammatory conditions inducing a significant up-regulation of local C3 synthesis. Local injection of proteinaceous therapeutics at the site of inflammation or tissue stress poses the problem that the injected therapeutics may diffuse away from the injection site. Production and secretion of C3 by DCs and macrophages under pathological conditions, however, is an ongoing process.

These problems are solved by the present invention in that methods for local inhibition of C3 activation via local consumption of C3 are provided leading to the depletion of C3. Thereby, local generation of C3 and C5 convertases is greatly reduced or even negligible, resulting in a significantly decreased concentration of activated C3- and C5-fragments at the site of inflammation and tissue stress, which in turn reduces T cell activation. Furthermore, the present invention discloses methods that allow sustained local presence of C3-depleting therapeutics at a concentration and for a period of time suitable for the purpose of a particular medical treatment within the field of the present invention.

### I. Depletion of C3 with recombinant human C3-derivatives (rhC3-derivatives)

For therapeutic targeting of C3, the present invention discloses recombinant C3-depleting therapeutics (recombinant human C3-derivatives, rhC3-derivatives). These rhC3-derivatives are also inhibitors of C5 activation, since depletion of C3 inhibits also the formation of C5 convertases and, thereby, generation of C5a.

In the process of C3 depletion with rhC3-derivatives, however, the complement system is activated. While depletion of C3 can be performed slowly, thereby generating only low concentrations of activated complement components including anaphylatoxins, the depletion process should be performed preferentially under non-acute inflammatory conditions. However, even under acute inflammatory conditions (e.g., acute autoimmune diseases) C3 depletion is feasable, provided the depletion is performed in a controlled fashion which avoids enhancement of the inflammatory condition.

As disclosed in US patent 7,553,931, US patent 8,119,769 and in US patent application 2010/0179092 A1, several human C3-derivatives with CVF-like functions have been designed and generated. In contrast to CVF, the human C3-derivatives exhibit only little to negligible immunogenicity which allows repeated therapeutic administrations or sustained delivery of rhC3-derivatives from depot-mediating polymers.

Recombinant human C3-derivatives useful for the present invention provide several important properties. Most important, such derivatives must be able to form a physico-chemically stable convertase displaying a relatively long half-life of its decay-dissociation similar to that of the CVF,Bb convertase. Second, such derivatives must be resistant to the action of Factor H or the combined action of Factors H and I. Furthermore, such derivatives should exert only low to negligible C5-cleaving activity to minimize or eliminate potential C5a-mediated toxicity.

Useful rhC3-derivatives were generated by replacing small stretches of human C3 at the very C-terminus of the C3 $\alpha$-chain with homologous stretches of sequence from the CVF $\beta$-chain which contains the crucial stability site responsible for forming a stable convertase.

Several rhC3-derivatives providing the desired properties have been generated and characterized. For example, rhC3-derivative H5 expressed in CHO and HEK293 cells provides a 90% sequence identity with human complement component C3 and exhibits approx. 85% of the complement-consuming activity of purified native CVF (Kölln et al., 2004; US patent 7,553,931; US patent 8,119,769). The recombinant protein activates Factor B by producing Bb and Ba in the presence of Factor D and $Mg^{2+}$ in an identical manner as C3(H2O) and CVF. Further analyses revealed a half-life of the rhC3-derivative H5-dependent convertase of approx. 5-6 hours, which is close to the reported 7 hour half-life of the CVF-dependent convertase (Vogel und Müller-Eberhard, 1982). The potential immunogenicity of this derivative in humans can be assumed to be very low.

The rhC3-derivative H6 expressed in CHO and HEK293 cells provides a 96% sequence identity with human complement component C3 and still exhibits approximately 70% of the complement activating activity of CVF. The derivative does not exert significant C5-convertase activity (Kölln et al., 2004; Kölln et al., 2005; US patent 7,553,931; US patent 8,119,769). As a result of the high sequence identity with human complement component C3, the potential immunogenicity of this derivative in humans can be assumed to be extremely low or even negligible.

Expression of rhC3-derivative HC3-1496 in Drosophila S2 cells yielded a molecule with comparable properties. HC3-1496, a construct in which the C-terminal 168 amino acid residues of the C3α-chain have been replaced with the corresponding 168 amino acid residues from the β-chain of CVF, forms a convertase which is more stable than CVF, Bb and provides a higher C3-cleaving activity than that of CVF. Furthermore, HC3-1496 exerts no or only residual C5-cleaving activity. Due to its 94% sequence identity with human complement component C3, the potential immunogenicity of this derivative in humans also can be assumed to be very low or even negligible (Vogel and Fritzinger, 2007; Fritzinger et al., 2009, for a review, see Vogel and Fritzinger, 2010; US patent 2010/0179092 A1).

**II. Safety aspects of C3 depletion with rhC3-derivatives**

Since human C3-derivatives exhibit CVF-like functions, studies performed with CVF provide valuable information about safety aspects of C3 depletion with rhC3-derivatives.

As revealed by numerous studies, complement depletion in laboratory animals by injection of CVF appears to be safe, since no adverse side effects have been observed in (for a review, see Vogel and Fritzinger, 2010). Most of these studies involved complement depletion for a few days up to one month. All known effects of CVF are mediated by binding to factor B and the resulting activation of complement. No off-target activity has been observed. Furthermore, transgenic mice constitutively expressing CVF (Andrä et al., 2002) display no apparent abnormal phenotype, exhibit a normal life span, reproduce normally. During approximately one decade of continued maintenance under normal animal housing conditions, no tendency to develop infections has been observed (for a review, see Vogel and Fritzinger, 2010).

The only acute side effect observed after massive intravascular activation of complement by CVF is a C5a-mediated transitory inflammation of the lung due to sequestration of activated neutrophils (Till et al., 1982; Till et al., 1987; Mulligan et al., 1996). However, the occurrence and intensity of this inflammatory lung injury can be easily controlled by the amount and the rate of C5a generation.

In contrast to CVF, however, rhC3-derivatives used for the present invention do not exhibit significant C5-cleaving activity. Accordingly, small to negligible amounts of C5a are generated upon action of complement with such rhC3-derivatives.

In a recent study, the rhC3-derivative HC3-1496 (designed as humanized CVF) in which the C-terminal 168 amino acid residues of the C3α-chain have been replaced with the corresponding 168 amino acid residues from the β-chain of CVF, has been injected into the arteria pulmonalis of cynomolgus monkeys to assess any potential acute lung damage or any other acute side effects (Fritzinger et al., 2008a). Upon injection of rhC3-derivative HC3-1496 at 250 $\mu$g/kg and even 1000 $\mu$g/kg multiple physiological lung parameters were not affected by the rapid complement depletion. Only at the very high dose of 1000 $\mu$g/kg a transient increase in the heart rate and systolic blood pressure was observed. Further important observations include the rapid removal of generated C3a from the circulation by carboxypeptidase N and the absence of any measurable C5a generation. Based on these data, complement activation by rhC3-derivatives used for the present invention is well tolerated and appears to be safe.

Only in individuals with a deficiency of carboxypeptidase N local depletion of complement component C3 with rhC3-derivatives would be contra-indicated, but such a deficiency is extremely rare. Only two siblings with 21% of normal carboxypeptidase N activity and a single patient with 3% of normal carboxypeptidase N activity have been reported (Mathews et al., 1980; Willemse et al., 2008). However, even in this patient population local depletion of complement component C3 with rhC3-derivatives may be feasible, if the administration of a suitable rhC3-derivative is combined with coincident administration of recombinant human carboxypeptidase N.

A potential limitation for repeated applications of rhC3-derivatives could be the immunogenicity of the CVF-portion of the derivatives. However, based on the high sequence identity of human complement component C3 with those rhC3-derivatives which are useful for the present invention (rhC3-derivative H5: 90%; rhC3-derivative H6: 96.3%; rhC3-derivative HC3-1496: 94%), the potential immunogenicity of these rhC3-derivatives in humans is expected to be very low or even negligible. Amino acid differences are limited to the very C-terminus of the α-chain. However, the CVF-specific residues are not contiguous but interspersed throughout the exchanged sequence. Furthermore, CVF and human C3 are structurally highly homologous. The exchanged region contains the C-terminal C345C domain which has the same three-dimensional structure in both CVF and human C3. In addition, the N-glycosides with immunogenic terminal galactosyl residues in CVF are absent in rhC3-derivatives expressed in insect or mammalian cells.

**III. Sustained local C3 depletion with rhC3-derivatives**

Locally produced C3 is most important for regulation of effector T cell responses, particularly under local inflammatory conditions which induce significant up-regulation of local C3 synthesis. Therefore, local C3 depletion is required for the modulation of effector T cell responses. A single injection of a recombinant human C3-derivatives at the site of inflammation or tissue stress is likely to effect local C3 depletion only for a limited period of time, but production and secretion of C3 by DCs and macrophages is an ongoing process under pathological conditions. Repeated local administrations of rhC3-derivatives are possible, but from a practical point of view multiple injections are unlikely to be accepted by patients and physicians. Therefore, effective therapeutic targeting of locally synthesized C3 requires a sustained local delivery of rhC3-derivates from a depot-mediating matrix for a period of time that is sufficient for the purpose of the particular medical treatment.

In a preferred embodiment, matrices are used for local delivery of suitable rhC3-derivatives that a) can serve as depot for substantial quantities of a suitable rhC3-derivative, b) allow the release of sufficient quantities of the embedded rhC3-derivative over a prolonged period of time (optimally for a few days), c) are biodegradable, and d) are chemically and physically compatible with the rhC3-derivative and other compounds necessary for modulation of effector T cell responses according to the method of the present invention.

In one embodiment of the invention, biodegradable polymers are used for controlled delivery of suitable rhC3-derivatives. Preferred biodegradable polymers approved by FDA and used in a clinical trial, include but are not limited to poly(D,L-lactic acid), poly(lactic-co-glycolic acid) (PLGA), and copolymers of L-lactide and D,L-lactide. An important characteristic of such polymers is their ability to be applied locally. All FDA approved polymers have been studied extensively for their biocompatibility, toxicology, and degradation kinetics. Furthermore, these polymers have been shown to release embedded therapeutics for several hours up to 40 weeks in *vitro* and several weeks *in vivo.*

In a more preferred embodiment, injectable in situ-forming gel systems which are biodegradable, are used for controlled delivery of suitable rhC3-derivatives. Preferred in situ-forming gel systems (hydrogels) undergo a sol-gel-sol transition, which is a free flowing sol at room temperature and a non-flowing gel at body temperature. Compared to other biodegradable polymers, the injectable thermogelling polymers are possessing several advantages including easy preparation, high encapsulation efficiency of bioactive molecules including therapeutic proteins, and free of harmful organic solvents in the formulation process (Qiao et al. 2005).

In one specific embodiment, biodegradable thermogelling block polymers are used which are based on monomethoxy poly(ethylene glycol) (MPEG) including but not limited to a) diblock copolymers consisting of MPEG and poly (ε-caprolactone) (PCL) (Hyun et al., 2007), b) MPEG-*b*-(PCL-*ran*-PLLA) diblock copolymers (Kang *et al.*, 2010), and c) diblock copolymers consisting of MPEG and PLGA (Peng et al., 2010). MPEG copolymers containing PCL provide the advantage that they do not create an acidic environment upon biodegradation in contrast to MPEG copolymers containing PLLA and PLGA (Hyun et al., 2007).

In another specific embodiment, biodegradable thermogelling triblock polymers are used including but not limited to a) PLGA-PEG-PLGA (Qiao et al., 2005), b) PEG-PLGA-PEG (Zhang et al., 2006), and c) PEG-PCL-PEG (PECE) (Gong et al., 2009a). Various biodegradable thermogelling triblock polymers made up of PLGA and PEG are disclosed in patent application WO 99/18142. At lower temperatures, hydrogen bonding between hydrophilic PEG segments of the copolymer chains and water molecules dominate in aqueous solutions, resulting in the dissolution of these copolymers in water. As the temperature increases, the hydrogen bonding becomes weaker, while hydrophobic forces of the hydrophobic segments such as PLGA segments are getting stronger, leading to sol-gel transition. PEG, PLGA and PCL are well-known FDA-approved biodegradable and biocompatible materials which have been widely used in the biomedical field.

In another specific embodiment, biodegradable thermo-gelling diblock and triblock copolymers are used which consist of polyethylene oxide (PEO) and a biodegradable polyester such as poly-L-lactic acid (PLLA) (Jeong et al., 1997). These block copolymers, however, are a free flowing sol at a higher temperature and form a gel at a lower temperature. For example, a 23% aqueous solution of PEO-PLLA-PEO ($M_r$ 5,000-2,040-5,000) is a sol at 45°C and becomes a gel at 37°C By changing the biodegradable block length, the sol-gel transition temperature can be manipulated, e.g., increasing the PLLA block length increases the aggregation tendency of a block copolymer in water, resulting in a steepening of the gel-sol transition curve slopes and the onset of gelation at lower concentrations. The sol-gel transition temperature is a function of concentration as well as composition of a block polymer.

In another specific embodiment, Poloxamers (trade name Pluronics) are used. Poloxamers are nonionic triblock copolymers composed of a central hydrophobic chain of poly(propylene oxide) (PPO) flanked by two hydrophilic chains of poly (ethylene oxide) (PEO) (Gilbert et al., 1987). Poloxamers exhibit also sol-gel transition behavior in aqueous solutions and have been used for sustained delivery of several therapeutic agents. However, Poloxamers are not biodegradable and can be accumulated in the body which may lead to toxic side effects. Thus, the application of Poloxamers in biomedical fields has been greatly restricted. In a recent study, Pluronic F127 (100-unit PEO chain surrounding one 65-unit PPO) has been used to form composite thermosensitive hydrogels with PECE (Gong et al., 2009b). Based on the results of this study Pluronic F127/PECE composite hydrogels are biocompatible with low cell cytotoxicity and, therefore, may also be suitable for the method of the present invention.

The various biodegradable thermogelling polymers provide different stability characteristics. For example, Poloxamer triblock polymers provide excellent thermosensitivity, but due to weak hydrophobicity of the PPO block such copolymers form fast eroding gels which have been reported to persist *in vivo* a few hours at most. Exposure of Poloxamer gels to phosphate-buffered saline under *in vitro* conditions demonstrated gel erosion within 2 days (Hyun et al., 2007). Similar results were observed when the polymer solutions were subcutaneously injected into rats. Poloxamer gels could not be observed after 2 days (Hyun et al., 2007). Different results were obtained with MPEG-PCL gels. Under *in vitro* conditions MPEG-PCL gels maintained their structural integrity for more than 28 days and after subcutaneous injection into rats MPEG-PCL gels maintained their structural integrity longer than 30 days. The stability of MPEG-PCL gels, however, may also create problems since the rate of degradation of PCL *in vivo* is rather slow (2-3 years) compared to that of PLA, PGA or PLGA (for a review, see Sinha et al., 2004). Thus, after serving the function in delivering a suitable rhC3-derivative, MPEG-PCL copolymers may remain in the body under physiological conditions for an uncertain period. Therefore, most preferred biodegradable thermogelling polymers for the method of the present invention are those which maintain their structural integrity for a few days but do not remain in the body for more than a month.

In a preferred embodiment of the present invention, biodegradable thermogelling polymers are used which allow to modify their degradation kinetics. For example, PLLA segments can be incorporated into the PCL segment of MPEG-PCL copolymers, since PLLA provides better accessibility of water to the ester bonds of PLLA which enhances the hydrolytic degradation of the copolymer (Kang et al., 2010). The resulting MPEG-b-(PCL-*ran*-PLLA) diblock copolymers offer a therapeutic window that is adjustable from a few weeks to a few months by varying the amount of PLLA in the PCL segment (Kang et al., 2010). In another example, the rate of PLGA-PEG-PLGA hydrogel erosion can be modified by altering the molar ratio of DL-lactide/glycolide in the PLGA segment. The DL-lactide moiety is more hydrophobic than the glycolide moiety. Therefore, by increasing the molar ratio of DL-lactide/glycolide in the PLGA segment of PLGA-PEG-PLGA triblock copolymers, more stable hydrogels are formed due to stronger hydrophobic interactions among the copolymer molecules (Qiao et al. 2005).

Several of the biodegradable thermogelling polymers have been analyzed for their ability to mediate sustained release of proteins. Although different proteins are likely to affect the release behavior of each copolymer in individual ways, characterization of the release of model proteins such as bovine serum albumin (BSA) provides important information. Using composite hydrogels containing different percentages of PECE and Pluronic F127, the *in vitro* release behavior of BSA proved to be dependent on the hydrogel composition, initial BSA loading amount, and hydrogel concentration (Gong et al., 2009b). Sustained release of BSA above 15 days was achieved with a composite hydrogel containing 60% PECE and 40% Pluronic F127, loaded with 4 mg BSA in the presence of 30wt% hydrogel. Using MPEG-PCL copolymers, sustained *in vitro* release of BSA proved to be above 20 days, and under *in vivo* conditions (after subcutaneous injection into rats) sustained release lasted for more than 30 days (Hyun et al., 2007).

While for most clinical applications a sustained release of therapeutic drugs from biodegradable thermogelling polymers over a period of several weeks is desirable, the method of the present invention does not require such an extended sustained release of rhC3-derivatives since the development of immunologic memory requires the engagement of the T cell receptor (TCR) for a period of only 1 to 2 days. Therefore, preferred are biodegradable thermogelling polymers which deliver rhC3-derivatives for a limited period only which does not exceed a week.

PLGA-PEG-PLGA triblock copolymers represent one example of hydrogels providing advantageous degradation and release kinetics for the method of the present invention. As demonstrated in a recent study, the release of insulin from PLGA-PEG-PLGA triblock copolymers lasted for approximately 4 days with an initial burst effect during the first day (Choi et al, 2003). The initial burst effect may be advantageous for effective depledtion of C3 in the initial phase. However, the release kinetics may be modified by reducing the solubility of rhC3-derivatives via complexation with zink as shown for zink-insulin complexes (Choi et al, 2003).

Although Poloxamer gels are not biodegradable, Poloxamer 407 (trade name Pluronic F127) gels represent also an example of thermogelling polymers providing advantageous degradation and release kinetics for the method of the present invention. Although under *in vitro* conditions the release of BSA from Poloxamer 407 gels proved to be almost complete within 1 day, the sustained release of BSA under *in vivo* conditions (after subcutaneous injection into rats) lasted for 3 days (Hyun et al., 2007).

**IV. Safety aspects of PEG/PLGA-based hydrogels**

Preferred biodegradable polymers include but are not limited to poly(ethylene glycol) (PEG), poly(D,L-lactic acid), copolymers of L-lactic acid and D,L-lactic acid, poly(glycolic acid), and poly(lactic-co-glycolic acid) (PLGA). The use of these polymers as sustained-release protein delivery systems has been studied extensively (for a review, see Pai et al., 2009) and they represent the most compelling biodegradable polymers for depot systems due their inclusion in the FDA's General Recognized as Safe (GRAS) list for use in medical devices and drug formulations (FDA, http: //vm.cfsan.fda.gov/%7Edms/eafus.html. Accessed various dates).

### V. Fields of application for local C3 depletion

The methods of the present invention for local C3 depletion at the site of antigen or allergen presentation can be used in any mammalian organism to modulate effector T cell responses.

In one embodiment, the methods of the present invention are used to modulate immune responses in mammals, preferably in humans, for the treatment of T cell-mediated diseases and pathological conditions selected from the group consisting of, but not limited to, allergy, allergic asthma, acute respiratory stress (ARDS), type 1 diabetes, systemic lupus erythemadodes, glomerulonephritis, rheumatoid arthritis, multiple sclerosis, dermatomyositis, nephritis, Parkinson's disease, Alzheimer's disease, pemphigoid, sepsis, myocardial ischemia, acute myocardial infarction (AMI), reperfusion, hemodialysis, paroxysmal nocturnal hemoglobinuria (PNH), age-related macula degeneration (AMD), cardiopulmonary bypass (CPB), angioplasty, hyperacute rejection, transplant rejection, stroke, burns, spinal cord injury, and traumatic brain injury (TBI).

In a preferred embodiment, local depletion of complement component 3 according to the methods of the present invention is used to modulate immune responses in mammals, preferably in humans, for the treatment of allergy, allergic asthma, and type 1 diabetes.

### VI. Local C3 depletion as adjuvant therapy for the treatment of allergy.

Specific allergen immunotherapy (IT) is the only widely used treatment capable of restoring clinical tolerance to allergen and is associated with the re-induction of allergen-specific Treg cells. Peripheral tolerance to allergen in the normal immune response of healthy individuals to allergen exposure and in specific immunotherapy is mainly the result of sufficient Treg numbers. Therefore, the main principle of specific immunotherapy in atopic patients is to increase the number of Tregs capable of controlling allergen-specific effector T cells to augment the induction of clinical tolerance to allergens. Successful IT is associated with a change of T cell memory from the pro-allergic Th2 phenotype towards a peripheral IL-10-producing T cell phenotype and a local phenotype that also shows FOXP3 expression in addition to IL-10 positive cells.

Inhibition of IL-4- and IL-13-mediated effects along with allergen stimulation of T cells represents one approach for increasing the number of Tregs capable of controlling allergen-specific effector T cells. Under *in vitro* conditions, the presence of allergen and the absence of TH1- or TH2-driving factors have been demonstrated to be essential for the induction of Tregs. Since IL-4 and IL-13 play key roles in the development of allergic inflammation, coincident *in vivo* inhibition of IL-4- and IL-13-mediated effects along with allergen stimulation of T cells has been applied to augment the induction of clinical tolerance to allergens (see EP patent application 11075261.5).

In one preferred embodiment of the present invention, local depletion of complement component C3 prior and along with allergen stimulation of T cells is performed as adjuvant therapy during specific immunotherapy of allergic individuals. This approach has been demonstrated to increase in mice the number of Tregs capable of controlling allergen-specific effector T cells (Peng et al., 2006). Using the expression of the *foxp3* gene as marker for CD4+CD25+ regulatory T cells (Fontenot et al., 2003), a recent study has demonstrated that $C3^{+/+}$ and $C3^{-/-}$ dendritic cells (DCs) have different abilities to elicit the development of regulatory T cells (Peng et al., 2006). Co-culturing of CD4+ T cells with allogeneic C3 deficient ($C3^{-/-}$) dendritic cells (DCs) for 9 days yielded a 4-fold higher level in T cells expressing the *foxp3* gene as compared to those stimulated with $C3^{+/+}$ DCs (Peng et al., 2006).

The impact of local C3 depletion on the development of tolerance to allergens is also evident from another recent study (Yalcindag et al., 2006). In order to investigate the role of C3 in inciting allergic skin inflammation and systemic immune responses after epicutaneous or intraperitoneal sensitization with allergens, C3-deficient ($C3^{-/-}$) mice and wild-type mice were subjected to epicutaneous or intraperitoneal immunization with ovalbumin (OVA) adsorbed onto alum. By comparison of the epicutaneous or intraperitoneal immunization procedure, the study addressed the local synthesis of C3 by keratinocytes and peritoneal macrophages, both of which are activated by the adjuvant alum. The study revealed that splenocytes from C3-deficient mice secreted less TH2-specific interleukins including IL-4, IL-5 and IL-13, and less TH1-specific IFN-$\gamma$ in response to OVA stimulation than splenocytes from wild-type control mice. C3-deficient mice had impaired IgG1, IgG2a, and IgE antibody responses after both epicutaneous and intraperitoneal immunization. It is important to note that the defect was corrected by the addition of purified C3 protein (Yalcindag et al., 2006).

Collectively, the data of theses studies indicate that local depletion of complement component C3 prior and along with allergen stimulation of T cells provides significant benefits to allergic individuals if applied as adjuvant therapy for allergen-specific immunotherapy.

### VII. Local C3 depletion as adjuvant therapy for the treatment of allergic asthma.

In another preferred embodiment of the present invention, local depletion of complement component C3 prior and along with allergen stimulation of T cells is performed as adjuvant therapy during specific immunotherapy of individuals suffering from allergic asthma.

As demonstrated by several animal studies, local depletion of complement component C3 prior and along with allergen stimulation of T cells represents a promising adjuvant approach for the therapy of allergic asthma. For example, using a mixture of *Aspergillus fumigatus* and OVA in a murine model of pulmonary allergy, C3 deficient mice were protected from airway reduction due to reduced airways responsiveness to inhaled methacholine (Drouin et al., 2001). Furthermore, in this study reduced levels of IL-4, antigen-specific IgE and IgG, and reduced eosinophil infiltration were observed. In another study, using the OVA model of antigen-induced airway hyper-responsiveness, near complete protection from the development of hyper-responsiveness to aerosolized methacholine was observed in C3aR deficient mice (Humbles et al., 2000). The same observation was made in a strain of guinea pigs with a natural C3aR defect (Bautsch et al., 2000). Using an OVA sensitization and challenge model of asthma, significant protection from airway broncho-constriction following antigen challenge (in contrast to airway hyper-responsiveness to methacholine) was noticed. There is also evidence for an important role of C5a in the pathogenesis of allergic asthma. Using a rat model, a synthetic hexapeptide C5a antagonist as well as inhibition of complement activation by a soluble CR1-derivative blocked the inflammatory response and airway hyper-responsiveness to methacholine (Abe et al., 2001).

As indicated by the data of theses studies, local depletion of complement component C3 prior and along with allergen stimulation of T cells provides also significant benefits to individuals suffering from allergic asthma if applied as adjuvant therapy for allergen-specific immunotherapy.

## VIII. Combination of local C3 depletion and local inhibition of IL-4/IL-13-mediated effects as aduvant therapy for the treatment of allergy and asthma

In another preferred embodiment of the present invention, local depletion of complement component C3 and coincident local inhibition of IL-4/IL-13-mediated effects is performed prior and along with allergen stimulation of T cells as adjuvant therapy during specific immunotherapy of individuals suffering from allergy and/or allergic asthma. The combination of local C3 depletion and local inhibition of IL-4- and IL-13-mediated effects (described in detail in EP patent application 11075261.5) along with allergen stimulation of T cells represents a particularly promising therapeutic approach, since the combined approach allows also modulation of the functions of macrophages and DCs in a manner that favours the development of regulatory T cells with the concomitant reduction or inhibition of TH1 and TH2 immune responses.

Reduction of TH1 immune responses is also supported by local depletion of C3. Macrophages express both C3a and C5a receptors (C3aR, C5aR) and several human dendritic cell subsets (e.g., monocyte derived, dermal, Langerhans, myeloid, plasmacytoid) also express C3aR and/or C5aR. In the absence of C3a-C3aR interactions, the functions of macrophages and DCs are impaired. For example, macrophages derived from C3 deficient mice (which are unable to generate C3a) had lowered surface expression of MHC class II and reduced ability to elicit allospecific TH1 responses *in vitro* and *in vivo* (Zhou et al., 2006). In the absence of C3a-C3aR interactions, bone marrow-derived DCs exhibit also a less activated phenotype with reduced capacity for antigen uptake/presentation and lowered ability to stimulate TH1 responses (i.e. IL-12 production, generation of IFN-$\gamma$ producing T cells) *in vitro* and *in vivo* (for a review, see Zhou, 2012).

As a result of the reduced ability of macrophages and DCs to stimulate TH1 responses in the absence of C3a-C3aR interactions, immune responses could shift potentially to TH2 responses as suggested by studies with C3aR deficient mice (Kawamoto et al., 2004). In C3aR deficient mice the immune response upon epicutaneous introduction of antigen has been demonstrated to shift towards TH2 responses, with significantly higher levels of serum IgG1 and lower levels of serum IgG2a, IgG3, and IgA compared with wild-type mice (Kawamoto et al., 2004). However, the potential shift towards TH2 responses under C3-depleted conditions is inhibited by two mechanisms, by C5a-C5aR interactions and by inhibition of IL-4- and IL-13-mediated effects along with allergen stimulation of T cells. Deficiency of C5 or C5aR has been shown to inhibit TH2 immune responses in asthma, thus providing protection to antigen challeged mice (Drouin et al., 2006; Köhl et al., 2006). Coincident blockade of IL-4/IL-13-mediated pathways is important, since both TH2-related cytokines are known to play key roles in the pathogenesis of asthma and other allergic diseases.

Restoring clinical tolerance to allergen(s) is associated with the re-induction of allergen-specific Treg cells, and the generation of Tregs represents a default pathway that requires T-cell receptor activation as for any other T-cell differentiation, but the absence of decision signals for effector T cells. Therefore, reduction or inhibition of TH1 and TH2 immune responses in the absence of C3a-C3aR and C5a-C5aR interactions, and the presence of an IL-4/IL-13 antagonist is extremely promising. Furthermore, dendritic cells from $C3^{-/-}$ mice have been demonstrated to effect *in vitro* a significant increase of the number of Tregs (Peng et al., 2006).

Employing alum-adsorbed allergens for specific immunotherapy, coincident blockade of IL-4/IL-13-mediated pathways provides an additional benefit. Aluminum-containing adjuvants, typically aluminum phosphate or aluminum hydroxide gels (generally referred to as alum) are known to elicit TH2-type immune responses. Although adjuvants eliciting primarily a TH2-type immune response are potentially interfering with the induction of Tregs, in the presence of inhibitors of Il-4- and IL-13-mediated pathways such adjuvants are better suited for the therapeutic aims of the present invention than those eliciting primarily a TH1-type immune response. In the presence of inhibitors of IL-4- and IL-13-mediated pathways the contra-productive activity of adjuvants eliciting TH2-type immune responses is significantly reduced or even abolished, whereas the activity of TH1-type promoting adjuvants cannot be influenced. As a result, TH1-type cytokines would interfere with the induction of Tregs. Therefore, adjuvants eliciting TH2-type immune responses including but not limited to aluminum salts, Montanide emulsions (squalene-based water-in-oil emulsions) and polyphosphazenes, are preferred for the method of the present invention.

In conclusion, the combination of local C3 depletion and local inhibition of IL-4/IL-13-mediated effects has the potential to improve the development of tolerance significantly and, thereby, to reverse the pathological processes in allergy and asthma.

## IX. Local C3 depletion as adjuvant therapy for the treatment of type 1 diabetes

In another preferred embodiment of the present invention, local depletion of complement component C3 prior and along with self-antigen presentation to T cells is performed as adjuvant therapy during self-antigen-specific vaccination of individuals suffering from type 1 diabetes.

Type 1 diabetes (previously known as juvenile diabetes) is a T cell-dependent autoimmune disease in which islet antigens from insulin-producing β-cells of the pancreas are presented by APCs to autoreactive T cells, breaking self tolerance. Expansion of autoreactive CD4+ and CD8+ T cells, autoantibody-producing B cells and activation of the innate immune system cause β-cell injury (Bluestone et al., 2010).

The appearance of diabetes-related autoantibodies has been shown to be able to predict the appearance of diabetes type 1 before any hyperglycemia arises, the main ones being islet cell autoantibodies, insulin autoantibodies, autoantibodies targeting the 65 kDa isoform of glutamic acid decarboxylase (GAD65) and autoantibodies targeting the phosphatase-related IA-2 molecule (Knip et al., 2005). The emergence of autoantibodies may be termed 'latent autoimmune diabetes', since not everyone with autoantibodies develops diabetes type 1. However, the risk increases with the number of autoantibody types and the presence of three to four antibody types is associated with a risk of progressing to type 1 diabetes of 60% to 100%. The time interval from emergence of autoantibodies to diabetes type 1 can be a few months in infants and young children, but in some people it may take years (Knip et al., 2005). Only after destruction of approximately 90% of the β-cells, type 1 diabetes becomes clinically manifest (for a review, see Homann and von Herrath, 2004). It has been proposed that diabetes Type 1 might be prevented at the latent autoimmune stage, before it starts destroying β-cells (Bluestone et al., 2010).

Type 1 diabetes causes an estimated 5-10% of all diabetes cases or 11-22 million worldwide. The incidence of type 1 diabetes has been increasing by about 3% per year. In 2006, it affected 440,000 children under 14 years of age and was the primary cause of diabetes in those less than 10 years of age. However, the majority of new-onset type 1 diabetes is seen in adults. Adult-onset type 1 diabetes is two to three times more common than classic childhood-onset type 1 diabetes (http://en.wikipedia.org/wiki/Diabetes_mellitus_type_1).

Eventually, type 1 diabetes (autoimmune diabetes) is fatal unless continuously treated with insulin. Prancreatic islet cell transplantation is performed, but serious limitations are associated with this kind of treatment. Immunotherapy with anti-CD3 antibodies, including the humanized anti CD3 mAb teplizumab (mutated to prevent binding to Fc receptors) and anti CD3 mAb otelixizumab (similarly Fc-mutated), has provided evidence for preserved insulin production in newly diagnosed type 1 diabetes patients (Bluestone et al., 2010), but phase III studies with both antibodies failed to show clinical efficacy (Tolerx, 2011; MacroGenics, 2011), potentially due to an insufficient dosing schedule. The anti-CD20 antibody retuximab inhibits B cells and has been shown to provoke C-peptide responses (evidence for insulin production) three months after diagnosis of type 1 diabetes, but long-term effects of this treatment have not been reported (Bluestone et al., 2010).

Adverse effects of the therapeutic approaches with broad immune-suppressive agents as well as the lack of permanent remission of disease with any agent tested to date have boosted interest in the development of antigen-specific interventions. Such an approach may require only a single β-cell-derived antigen despite the polyclonal nature of the autoimmune response, reflected by the presence of multiple autoantibodies and multiple T cell epitopes that are recognized by peripheral blood cells. Immunological tolerance mechanisms, even by antigen-specific cells, are not restricted to a single antigen. Cytokines such as IL-10 and TGF-β, produced by T cells in response to antigen or antigen-presenting cells, can modulate the function of effector T cells in the vicinity of the antigen. Thereby, activated regulatory T cells can exert their function at the site of the immune response without the need to recognize those antigen(s) recognized by effector T cells. In addition, CD4+ CD25+ FoxP3+ and other regulatory T cells are able to modulate the function of effector T cells through contact-dependent mechanisms. It has been demonstrated that polyclonal diabetogenic T cells can be inhibited by antigen-specific Treg cells in vitro and in an adoptive transfer model of diabetes (for a review, see Bluestone et al., 2010).

A promising approach is the vaccination with the 65 kDa isoform of glutamic acid decarboxylase (GAD65).

Immunization with alum-GAD65 was shown to attenuate the loss of C-peptide in individuals treated within six months of diagnosis (Ludvigsson et al., 2008). Furthermore, oral insulin administration yielded also promising results in a subset of patients with high levels of insulin autoantibodies (Skyler et al., 2005), an observation that is now being studied in detail. These data support the concept that an antigen-specific intervention may have broad immunological effects, provided the selected antigen is directly involved in the disease pathogenesis and/or regulation. Phase III trials with alum-GAD65 are underway in the USA and in Europe (Diamyd, 2011a and 2011b). In addition, prevention studies are underway in which this vaccine is given to persons who have not yet developed type 1 diabetes (Diamyd, 2011c).

In one embodiment of the present invention, methods are disclosed which support the antigen-specific intervention approach by local depletion of complement component C3. In animal studies, immune cell-derived C3, C3a- and C5a-receptors have been demonstrated to be essential for the development of T cell-dependent type 1 diabetes induced by multiple low doses of streptozotocin (Lin et al., 2010). Therefore, local depletion of complement component C3 prior and along with self-antigen stimulation of T cells provides significant potential to support the induction of clinical tolerance to self-antigens.

Based on recent evidence, autoimmune processes such as diabetes type 1 are composed not only of autoaggressive T cell responses, but also of autoreactive regulatory components. Therefore, successful approaches for immunotherapy of type 1 diabetes obviously require enhancement of regulatory T cell responses and inhibition of autoreactive CD8+ T cells responses.

Regulatory T cells expressing CD4 and CD25 play an important role in normal immune homeostasis, as depletion of these cells by thymectomy results in the spontaneous development of various autoimmune diseases (Sakaguchi et al., 1985). There is evidence that human patients with diabetes type 1 or multiple sclerosis have a reduced frequency or functionality of CD4+ CD25+ regulatory T cells (Kukreja et al., 2002; Viglietta et al., 2004). Important for therapeutic approaches is the observation that human CD4+ CD25+ regulatory T cells may also be generated in the periphery after activation of CD4+ $CD25^-$ T cells (Akbar et al., 2003; Walker et al., 2003). The necessity for increasing the number of CD4+ $CD25^-$ regulatory T cells in patients with type I diabetes is supported by the observation that several therapeutic approaches which protect from diabetes, are correlated with an increase in CD4+ CD25+ regulatory T cells (for a review, see Juedes and von Herrath, 2004). For example, increased numbers of circulating CD4+ CD25+ regulatory T cells have been reported in patients receiving successful islet transplants and were treated with the anti-CD3 mAb hOKT3$\gamma$1 (Ala-Ala) (Hering et al., 2004).

Several studies have provided evidence that Treg cells are selectively preserved by treatment with anti-CD3 monoclonal antibodies (for a review, see Bluestone et al., 2010). *In vitro*, anti-CD3 mAbs have been shown to induce FoxP3 and CD25 expression and regulatory function on CD4+ $CD25^-$ T cells (Walker et al., 2003), and immunotherapy with anti-CD3 antibodies, including teplizumab (Herold et al., 2002) and otelixizumab (Keymeulen, 2005), has provided evidence for preserved insulin production in newly diagnosed type 1 diabetes patients.

Currently, anti-CD3 therapy is one of the most promising approaches for increasing the number of CD4+ $CD25^-$ regulatory T cells in patients with type I diabetes. However, supportive adjuvant therapeutic approaches are required to achieve a lasting remission. Coincident suppression of priming and expansion of islet-autoreactive CD8+ cytotoxic T cells appears to be important, since a very recent study has provided first time evidence for islet-autoreactive CD8+ T cells in insulitic lesions from recent-onset and long-term type 1 diabetes patients, pointing to the CD8+ T cells as being one of the most important cells in type I diabetes progression (Coppieters et al., 2012). Tolerization of these CD8+ T cell species may thus be a requirement to achieve long-lasting remission. At least for some patients, however, therapeutic tolerization will require a combination of treatment modalities, since the functionality of CD4+ CD25+ regulatory T cells can be impaired in human patients with diabetes type 1. As a result, the regulatory functions of Tregs may not be sufficient in some type I diabetes patients to efficiently suppress priming and expansion of autoreactive CD8+ cytotoxic T cells.

Animal studies have provided strong evidence that complement component C3 is required for priming and expansion of CD8+ T cells. For example, primed CD8+ T cell responses to allogeneic endothelial cells are controlled by local complement activation (Raedler et al., 2009), and activation as well as expansion of CD8+ T cells during a systemic viral infection was markedly reduced in C3-deficient mice (Suresh et al., 2003. Furthermore, priming of CD8+ T cells requires help from CD4+ T cells, and local complement has been shown to regulate the help of CD4+ cells for CD8+ T cells required for murine allograft rejection (Vieyra et al., 2011). Cognate interactions between CD4+ T cells and dendritic cells (DCs) induce C3a and C5a production and CD4-cell help is mediated, in part, by interaction of DC-derived C3a/C5a with CD8+ T cell-expressed C3aR/C5aR. This concept is supported by two observations. First, CD8+ T cells lacking C3aR and C5aR proliferate weakly to allogeneic DCs despite CD4 help. Second, augmented production of C3a/C5a by DCs bypasses the requirement for CD4- and CD40-dependent help to wild-type CD8+ T cells (Vieyra et al., 2011).

The impact of these observations for type I diabetes is evident from another recent animal study (Munegowda et al., 2011). The data of this study demonstrate that CD4+ TH17-cells and TH17-stimulated CD8+ cytotoxic T cells are involved in type I diabetes in mice. However, induction of type I diabetes required only TH17-stimulated CD8+ T cells, which is in accordance with the role of human islet-autoreactive CD8+ T cells in the pathogenesis of type I diabetes (Coppieters et al., 2012). Although the role of local complement activation for TH17-mediated stimulation of CD8+ T cells has not been investigated in this study, the mechanisms of activation are most likely to be identical with those observed in other immune responses. Therefore, local depletion of C3 by rhC3-derivatives at the site of self-antigen presentation represents an effective adjuvant therapeutic approach for a significant reduction of CD4+ helper T cell-primed autoreactive CD8+ cytotoxic T cells.

In conclusion, local C3 depletion as adjuvant therapy in combination with self-antigen vaccination and/or anti-CD3 therapy has the potential to improve the development of tolerance in type q diabetes patients significantly and, thereby, to reverse the pathological processes.

### X. Combination of local C3 depletion with local IL-4 inhibition as adjuvant therapy for the treatment of type 1 diabetes

In another preferred embodiment of the present invention, local depletion of complement component C3 and coincident local inhibition of IL-4-mediated effects prior and along with self-antigen presentation to T cells is performed as adjuvant therapy during self-antigen-specific vaccination of individuals suffering from type 1 diabetes.

Local inhibition of IL-4-mediated effects may contribute to the reduction of activated autoreactive CD8+ cytotoxic T cells via local C3-depletion at the site of self-antigen presentation, since IL-4 has been shown to be crucial for the priming of antigen-specific CD8+ T cells after vaccination with the Leishmania antigen HASPB-1 (Stäger et al., 2003). Further analyses revealed that immune complexes formed by reaction of natural antibodies with the Leishmania antigen causes complement activation, which in turn stimulates primary IL-4 secretion from CD11b+ CD11c+ phagocytes. In the presence of specific antibodies to ovalbumin (OVA), OVA injection induced IL-4 secretion comparable to that induced by the Leishmania antigen. Therefore, the authors concluded that immune complexes can, in general, provide a means of stimulating primary IL-4 secretion from CD11b+ CD11c+ phagocytes. Complement activation is essential for this process since in C1q-deficient mice neither IL-4 secretion nor priming of antigen-specific CD8+ T cell could be observed (Stäger et al., 2003).

Since in type 1 diabetes patients undergoing vaccination therapy, circulating auto-antibodies will also form immune complexes with injected self-antigens, local C3 depletion and inhibition of IL-4-mediated effects represents a promising approach to reduced priming of harmful autoreactive CD8+ cytotoxic T cells.

### XI. Compositions for local depletion of C3 comprising rhC3-derivatives, one or more antigens or allergens, and one or more matrices mediating sustained delivery of the components

In one embodiment of the present invention, compositions for specific immunotherapy of patients with type 1 allergy and/or allergic asthma and for self-antigen vaccination of patients with type 1 diabetes are disclosed, comprising a suitable rhC3-derivative, one or more self-antigens or allergens (including allergenic extracts), and for sustained delivery of the components of the composition one or more depot-mediating matrices as listed in EP patent application 11075261.5.

In a specific embodiment, a suitable rhC3-derivative is co-injected with one or more self-antigens or allergens (or allergenic extracts) adsorbed onto aluminium salts or onto another depot-mediating material as listed in EP patent application 11075261.5. The immunotherapeutic or vaccination process may include one or more injections of a suitable rhC3-derivative at the presentation site of self-antigen(s) or allergen(s).

In another specific embodiment, suitable rhC3-derivatives are released continuously from a depot-mediating matrix as listed in EP patent application 11075261.5, at the presentation site of self-antigen(s) or allergen(s) along with a sustained presentation of self-antigen(s) or allergen(s).

In a preferred specific embodiment of the present invention, polymer-embedded rhC3-derivatives and one or more self-antigens or allergens (or allergenic extracts) partially or completely adsorbed onto alum or onto another depot-mediating material as listed in EP patent application 11075261.5, are injected at the same location, but as separate preparations. After gellation of the polymer composit at the injection site, one or more self-antigens or allergens (or allergenic extracts) adsorbed onto alum or onto another depot-mediating material as listed in EP patent application 11075261.5, are injected in close proximity of the gelled polymer composit. The polymer composit comprises a biodegradable thermogelling polymer and a suitable rhC3-derivative, and is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released rhC3-derivatives is sufficient for the therapeutic aims of the method of the present invention.

In another preferred specific embodiment of the present invention, the composition comprises a biodegradable thermogelling polymer containing one or more self-antigens or allergens (or allergenic extracts) partially or completely adsorbed onto alum or onto another depot-mediating material as listed in EP patent application 11075261.5, and a suitable rhC3-derivative. The composition is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released components is sufficient for the therapeutic aims of the method of the present invention.

In still another preferred specific embodiment of the present invention, the composition comprises a biodegradable thermogelling polymer containing one or more non-adsorbed self-antigens or allergens (or allergenic extracts) and a suitable rhC3-derivative. Biodegradable polymers have the potential to provide both prolonged antigen or allergen presentation and sustained release of a suitable rhC3-derivatives sufficient for the requirements of the method of the present invention. Therefore, partial or complete adsorption of self-antigens or allergens (or allergenic extracts) onto alum or onto another depot-mediating material may be omitted. The composition is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released components is sufficient for the therapeutic aims of the method of the present invention.

## XII. Compositions comprising rhC3-derivatives, IL4/IL-13 inhibitors, one or more antigens or allergens, and one or more matrices mediating sustained delivery of the components

In another embodiment of the present invention, compositions for specific immunotherapy of patients with type 1 allergy and/or allergic asthma and for self-antigen vaccination of patients with type 1 diabetes are disclosed, comprising a suitable rhC3-derivative, one or more self-antigens or allergens (including allergenic extracts), a suitable IL-4/IL-13 inhibitor (as listed in EP patent application 11075261.5) and for sustained delivery of the components of the composition one or more depot-mediating matrices as listed in EP patent application 11075261.5.

In a specific embodiment, a suitable rhC3-derivative and one or more suitable IL-4/IL-13 inhibitors are co-injected with one or more self-antigens or allergens (or allergenic extracts) adsorbed onto aluminium salts or onto another depot-mediating material as listed in EP patent application 11075261.5. The immunotherapeutic or vaccination process may include one or more injections of a suitable rhC3-derivative and of one or more suitable IL-4/IL-13 inhibitors at the presentation site of self-antigen(s) or allergen(s).

In another specific embodiment, a suitable rhC3-derivative and one or more suitable IL-4/IL-13 inhibitors are released continuously from a depot-mediating matrix as listed in EP patent application 11075261.5, at the presentation site of self-antigen(s) or allergen(s) along with a sustained presentation of self-antigen(s) or allergen(s).

In a preferred specific embodiment of the present invention, a suitable rhC3-derivative and one or more suitable IL-4/IL-13 inhibitors, both embedded in a polymer, and one or more self-antigens or allergens (or allergenic extracts) partially or completely adsorbed onto alum or onto another depot-mediating material as listed in EP patent application 11075261.5, are injected at the same location, but as separate preparations. After gellation of the polymer composit (containing a suitable rhC3-derivatives and one or more suitable IL-4/IL-13 inhibitors) at the injection site, one or more self-antigens or allergens (or allergenic extracts) adsorbed onto alum or onto another depot-mediating material as listed in EP patent application 11075261.5, are injected in close proximity of the gelled polymer composit. The polymer composit comprises a biodegradable thermogelling polymer, a suitable rhC3-derivative and one or more suitable IL-4/IL-13 inhibitors, and is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released rhC3-derivative and one or more IL-4/IL-13 inhibitors is sufficient for the therapeutic aims of the method of the present invention.

In another preferred specific embodiment of the present invention, the composition comprises a biodegradable thermogelling polymer containing one or more self-antigens or allergens (or allergenic extracts) partially or completely adsorbed onto alum or onto another depot-mediating material as listed in EP patent application 11075261.5,, a suitable rhC3-derivative and one or more suitable IL-4/IL-13 inhibitors. The composition is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released components is sufficient for the therapeutic aims of the method of the present invention.

In still another preferred specific embodiment of the present invention, the vaccine composition comprises a biodegradable thermogelling polymer containing one or more non-adsorbed self-antigens or allergens (or allergenic extracts), a suitable rhC3-derivative and one or more suitable IL-4/IL-13 inhibitors. The composition is prepared by mixing all components at a temperature at which the biodegradable thermogelling polymer is a free flowing sol (e.g., at room temperature). The quantity of each component in the composition is balanced in a way that a) upon injection the biodegradable thermogelling polymer forms a non-flowing gel in which the other components are embedded, and b) upon gellation of the polymer composit at body temperature the amount of released components is sufficient for the therapeutic aims of the method of the present invention.

**XIII. Methods for local C3 depletion and local IL-4/IL-13 inhibition as adjuvant therapy for allergen-specific immunotherapy or vaccination with self-antigens**

In one embodiment of the present invention, methods for local C3-depletion at the site of allergen presentation as adjuvant therapy prior and during specific immunotherapy in patients with type 1 allergy and/or allergic asthma are disclosed.

In one specific embodiment of the present invention, a suitable recombinant human C3-derivative (rhC3-derivative) is administered subcutaneously or intradermally. After a period of time, sufficient for local depletion of C3, one or more allergens (or allergenic extracts) partially or completely adsorbed onto alum or onto another depot-mediating material are injected at the same site. Dependent on the properties of the rhC3-derivative, repeated injections of the rhC3-derivative at the site of allergen presentation may be performed, in doses sufficient for maintaining local C3 depletion within the period of allergen-presentation to T cells.

In another specific embodiment of the present invention, a suitable recombinant human C3-derivative (rhC3-derivative) is administered subcutaneously or intradermally in a depot-mediating matrix for sustained delivery of the rhC3-derivative. After a period of time, sufficient for local depletion of C3, one or more allergens (or allergenic extracts) partially or completely adsorbed onto alum or onto another depot-mediating material are injected at the same site. Dependent on the properties of the rhC3-derivative embedded in the depot-mediating matrix, additional injection(s) of non-embedded rhC3-derivative at the site of allergen presentation may be performed to support local C3 depletion within the period of allergen-presentation to T cells.

In still another specific embodiment of the present invention, a suitable recombinant human C3-derivative (rhC3-derivative) and one or more allergens (or allergenic extracts), both embedded in a depot-mediating matrix for sustained delivery, are administered subcutaneously or intradermally. Dependent on the properties of the rhC3-derivative embedded in the depot-mediating matrix, additional injection(s) of non-embedded rhC3-derivative at the site of allergen presentation may be performed to support local C3 depletion within the period of allergen-presentation to T cells.

In another embodiment of the present invention, methods for local C3-depletion and local inhibition of IL-4/IL-13-mediated effects at the site of allergen presentation as adjuvant therapy prior and during specific immunotherapy in patients with type 1 allergy and/or allergic asthma are disclosed.

In one specific embodiment of the present invention, a suitable recombinant human C3-derivative (rhC3-derivative) is administered subcutaneously or intradermally. After a period of time, sufficient for local depletion of C3, one or more suitable inhibitors of IL-4/IL-13-mediated effects, embedded in a depot-mediating matrix for sustained delivery of the inhibitor(s), and one or more allergens (or allergenic extracts) partially or completely adsorbed onto alum or onto another depot-mediating material are injected at the same site. Dependent on the properties of the rhC3-derivative, repeated injections of the rhC3-derivative at the site of allergen presentation may be performed, in doses sufficient for maintaining local C3 depletion within the period of allergen-presentation to T cells.

In another specific embodiment of the present invention, a suitable recombinant human C3-derivative (rhC3-derivative) and one or more suitable inhibitors of IL-4/IL-13-mediated effects, both embedded in a depot-mediating matrix for sustained delivery, are administered subcutaneously or intradermally. After a period of time, sufficient for local depletion of C3, one or more allergens (or allergenic extracts) partially or completely adsorbed onto alum or onto another depot-mediating material are injected at the same site. Dependent on the properties of the rhC3-derivative embedded in the depot-mediating matrix, additional injection(s) of non-embedded rhC3-derivative at the site of allergen presentation may be performed to support local C3 depletion within the period of allergen-presentation to T cells.

In still another specific embodiment of the present invention, a suitable recombinant human C3-derivative (rhC3-derivative), one or more suitable inhibitors of IL-4/IL-13-mediated effects, and one or more allergens (or allergenic extracts), all components embedded in a depot-mediating matrix for sustained delivery, are administered subcutaneously or intradermally. Dependent on the properties of the rhC3-derivative embedded in the depot-mediating matrix, additional injection(s) of non-embedded rhC3-derivative at the site of allergen presentation may be performed to support local C3 depletion within the period of allergen-presentation to T cells.

In another embodiment of the present invention, methods for reducing the activation of autoreactive CD8+ cytotoxic T cells by local C3-depletion at the site of self-antigen presentation as adjuvant therapy prior and during self-antigen-specific vaccination in patients with type 1 diabetes are disclosed.

In one specific embodiment of the present invention, a suitable recombinant human C3-derivative (rhC3-derivative) is administered subcutaneously or intradermally. After a period of time, sufficient for local depletion of C3, one or more self-antigens partially or completely adsorbed onto alum or onto another depot-mediating material are injected at the same site. Dependent on the properties of the rhC3-derivative, repeated injections of the rhC3-derivative at the site of antigen presentation may be performed, in doses sufficient for maintaining local C3 depletion within the period of antigen-presentation to T cells.

In another specific embodiment of the present invention, a suitable recombinant human C3-derivative (rhC3-derivative) is administered subcutaneously or intradermally in a depot-mediating matrix for sustained delivery of the rhC3-derivative. After a period of time, sufficient for local depletion of C3, one or more self-antigens partially or completely adsorbed onto alum or onto another depot-mediating material are injected at the same site. Dependent on the properties of the rhC3-derivative embedded in the depot-mediating matrix, additional injection(s) of non-embedded rhC3-derivative at the site of antigen presentation may be performed to support local C3 depletion within the period of antigen-presentation to T cells.

In still another specific embodiment of the present invention, a suitable recombinant human C3-derivative (rhC3-derivative) and one or more self-antigens both embedded in a depot-mediating matrix for sustained delivery, are administered subcutaneously or intradermally. Dependent on the properties of the rhC3-derivative embedded in the depot-mediating matrix, additional injection(s) of non-embedded rhC3-derivative at the site of antigen presentation may be performed to support local C3 depletion within the period of antigen-presentation to T cells.

In another embodiment of the present invention, methods for reducing the activation of autoreactive CD8+ cytotoxic T cells by local C3-depletion and local inhibition of IL-4-mediated effects, at the site of self-antigen presentation as adjuvant therapy prior and during self-antigen-specific vaccination in patients with type 1 diabetes are disclosed.

In one specific embodiment of the present invention, a suitable recombinant human C3-derivative (rhC3-derivative) is administered subcutaneously or intradermally. After a period of time, sufficient for local depletion of C3, one or more suitable inhibitors of IL-4/IL-13-mediated effects, embedded in a depot-mediating matrix for sustained delivery of the inhibitor(s), and one or more self-antigens partially or completely adsorbed onto alum or onto another depot-mediating material are injected at the same site. Dependent on the properties of the rhC3-derivative, repeated injections of the rhC3-derivative at the site of antigen presentation may be performed, in doses sufficient for maintaining local C3 depletion within the period of antigen-presentation to T cells.

In another specific embodiment of the present invention, a suitable recombinant human C3-derivative (rhC3-derivative) and one or more suitable inhibitors of IL-4/IL-13-mediated effects, both embedded in a depot-mediating matrix for sustained delivery, are administered subcutaneously or intradermally. After a period of time, sufficient for local depletion of C3, one or more self-antigens partially or completely adsorbed onto alum or onto another depot-mediating material are injected at the same site. Dependent on the properties of the rhC3-derivative embedded in the depot-mediating matrix, additional injection(s) of non-embedded rhC3-derivative at the site of antigen presentation may be performed to support local C3 depletion within the period of antigen-presentation to T cells.

In still another specific embodiment of the present invention, a suitable recombinant human C3-derivative (rhC3-derivative), one or more suitable inhibitors of IL-4/IL-13-mediated effects, and one or more self-antigens, all components embedded in a depot-mediating matrix for sustained delivery, are administered subcutaneously or intradermally. Dependent on the properties of the rhC3-derivative embedded in the depot-mediating matrix, additional injection(s) of non-embedded rhC3-derivative at the site of antigen presentation may be performed to support local C3 depletion within the period of antigen-presentation to T cells.

In another embodiment of the present invention, methods for systemic anti-CD3 therapy in patients with type I diabetes are combined with methods for reducing the activation of autoreactive CD8+ cytotoxic T cells by local C3-depletion at the site of self-antigen presentation as adjuvant therapy prior and during self-antigen-specific vaccination as described above.

In still another embodiment of the present invention, methods for systemic anti-CD3 therapy in patients with type I diabetes are combined with methods for reducing the activation of autoreactive CD8+ cytotoxic T cells by local C3-depletion and local inhibition of IL-4-mediated effects, at the site of self-antigen presentation as adjuvant therapy prior and during self-antigen-specific vaccination as described above.

Preferred type 1 diabetes patients for the therapeutic approaches of the present invention are those with a latent autoimmune stage or with a new- or recent-onset type 1 diabetes. However, even patients with long-term type 1 diabetes may qualify for the therapeutic approaches of the present invention.

XIV. Therapeutic doses of rhC3-derivatives

The determination of a therapeutically effective dose of a suitable rhC3-derivative necessary for local depletion of C3, depends on the characteristics of the administered rhC3-derivative and the kind of treatment. For example, repeated injections of rhC3-derivatives at the site of antigen presentation are likely to require a lower dose of rHC3-derivative per injection as compared to single injection regimens. Furthermore, sustained complement activation by hydrogel-mediated release of rhC3-derivatives may require incorporation of high doses of a suitable rhC3-derivative into the hydrogel due to potential conformational changes of rhC3-derivatives upon polymer incorporation resulting in a partial loss of activity. It is known that protein conformation is very sensitive to local environments and interfacial adsorption of polymer-embedded proteins has been found to limit the performance of sustained protein release from biodegradable depots as a result of impaired biological activity and reduced release from the polymer (for a review, see Pai et al., 2009).

However, the determination of a therapeutically effective dose is well within the capability of those skilled in the art. The therapeutically effective dose can be estimated initially in animal models, usually mice, rats, rabbits, dogs, pigs, or non-human primates. The animal model also can be used to determine the appropriate concentration range and route of administration. Such information can then be used to determine useful doses and routes for administration in humans.

Several animal studies performed in the recent past with rhC3-derivative HC3-1496 have provided useful data which allow to correlate the quantity of injected rhC3-derivative and the route and the number of injections with the degree of complement activation resulting in the depletion of complement components (for a review, see Vogel and Fritzinger, 2010). Furthermore, these studies provide also information about the time required for re-synthesis of depleted complement components.

Injection of HC3-1496 into the pulmonary artery of cynomolgus monkeys over a period of 1-5 min at 250 $\mu$g/kg or 1000 $\mu$g/kg resulted in complete depletion of complement within 5 min (approximately 20% residual hemolytical activity) and remained depleted for at least 1 h, followed by a gradual increase to approximately 50% activity over the next 6 h (Fritzinger et al., 2008a, Vogel and Fritzinger, 2010). No reduction of serum complement was observed with 62.5 $\mu$g/kg of HC3-1496.

Intraperitoneal injection of HC3-1496 into adult Sprague-Dawley rats at 280 $\mu$g/kg or 760 $\mu$g/kg resulted in complete depletion of complement (approximately 5% residual activity) and remained depleted for at least 4 h, followed by a gradual increase to approximately 50% over the next 20 h (Fritzinger et al., 2008b, Vogel and Fritzinger, 2010).

Intraperitoneal injection of biotinylated HC3-1496 into mice at 500 $\mu$g/kg resulted in complete depletion of complement (less than 5% residual activity) and remained depleted for at least 4 h, followed by a gradual increase to approximately 80% over the next 6 h (Vogel and Fritzinger, 2010).

In a murine model of collagen-induced arthritis, complement depletion was maintained for two or three weeks by an initial intraperitoneal injection of HC3-1496 at 500 $\mu$g/kg, followed by a maintenance dose of 250 $\mu$g/kg on 5 days/week (Fritzinger et al., 2008b, Vogel and Fritzinger, 2010).

In a murine model of age-related macular degeneration intraperitoneal injection of HC3-1496 at a very low dose of 25 $\mu$g/kg for 28 days resulted in an average reduction of about 30% complement haemolytic activity on day 28, with significant animal to animal variation (Fritzinger et al., 2008b, Vogel and Fritzinger, 2010).

In summary, these data provide useful guidelines for the determination of a therapeutically effective dose for those skilled in the art.

## XV. Modes of administration of therapeutic compositions

Routes of administration of the compositions of the present invention by injection or by implantation include but are not limited to subcutaneous, intradermal, intramuscular, nasal, transbucal, transmucosal, sublingual, rectal, vaginal, intraocular, or topical administration. Preferred examples of routes of administration of the compositions of the present invention include but are not limited to intradermal, subcutaneous, and intramuscular administration.

For mucosal immunization, the physicochemical characteristics of the administered components and the delivery vehicle have to be adjusted to stimulate their uptake through the various mucosal routes. For example, alum salts are ineffective when administered by the oral or nasal route. In contrast, cationic chitosan derivatives are of special interest in nasal delivery because of their excellent biocompatibility and mucoadhesive nature (Hagenaars et al., 2010). For example, a thermal-sensitive hydrogel which was formulated as intranasal vaccine with N[(2-hydroxy-3-trimethylammonium)propyl] chitosan chloride (HTCC) and $\alpha$,$\beta$-glycerophosphate, was shown to significantly prolong the antigen residence time in the nasal cavity and to enhance the transepithelial transport via the paracellular routes (Wu et al., 2012).

Dosage regimens may be adjusted to provide the optimum therapeutic response. The quantity of the polymer-embedded components depends on the release kinetics of the biodegradable thermogelling polymer and is adjusted to a level that guarantees the continuous release of therapeutically effective doses over a period of 3 to 5 days. The quantity of embedded components will vary according to factors such as the weight and the age of the individual, and the ability of the composition to induce an effective immune response in the individual.

## XVI. Pharmaceutical formulations

In one embodiment, the therapeutic compositions of the present invention are incorporated into pharmaceutical compositions suitable for administration. Such compositions typically comprise the therapeutic compositions of the present invention and a pharmaceutically acceptable carrier. As used herein, a 'pharmaceutically acceptable carrier' is intended to include any and all solvents, dispersion media, coatings, antibacterial and antifungal agents, isotonic systems, and the like, compatible with the components of the therapeutic compositions of the present invention and pharmaceutical administration. The use of such media and agents for pharmaceutically active substances is well known in the art. Supplementary active compounds can also be incorporated into the composition.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents, antioxidants such as ascorbic acid or sodium bisulfite, chelating agents such as ethylenediaminetetraacetic acid, buffers such as acetates, citrates or phosphates and agents for the adjustment of toxicity such as sodium chloride or dextrose. The pH can be adjusted with acids or bases, such as hydrochloric acid or sodium hydroxide. In many cases, it will be preferable to include isotonic agents, for example, sugars, polyalcohols such as mannitol, sorbitol, sodium chloride in the composition. The composition should be fluid to the extent that easy syringability exists. The proper fluidity can be maintained, for example, by the use of a coating such as lecithin, by the maintenance of the required particle size in the case dispersion and by use of surfactants. The composition must be stable under the conditions of manufacture and storage and must be preserved against the contaminating action of microorganisms such as bacteria and fungi. Prevention of the action of microorganisms can be achieved by various antibacterial and antifungal agents such as parabens, chlorobutanol, phenol, ascorbic acid, thimoseral, and the like. In all cases, the composition must be sterile. Sterile injectable solutions can be prepared by filtered sterilization. The preparation can be enclosed in ampoules, disposable syringes or multiple dose vials made of glass or plastic. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof. For practical purposes it should be kept in mind that aluminum-adsorbed vaccines are frost sensitive and therefore not lyophilizable.

## XVII. Definitions

"Allergic diseases" include but are not limited to diseases triggered by exposure to proteins as identified in the Allergome project (www.allergome.org), IgE reactive allergens, contact allergens, inhalative allergens, injection allergens (insects), including diseases like Allergic Rhinitis, Asthma Bronciale, Conjunctivitis, Atopic Dermatitis, Urticaria, Anaphylactic Shock, vomiting and/or diarrhea upon exposure to an allergen.

Specific antigenes of compounds, cells or organisms related with said diseases are published in the scientific literature and are easily identified by a person skilled in the art.

The phrase "recombinant C3-derivative" includes a polypeptide or protein having a length of 1276 to 1663 amino acids, which is a derivative of human complement component C3 (human C3), the amino acid sequence of which is shown in SEQ ID NO:2 of US-8,119,769, wherein the carboxy terminal part of at least 68 amino acid residues of human C3 is replaced by a partial sequence of Cobra Venom Factor (CVF), the amino acid sequence of which is shown in SEQ ID NO:4 of US-8,119,769 , which partial sequence comprises at least the 68 carboxy terminal amino acid residues of CVF or a fragment thereof lacking 1 to 25 carboxy terminal amino acids, wherein said protein has at least 70% identity to human C3. Thus, the invention utilizes a protein comprising a derivative of human complement. component C3 (human C3), the human C3 having an amino acid sequence set forth as SEQ ID NO: 2 of US-8,119,769 , wherein the carboxy terminal part of at least 68 amino acids of said human C3 is replaced by a partial sequence of Cobra Venom Factor (CVF), the CVF having an amino acid sequence set forth as SEQ ID NO: 4 of US-8,119,769, wherein the partial sequence of CVF comprises at least at least 68 carboxy terminal amino acids of CVF or a fragment thereof, said fragment lacking 1 to 25 carboxy terminal amino acids, and wherein said protein has at least 70 percent identity to said human C3. An alignment of the C3 and CVF sequences is shown in FIG. 1 of US-8,119,769. In addition, without departing from the spirit of the invention, it may be desired for specific purposes, however, to attach additional non-C3 and non-CVF amino acids to the carboxy terminus of the hybrid proteins. Decomplementing activity of C3/CVF hybrid proteins is observed with polypeptides where the C3 alpha-chain is replaced by the corresponding carboxy terminal amino acids of the CVF chain (including the gamma- and the beta-chain of CVF). This construct has a length of 1276 amino acids as processed protein (SEQ ID NO:7 of the instant application). However, due to the immunogenicity of such polypeptides, a higher degree of humanization is preferred. Thus, according to a preferred embodiment, the polypeptides comprise an amino terminal C3 fragment containing the amino acids forming the beta-chain (amino acids 23 to 667 of SEQ ID NO:2 of US-8,119,769) as well as additional amino acids of the C3 chain following at the carboxy terminal end of the beta-chain, i.e. from amino acid 668 towards the carboxy terminus of the peptide. At least 68 amino acids of the C3 sequence are replaced by amino acids of the corresponding CVF sequence. Reference is made in this respect to FIG. 1 of US-8,119,769, showing the alignment of the C3 and CVF sequences. The requirement that the amino acid sequence of the polypeptides of the invention have at least 70% identity to the amino acid sequence of human C3 is intended to ensure that immunogenicity of the hybrid proteins is kept at a tolerable level. The 70% value thus also determines the minimum sequence stretch of the C3 sequence which is required for being combined with the amino acids of the CVF sequence which replace the corresponding carboxy terminal C3 amino acids. It is preferred to provide polypeptides where the identity with the human C3 sequence is at least 80%, or more preferably at least 90% and most preferably at least 95%. According to a preferred embodiment of the invention, the protein or polypeptide, which is a derivative of human complement component C3 (human C3), has an amino acid sequence, which is selected from the group consisting of: a) the sequence shown in SEQ ID NO:6; b) the sequence shown in SEQ ID NO:8; c) the sequence shown in SEQ ID NO: 10; and d) the sequence shown in SEQ ID NO:12, all of which are disclosed in US-8,119,769. The most preferred constructs represented by SEQ ID NO:6 and SEQ ID NO:8 of US-8,119,769 and the identity of the amino acid sequences with human C3 amino acid sequence is approx. 90.7% (91%) for SEQ ID NO:6, and 96.3% (96%) for SEQ ID NO:8. Reference is made in this regard to FIG. 2 of US-8,119,769. After appropriate processing upon recombinant expression, constructs H6 and H5 (represented by SEQ ID NO:6 and SEQ ID NO:8 of US-8,119,769) have both a length of 1637 amino acids (processed H6 construct: SEQ ID NO:8 of the instant specification; processed H5 construct: SEQ ID NO: 9 of the instant specification).

The phrase "sequence identity" refers to the BLAST (R) programm provided at http://blast.ncbi.nlm.nih.gov/Blast.cgi?PROGRAM=blastp&BLAST_P ROGRAMS=blastp&PAGE_TYPE=BlastSearch&SHOW_DEFAULTS=on&LINK_LOC =blasthome in the version online on June 11, 2012.

A "stable" convertase has an extended half-life of its decay-dissociation that is by at least 10%, better at least 100%, more stable than the human C-3 convertase, measured as evident from the Examples provided hereinafter.

"Little or negligible C5-cleaving activity" is preferably an activity of less than 50%, better of less than 20%, than that of the human C-3 convertase, measured as evident from the Examples provided hereinafter.

Human IL-4 has an amino acid sequence according to NP_000580.1. Mouse IL-4 has an aminoacid sequence according to NP_067258.1.

Derivatives of C3 and/or IL-4 may comprise a signal sequence or lack such. An additional tag-sequence, e.g. histag, may be present. The preteins may be truncated at one or both terminals by removal of non-functional sequence parts.

IL-4 and/or IL-13 inhibitors, i.e. inhibitors of decision signals for T-cell differentation, may be of any kind. It is possible to employ small molecules like Oxacyclododeindione or Aspirin®. Further, anti-sense polynucleotides, aptamers, or antibodies directed to IL-4 and/or IL-13 may be designed in a standard manner and applied. Interacting proteins like SOCS-1 (NP_003736.1) may as well be used. Additional reference is made to EP patent application no. 11075261.5, the whole content of which is herewith incorporated by reference. Preferred embodiments, in particular mutants of human IL-4, are specified further in the following.

*A. Inhibitors of decision signals for T-cell differentiation*

For the present invention several principles for local inhibition of the effects of IL-4 and IL-13 are suitable (for a review, see Long, A.A. Monoclonal antibodies and other biologic agents in the treatment of asthma. MAbs 1(3), 237-246; 2009). Preferred principles include but are not limited to the application of antagonistic cytokine derivatives, soluble cytokine receptor constructs, monoclonal antibodies, fragments thereof or other proteinaceous constructs with specificity for IL-4 and Il-13 or their respective receptors, and anti-cytokine and anti-cytokine receptor aptamers. Any compound that functions as an IL-4, IL-13 or IL-4/IL-13 antagonist and is suitable for administration in accordance with the method of the present invention may be employed. Antagonists do not need to completely abolish IL-4- or IL-13-induced biological activity to be useful. Rather, a given antagonist may reduce a biological activity of IL-4 and/or IL-13. Combinations of two or more antagonists may be employed in methods and compositions of the present invention.

Most preferred inhibitors of IL-4 and IL-13 are those which a) provide a small to moderate molecular size and high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects mediated by interaction of the inhibitor with targets away from the site of allergen presentation.

The vast majority of the anti-IL-4 and anti-IL-13 principles which are useful for the therapeutic aims of this invention, have been published and evaluated in animal models as well as in clinical trials for the treatment of asthma (for a review, see Long, 2009). However, application of these inhibitors according to the method of the present invention has not been disclosed.

Many inhibitors of IL-4- and IL-13-mediated pathways including antagonistic cytokine derivatives, soluble cytokine receptor constructs, anti-cytokine and anti-cytokine receptor antibodies have been developed to interrupt or to reduce Th2 dependent allergic inflammation in patients with asthma. The therapeutic aim is a shift of the patient's immune response towards a cytotoxic T-cell-mediated immune response, e.g., characterized by a Th1-type immune phenotype.

In contrast, the therapeutic aim of the present invention is not a shift from a Th2-type immune phenotype to a Th1-type immune phenptype, but the induction of Tregs in a local micro-environment that is free of Th1- or Th2-driving factors despite the presence of allergens and adjuvants eliciting a Th2-type immune response.

In addition to applications for treatment of asthma, patent WO 2009/081201 A2 discloses clinical applications of an IL-4Rα inhibitor for use a) in allergy therapy to suppress IgE synthesis (including for example atopic dermatitis and food allergy), b) for transplantation therapy to prevent transplant rejection, and c) for suppression of delayed-type hypersensitivity or contact hypersensitivity reactions. However, details of the suggested clinical applications are not disclosed.

In general, the rationale in published applications of IL-4 inhibition is a shift of the patient's Th2-type immune response towards a Th1-type immune response. For example, the application of inhibitors of IL-4-mediated pathways for the treatment of cancer is based on the assumption that it is sometimes beneficial for the patient to promote a Th1-type immune response (WO 02/04009 A2). Furthermore, US Patent No 2011/0002013 A1 discloses that IL-4 antagonists find use as adjuvants to allergy immunotherapy and as vaccine adjuvants, especially when directing the immune response towards a Th1 response would be beneficial in treating or preventing the disease in question. Again, details of the suggested application of IL-4 antagonists as adjuvants to allergy immunotherapy are not disclosed.

*B. Inhibition of decision signals for T-cell differentiation by interleukin variants*

In one embodiment of the invention, interleukin variants capable of antagonizing the activity of IL-4 and IL-13 are used as inhibitors. Any interleukin variant that functions as an IL-4, IL-13 or IL-4/IL-13 antagonist and is suitable for administration in accordance with the method of the present invention may be employed. Interleukin-based antagonists do not need to completely abolish IL-4- or IL-13-induced biological activity to be useful. Rather, a given antagonist may reduce a biological activity of IL-4 and/or IL-13. Antagonistic IL-4 mutants have been described (Kruse et al., 1992; Muller et al., 1994; US patent 6,028,176; US patent 5,723,118). Within the four helix bundle of human IL-4, tyrosine (Y124) on the D helix forms an important contact with the common γ-chain ($\gamma_c$). When Y124 is mutated to aspartic acid (Y124D), the resulting molecule binds to IL-4Rα with the same affinity as the wild-type IL-4, but has only 0.5% of the agonistic activity (Letzelter et al., Eur. J. Biochem. 257: 11-20; 1998). Similarly, the arginine at position 121 of human IL-4 interacts with IL-13Rα1. Mutations of R121 generates mutants that can bind to IL-4Rα, but are antagonists of both IL-4- and IL-13-induced responses (Kruse, N., et al., EMBO J. 12: 5121-5129; 1993). However, other or additional mutations of IL-4 may also be considered for generating IL-4 variants suitable for the method of the present invention. For example, by mutation of serine at position 125 of IL-4 mutants have been generated that can bind to IL-4Rα, but are antagonists of both IL-4- and IL-13-induced responses (Kruse et al., 1993). Suitable for the method of the present invention are antagonistic interleukin-4 variants with single, double and triple mutations, single mutations including but not limited to amino acid positions R121, Y124, and S125; double and triple mutations including but not limited to combinations of the above listed amino acid positions. Preferred are antagonistic IL-4 variants with a relatively short half-life to limit adverse effects distant from the site of allergen presentation.

The double mutant of human IL-4 (R121D/Y124D) has been shown to protect allergic cynomolgus monkeys from allergen-induced airways hyper-responsiveness when administered either subcutaneously or via nebulisation. Furthermore, subcutaneous administration of this double mutant in monkeys for 6 weeks or more also reduced the cutaneous wheal response and circulating concentrations of allergen-specific IgE. The effect of the double mutant of IL-4 (R121D/Y124D) on late phase asthmatic response to allergen challenge in asthmatic patients has been evaluated recently in clinical studies (Wenzel, S., et al., Lancet 370: 1422-1431; 2007). The studies demonstrate that dual inhibition of IL-4 and Il-13 can positively affect the course of late asthmatic response after experimental allergen challenge. Treatment with the double mutant was associated with few adverse events, whether administered by subcutaneous injection (up to 30 mg for up to 13 weeks) or by inhalation (up to 60 mg for up to 4 weeks) in participants with atopic asthma or atopic eczema. However, it should be stressed that application of this double mutant for allergy immunotherapy according to the method of the present invention has not been disclosed.

More than 10 years ago animal experiments have demonstrated that inhibition of the IL-4/Il-13 receptor system can completely abrogate humoral immune response to allergen and development of allergic symptoms *in vivo* (Grunewald et al., 1997 and in J. Immunl. 160: 404-4009; 1998). Treatment of BALB/c mice with the murine IL-4 mutant Q116D/Y119D (the murine equivalent of the human IL-4 double mutant R121D/Y124D) before and after immunization with ovalbumin (OVA) completely inhibited synthesis of OVA-specific IgE and IgG1. In addition, the synthesis of specific IgG2a, IgG2b and IgG3 was suppressed, which may indicate the development of tolerance toward OVA. Immunization was performed by i.p. injection of 10 μg OVA in the presence of Alum as an adjuvant. At day 0, the murine IL-4 mutant was applied by i.p. injection 2 hours pre-and post-OVA-immunization as a 50 μg dose each. From day 1 to 8 treatment was continued with 30 μg mutant twice a day by i.p. injection. The authors concluded that the murine IL-4 mutant Q116D/Y119D inhibits antigen-specific humoral immune responses and allergic symptoms mediated either by IgE or IgG1 *in* vivo and, therefore, should be advantageous for therapy of atopic disorders and other Th2-dominated diseases. Although the results of this study are close to the method and the therapeutic aim of the present invention, the study of Grunewald et al. (1998/1999) does not disclose how to provide a high local concentration of inhibitors for a prolonged period of time at the site of allergen presentation associated with a limited radius of inhibitory activity away from the site of allergen presentation due to rapid clearance. Grunewald et al. (1998/1999) report the apparent induction of tolerance to the OVA allergen by starting the application of the murine IL-4 mutant Q116D/Y119D before immunization with OVA, which is not comparable to immunotherapy of patients with an existing allergy. For the induction of tolerance to allergens in individuals with a Th2-type immune phenotype only the method of the present inventions provides appropriate means to ensure a sufficient induction of Tregs in a local micro-environment that is free of Th1- or Th2-driving factors despite the presence of allergens.

IL-13 mutants useful for the method of the present invention include but are not limited to the IL-13 mutant E13K which is a powerful antagonist of human IL-13 and capable of partially inhibiting the responses of human IL-4 as well (Kioi, M., et al., Cell. Immunol. 229: 41-51; 2004.). Glutamate at position 13 in IL-13 associates with IL-4Rα and a mutation to lysine decreases its binding to IL-4Rα. Although IL-13E13K prevents binding of IL-4Rα to IL-13Rα1, full inhibition of IL-4 responses cannot be achieved due to the availability of the alternative IL-4R complex, IL-4Rα:$\gamma_c$.

In a preferred embodiment the human IL-4 double mutant R121D/Y124D is used for inhibition of IL-4- and IL-13-mediated pathways in accordance with the method of the present invention. The human IL-4 double mutant R121D/Y124D is able to antagonize the activity of IL-4 as well as that of IL-13 (Grunewald et al., 1998; Tony, H.P., et al., Eur. J. Biochem. 225: 659-665; 1994).

*C. Inhibition of decision signals for T-cell differentiation by soluble cytokine receptor constructs*

In one embodiment of the invention, soluble cytokine receptor constructs are used for the method of the present invention to inhibit IL-4- and IL-13-mediated pathways. Preferred constructs include but are not limited to the soluble extracellular domain of IL-4Rα, variants, fragments and derivatives thereof that retain the ability to bind IL-4. Human as well as murine IL-4 receptors and their nucleotide sequences have been described (US patent 5,599,905; Idzerda et al., J. Exp. Med. 171(3): 861-873; 1990. (human IL-4 receptor); Mosley, B., et al., Cell 59: 335-348; 1989 (murine IL-4 receptor). The encoded human IL-4 receptor comprises an N-terminal signal peptide, followed by an extracellular domain, a transmembrane region corresponding to amino acids 208 through 231, and a cytoplasmic domain corresponding to amino acids 232 through 800. IL-4 receptor polypeptides arising from alternative mRNA constructs, e.g. which can be attributed to different mRNA splicing events following transcription, and which yield polypeptides capable of binding IL-4, are among the IL-4 receptor polypeptides disclosed herein. IL-4 receptor variants include those having amino acid or nucleotide acid sequences that vary from a native sequence by one or more substitutions, deletions, or additions, but retain a biological activity of the IL-4 receptor protein. The present invention also includes IL-4 receptor proteins with or without associated native-pattern glycosylation. The glycosylation pattern may vary according to the type of host cells in which the protein is produced. Another option is inactivation of N-glycosylation sites by site-directed mutagenesis. Derivatives of the IL-4 receptor protein include those exibiting a different structure as compared to the native protein, having derivatized amino acids (e.g., by cross-linking reagents, or polyethylene glycol).

In another embodiment, the soluble extracellular domain of IL-4Rα, variants, fragments and derivatives thereof that retain the ability to bind IL-4, are conjugated chemically or fused to the N-terminus or C-terminus of other proteins or polypeptides. Preferred IL-4 receptor fusion proteins or polypeptides include but are not limited to peptides facilitating purification (e.g., His-tag) or identification (e.g., Flag peptide), and proteins or peptides that have the property of promoting oligomerization (e.g., leucine zippers). In a preferred specific embodiment, the soluble extracellular domain of IL-4Rα is fused to the Fc portion or selected constant domains of a human immunoglobulin of any isotype, or a fragment thereof capable of dimerization. If fusion proteins are made with both heavy and light chains of an antibody, it is possible to form an oligomer with as many as four extracellular regions of the IL-4 receptor. Preferred constructs include also hetero-oligomers in which both the soluble extracellular domain of IL-4Rα and the soluble extracellular domain of IL-13Rα1 are fused to a human protein or polypeptide capable of oligomerization.

Among cytokine receptor-based inhibitors suitable for the method of the present invention heterodimers containing both the soluble extracellular domain of IL-4Rα and the soluble extracellular domain of IL-13Rα1 are most preferred. The solubilized IL-4 receptor fragment consisting of the extracellular portion of the human IL-4R-α chain (shIL-4R) that competitively inhibits the binding of IL-4 to its receptor, appeared promising in early human studies (Borish, L.C., et al., J. Allergy Clin. Immunol. 107: 963-970; 2001). However, a subsequent large scale clinical trial indicated that this reagent had no clinical efficacy in asthma. This poor response could be a consequence of complex formation of shIL-4R with IL-13Rα1 on the cell surface, thereby stabilizing the weak interaction of IL-13 with IL-13Rα1 leading to enhanced IL-13 responses. In order to inhibit both IL-4- and IL-13-mediated responses, a soluble hetero-dimeric receptor construct has been generated which contains the extracellular domains of IL-4Rα and IL-13Rα1, both fused to the Fc portion of human IgG1 (Economides, A.E., et al., Nature Med. 9: 47-52; 2003). This heterodimeric construct binds IL-4 and IL-13 with high affinity. IL-13 binds to non-complexed IL-13Rα1 with low affinity, but complexes of IL-13Rα1 and IL-4Rα form a high affinity binding site for IL-13 and IL-4 binds even to non-complexed IL-4Rα with high affinity (Andrews, A.-L., et al., J. Immunol. 176: 7456-7461; 2006). Application of a heterodimeric construct providing binding sites for IL-13 and IL-4 for allergy immunotherapy according to the method of the present invention has not been disclosed.

*D. Inhibition of decision signals for T-cell differentiation by antibodies*

In another embodiment, antibodies that function as IL-4 or IL-13 antagonists are employed for the method of the present invention. The antibodies preferably are monoclonal antibodies or antigen-binding fragments thereof. Advantageously, humanized or chimeric monoclonal antibodies are employed. Most preferred are human monoclonal antibodies. Examples of suitable antibodies are those that interact with the binding of IL-4 or IL-13 to an IL-4 receptor or Il-13 receptor, respectively. Such antibodies, referred to herein as blocking antibodies, may be raised against either the cytokines IL-4 and IL-13 or the cytokine receptors IL-4Rα and IL-13Rα1, and screened in conventional assays for the ability to interfere with binding of IL-4 and IL-13 with their respective receptor subunits. Antigen-binding fragments of such antibodies including Fab and F(ab')2 or artificial antibody constructs such as scFv or other proteinaceous constructs such as anticalins are also suitable for the method of the present invention. Additional embodiments include chimeric antibodies and antigen-binding fragments thereof, e.g., humanized versions of murine monoclonal antibodies, and human monoclonal antibodies as well as antigen-binding fragments thereof.

In a preferred embodiment, combinations of two or more humanized or human monoclonal antibodies with specificity for IL-4 and IL-13 which are capable of blocking the biologic activity of both cytokines, are used for the method of the present invention. In accordance with the method of the present invention, the biologic activity of both cytokines needs to be blocked to create a local micro-environment at the site of allergen presentation that is free of Th1- or Th2-driving factors.

Suitable monoclonal antibodies capable of blocking the biologic activity of IL-4 and IL-13 have been reviewed recently (Holgate, S.T., et al., Nat. Rev. Immunol. 8: 218-230; 2008). For example, the anti-IL-4 humanized monoclonal antibody pascolizumab has been shown to effectively block IL-4 responses *in vitro* (Hart, T.K., et al., Clin. Exp. Immunol. 130: 93-100; 2002). Although this antibody yielded unimpressive results in the treatment of asthma, it may be a promising candidate for the method of the present invention. Binding of IL-13 to IL-4Rα can be blocked effectively with the fully humanized monoclonal IgG1 antibody IMA-638 and the interaction of IL-13 with IL-13Rα1 can be inhibited with the fully humanized monoclonal IgG1 antibody IMA-026 (Gauvreau, G.M., et al. The effects of IL-13 blockade on allergen-induced airway responses in mild atopic asthma. Am. J. Respir. Crit. Care Med.; accepted Nov 5 2010, in print.). Both antibodies have shown efficacy in the treatment of asthma in cynomolgus monkeys, but only IMA-638 was able to reduce both early and late phase asthmatic responses in a clinical Phase II study (Gauvreau et al., 2010). Application of such antibodies, fragments and artificial constructs thereof, as well as other anti-cytokine proteinaceous constructs for allergy immunotherapy according to the method of the present invention has not been disclosed.

In another embodiment of the invention, antibodies capable of inactivating cytokine receptors are used to inhibit IL-4- and IL-13-mediated pathways. Preferred are humanized or human monoclonal antibodies with specificity for IL-4Rα which are capable of blocking the biologic activity of both IL-4 and IL-13. For example, the fully human monoclonal IgG2 antibody AMG317 binds with high affinity ($K_d$ = 1.8 x $10^{-10}$ M) to IL-4Rα and potently blocks the biologic activities of both IL-4 and IL-13 (Corren, J., et al., Am. J. Respir. Crit. Care Med. 181: 788-796; 2010). Furthermore, AMG317 has been shown to have important effects on inflammation in vitro. Although in a Phase II study in patients with asthma AMG317 did not demonstrate clinical efficacy across the overall group of patients (Corren et al., 2010), this antibody provides preferred properties for the method of the present invention. However, it should be stressed that application of this antibody and other anti-cytokine receptor antibodies, fragments and artificial constructs thereof, as well as other anti-cytokine receptor proteinaceous constructs for allergy immunotherapy according to the method of the present invention has not been disclosed.

*D. Inhibition of decision signals for T-cell differentiation by aptamers*

In another embodiment of the invention, aptamers are used to inhibit IL-4- and IL-13-mediated pathways. Aptamers are nucleic acid binding species generated by iterative rounds of in vitro selection (for reviews, see Famulok, M., et al., Curr. Top. Microbiol. Immunol. 243: 123-136; 1999.; Yan, A.C., et al., Frontiers Biosci. 10: 1802-1827; 2005). Typically, selected aptamers bind very tightly (often in the low nanomolar range) and specifically to their targets. Aptamers can also discriminate between closely related protein targets. Aptamers have several key features that make them particularly well suited as reagents for the method of the present invention. First, aptamers are relatively small and can readily access sites which are difficult to target with large molecules such as the immunological synapse that is formed between the antigen presenting cells and the T cells. Another advantage for the present invention is the possibility to engineer the stability of aptamers towards degrading nucleases, thereby determining the period of their inhibitory activity. Unmodified RNA- and DNA-based aptamers can begin degrading in serum within minutes, whereas aptamers containing modified bases and phosphorothioate linkages display a significantly increased stability towards degrading nucleases.

For example, 2'-aminopyrimidine-modified RNA has been shown to remain stable for days. Furthermore, modifications can also be applied to the ends of aptamers to confer greater stability. For example, a 3'-3' linkage can be added to prevent 3'-exonuclease degradation. Stable aptamers can be generated by the Spiegelmer-technology (for a review, see Famulok et al., 1999) or by utilizing peptide nucleic acids (EP 1 785 490 B1). Peptide nucleic acids (PNA) represents a class of nucleic acid analogues where the charged deoxyribose-phosphodiester backbone has been replaced by a peptide strand comprising N-(2-aminoethyl)glycine monomers with the nucleobases adenine, thymine, guanine and cytosine attached to their alpha-carbon. Due to its artificial character PNA provides complete resistance against nucleases and proteases.

In a preferred embodiment, combinations of two or more aptamers with specificity for IL-4 and IL-13 which are capable of blocking the biologic activity of both cytokines, are used for the method of the present invention.

In another preferred embodiment of the invention, aptamers capable of inactivating cytokine receptors are used to inhibit IL-4- and IL-13-mediated pathways. Preferred are aptamers with specificity for IL-4Rα which are capable of blocking the biologic activity of both IL-4 and IL-13.

In another preferred embodiment of the invention, aptamers are used which provide limited resistance against enzymatic degradation that is sufficient for effective inhibition of IL-4- and IL-13-mediated pathways at the site of allergen presentation and in its close proximity, but guarantees rapid degradation of the aptamer while diffusing away from this site. In a specific embodiment, the stability of an inhibitory aptamer is adjusted according to the requirement of the method of the present invention by modification of its structure including but not limited to the introduction of phosphorothioate linkages, modification of bases (e.g., by modification with 2'-aminopyrimidine), and addition of a 3'-3' linkage to the end of the aptamer.

In another preferred embodiment of the invention, the physiological clearance of an inhibitory aptamer is adjusted according to the requirement of the method of the present invention. In cases of an extremely fast clearance, the plasma life-time of an inhibitory aptamer can be extended by attachment of lipids or high molecular weight compounds such as 40 kD polyethylene glycol moieties.

## XVIII: Preferred treatment methods

In a method for the modulation of effector T cell responses in patients with type 1 allergy and/or allergic asthma, a composition of the invention is administered prior and during specific immunotherapy in a therapeutically effective dose, wherein at least one recombinant human C3 derivative of the invention and, optionally, at least one inhibitor of IL-4/IL-13-mediated effects, are coated or absorbed or embedded in a matrix of the invention, perferably embedded in a biodegradable thermogelling hydrogel of the invention, and wherein the composit is injected or implanted once or more at or in proximity of the injection or implantation site of one or more allergens adsorbed onto a matrix of the invention, preferably onto aluminium salts.

In a further method for the modulation of effector T cell responses in patients with type 1 allergy and/or allergic asthma, a composition of the invention is administered prior and during specific immunotherapy in a therapeutically effective dose, wherein at least one recombinant human C3 derivative of the invention, optionally at least one inhibitor of IL-4/IL-13-mediated effects, and at least one allergen are coated or absorbed or embedded in a matrix of the invention, perferably embedded in a biodegradable thermogelling hydrogel of the invention, and wherein the composit is injected or implanted.

In a further method for the modulation of autoreactive CD8+ cytotoxic T cell responses in patients with type 1 diabetes, a composition of the invention is administered prior and during self-antigen-specific vaccination in a therapeutically effective dose, wherein at least one recombinant human C3 derivative of the invention is injected or implanted once or more at the site of self-antigen presentation, wherein the presented self-antigen(s) is adsorbed onto a matrix of the invention, preferably onto aluminium salts.

In a further method for the modulation of autoreactive CD8+ cytotoxic T cell responses in patients with type 1 diabetes, a composition of the invention is administered prior and during self-antigen-specific vaccination in a therapeutically effective dose, wherein at least one recombinant human C3 derivative of the invention and, optionally, at least one inhibitor of IL-4/IL-13-mediated effects, are coated or absorbed or embedded in a matrix of the invention, perferably embedded in a biodegradable thermogelling hydrogel of the invention, and wherein the composit is injected or implanted once or more at or in proximity of the injection or implantation site of one or more self-antigens adsorbed onto a matrix of the invention, preferably onto aluminium salts.

In a further method for the modulation of autoreactive CD8+ cytotoxic T cell responses in patients with type 1 diabetes, wherein a composition of the invention is administered prior and during self-antigen-specific vaccination in a therapeutically effective dose, wherein at least one recombinant human C3 derivative of the invention, optionally at least one inhibitor of IL-4/IL-13-mediated effects, and at least one self-antigen are coated or absorbed or embedded in a matrix of the invention, perferably embedded in a biodegradable thermogelling hydrogel of the invention, and wherein the composit is injected or implanted.

In a further method for the modulation of autoreactive CD8+ cytotoxic T cell responses in patients with type 1 diabetes, administration of a composition of the invention prior and during self-antigen-specific vaccination as specified above is combined with systemic antibody-based anti-B cell or anti T cell immunotherapy, preferably with systemic antibody-based anti-CD3 therapy.

LITERATURE

Abe, M., et al. (2001) J. Immunol. 167: 4651-4660.
Akbar, A.N., et al. (2003) Immunology 109: 319-325.
Andersson, A., et al. (1997) Eur. J. Immunol. 27: 1762-1768.
Andrä, J., et al. (2002) Mol. Immunol. 39: 357-365.
Avni, O., et al. (2002) Nat. Immunol. 3 : 643-651.
Ballow, M., Cochrane, C. G. (1969) J. Immunol. 103: 944-952.
Banchereau, J., et al. (2000) Annu. Rev. Immunol. 18: 767-811.
Bao, L., et al. (2009) J. Clin. Invest. 119: 1264-1274.
Bautsch, W., et al. (2000) J. Immunol. 165: 5401-5405.
Bluestone, J.A., et al. (2010) Nature 464: 1293
Calabresi, P.A., et al. (2003) J. Neuroimmunol. 139: 58-65.
Cardone, J., et al. (2010) Nat. Immunol. 11 :862-872.
Carrol, M.C. (2004) Nat. Immunol. 5: 981-986.
Choi, S., et al. (2003) Pharmaceut. Res. 20: 2008-2010.
Cochrane, C. G., et al. (1970) J. Immunol. 105: 55-69.
Coppieters, K.T., et al. (2012) J. Exp. Med. 209: 51-60. Diamyd (2011)

a) Diamyd US phase III trial:

http://clinicaltrials.gov/ct2/show/NCT00751842

b) Diamyd European phase III trial:

http://clinicaltrials.gov/ct2/show/NCT00723411

c) Diabetes prevention - immune tolerance (DIAPREV-IT)

http://clinicaltrials.gov/ct2/show/NCT01122446
?term=diamyd&rank=6

Drouin, S.M., et al. (2001) J. Immunol. 167: 4141-4145.
Drouin, S.M., et al. (2006) Am. J. Respir. Crit. Care Med. 173: 852-857.
Dunkelberger, J.R., Song, W.-C. (2010) Cell Res. 20: 34-50.
Duschl, A., et al. (1992) Eur. Cytokine Netw. 3: 97-102.
Ehirchiou, D., et al. (2007) J. Exp. Med. 204: 1510-1524.
Ezekowitz, R.A., et al. (1984) J. Exp. Med.150: 244-260.
Fang, C., et al. (2009) Blood 114: 1005-1015.
Farrar, C.A., et al. (2006). FASEB J. 20: 217-226.
Fontenot, J.D., et al. (2003) Nat. Immunol. 4: 330-336.
Fritzinger, D.C., et al. (2008a). Mol. Immunol. 45: 4112.
Fritzinger, D.C., et al. (2008b) Adv. Exp. Med. Biol. 632: 293-307.
Fritzinger, D.C., et al. (2009) Dev. Comp. Immunol. 33: 105-116.
Gerard, PN.P., Gerard, C. (2002) Curr. Opin. Immunol. 14: 705-708.
Ghannam, A., et al. (2008) J. Immunol. 181: 5158-5166.
Gilbert, J.C., et al. (1987) J. Control. Release 5: 113-118.
Gong, C.Y., et al. (2009a) Int. J. Pharm. 365: 89-99.
Gong, C.Y., et al. (2009b) BMC Biotechnol. 9: 8.
Grote, A., et al. (2005) NAR 33: W526-531.
Grunewald, S.M., et al. (1997) J. Biol. Chem. 272: 1480-1483.

Gueler, F., et al. (2008) J. Am. Soc. Nephrol. 19: 2302-2312.
Hagenaars, N., et al. (2010) J. Control. Release 144: 17-24.
Hashimoto, M., et al. (2010) J. Exp. Med. 207: 1135-1143.
Hawlisch, H., et al. (2004) Mol. Immunol. 41: 123-131.
Heeger, P.S., Kemper, C. (2012) Immunobiology 217: 216-224.
Hering, B.J., et al. (2004) Am. J. Transplant. 4: 390-401.
Herold, K.C., et al. (2002) N. Engl. J. Med. 346: 1692-1698.
Homann, D., von Herrath, M. (2004) Clin. Immunol. 112: 202-209.
Humbles, A.A., et al. (2000) Nature 406: 998-1001.
Hyun, H., et al. (2007) Biomacromolecules 8: 1093-1100.
Janssen, B.J.C., et al. (2009) EMBO J. 28: 2469-2478.
Jeong, B., et al. (1997) Nature 388: 860-862.
Juedes, A.E., von Herrath, M.G. (2004) Diabetes Metab. Res. Rev. 20 : 446-451.
Kang, Y.M., et al. (2010) Biomaterials 31: 2453-2460.
Karp, C.I., et al. (2000) Nat. Immunol. 1: 221-226.
Karsten, C.M., Köhl, J. (2010) Nat. Immunol. 11: 775-777.
Kawamoto, S., et al. (2004) J. Clin. Invest. 114: 399-407.
Kemper, C., Atkinson, J.P. (2007) Nat. Rev. Immunol. 7: 9-18.
Keymeulen, B. (2005) N. Engl. J. Med. 352: 2598-2608.
Knip, M., et al. (2005) Diabetes 54: S125-S136.
Köhl, J., et al. (2006) J. Clin. Invest. 116: 783-796.
Kölln, J., et al. (2004) J. Immunol. 173: 5540-5545.
Kölln, J., et al. (2005) Immunol. Lett. 98: 49-56.
Kretschmer, K., et al. (2005) Nat. Immunol. 6: 1219-1227.
Kukreja, A., et al. (2002) J. Clin. Invest. 109: 131-140.
Kwan, W.-h., et al. (2012) Immunol. Res. DOI 10.1007/s12026-012-8327-1
Li, K., et al. (2008) Blood 112: 5084-5094.
Li, Q., et al. (2010) J. Am. Soc. Nephrol. 21: 1344-1353.
Lin, M., et al. (2010) Diabetes 59: 2247-2252.
Liu, J., et al. (2008) J. Immunol. 180: 5882-5889.
Ludvigsson, J., et al. (2008) N. Engl. J. Med. 359: 676-781.
MacroGenics press release (October 20, 2011) Pivotal clinical trial of teplizumab did not meet primary efficacy endpoints.
http://www.macrogenics.com/press_releases-284.html
Mathews, K.P. (1980) Ann. Intern. Med. 93: 443-445.
Morgan, B.P., Gasque, P. (1997) Clin. Exp. Immunol. 107: 1-7.
Mulligan, M.S., et al. (1996) J. Clin. Invest.98: 503-512.
Munegowda, M.A., et al. (2011) J. Clin. Immunol. 31: 811-826.
Pai, S.S., et al. (2009). AAPS J. 11: 88-98.
Pavlov, V., et al. (2008) J. Immunol. 181: 4580-4589.
Peng, Q., et al. (2006) J. Immunol. 176: 3330-3341.
Peng, Q., et al. (2008) Blood 111: 2452-2461.
Peng, K.-T., et al. (2010) Biomaterials 31: 5227-5236.
Petrella, E.C., et al. (1987) J. Immunol. Meth. 104: 159-172.
Pratt, J.R., et al. (2002) Nat. Med. 8: 582-587.
Puigbo P., et al. (2007) NAR 35: W126-131.
Qiao, M. et al. (2005) Int. J. Pharm. 294: 103-112.
Raedler, H., et al. (2009) Am. J. Transplant. 9: 1784-1795.
Raedler, H., et al. (2011) Am. J. Transplant. doi:10.1111/j.1600-6143.2011.03561.x.
Razeghifard, M.R. (2004) Prot. Expr. Purif. 37: 180-186.
Reis e Sousa (2006) Nat. Rev. Immunol. 6: 476-483.
Reis e Sousa, et al. (2007) Immunobiology 212: 151-157.
Sakaguchi, S., et al. (1985) J. Exp. Med. 161: 72-87.
Sandor, N., et al. (2009) Mol. Immunol. 47: 438-448.
Shortman, K., Liu, Y.J. (2002) Nat. Rev. Immunol. 2: 151-161.
Sinha, V.R., et al. (2004). Int. J. Pharm. 278: 1-23.

Skyler, J.S., et al. (2005) Diabetes Care 28: 1068-1076.
Stäger, S., et al. (2003) Nat. Med. 9: 1287-1292.
Strainic, M.G., et al. (2008) Immunity 28: 425-435.
Suresh, M., et al. (2003) J. Immunol. 170: 788-794.
Szabo, S.J., et al. (1995) Immunity 2: 665-675.
Taniguchi, S., et al. (1996) Transplantation 62: 678-681.
Till, G.O., et al. (1982) J. Clin. Invest. 69: 1126-1135.
Till, G.O., et al. (1987) Am. J. Pathol. 129: 44-53.
Tolerx, Inc. And GlaxoSmithKline (Novewmber 29, 2011) Phase 3 defend-1 study of otelixizumab in type I diabetes did not meet its primary endpoint. http://www.biospace.com/news_story.aspx?StoryID=21361&full =1
Van Kooten, C., et al. (2008) Mol. Immunol. 45: 4064-4072.
Verschoor, A., et al. (2003) J. Immunol. 171: 5363-5371.
Vieyra, M., et al. (2011) Am. J. Pathol. 179: 766-744.
Viglietta, V., et al. (2004) J. Exp. Med. 199: 971-979.
Vogel, C. W., Müller-Eberhard, H. J. (1982) J. Biol. Chem. 257: 8292-8299.
Vogel, C. W.; et al. (1985). Haematol. Blood Transfus. 29: 514-517.
Vogel, C.W., Fritzinger, D.C. (2007) Curr. Pharm. Des. 13: 2916-2926.
Vogel, C.W., Fritzinger, D.C. (2010) Toxicon 56: 1198-1222.
Walker, M.R., et al. (2003) J. Clin. Invest. 112: 1437-1443.
Willemse, J.L., et al. (2008) Clin. Chim. Acta 389: 181-182.
Wu, Y., et al. (2012) Biomaterials 33: 2351-3260.
Yamazaki, S., et al. (2003) J. Exp. Med. 198: 235-247.
Yiamouyiannis, C.A., et al. (1999) Am. J. Pathol. 154 : 1911-1921.
Zhang, J., et al. (2006) Biomacromolecules 7: 2492-2500.
Zhou, W., et al. (2006) Blood 107: 2461-2469.
Zhou, W., et al. (2007) Mol. Immunol. 44: 57-63.
Zhou, W. (2012) Immunobiology 217 : 225-234.

## EXAMPLES

The following examples are intended to illustrate but not limit the present invention.

EXAMPLE 1. Tissue culture and recombinant expression

1.1. Cell culture

COS-7 cells, HEK293 and CHO cells are cultured in an incubator (Heraeus Instruments Begasungsbrutschrank 6060) in a water saturated atmosphere (5 % $CO_2$) at 37°C. Growth is performed in DMEM medium (Gibco/BRL, Eggenstein, Germany) which is supplemented with 10% fetal calf serum (Biochrom, Berlin, Germany). As soon as cells grow confluently in the tissue culture flasks (75 $cm^3$, Cellstar, Greiner Labortechnik, Frickenhausen, Germany), they are passaged into a new culture flask (approximately every 3 days). The cell supernatant is removed and the cells are washed with phosphate-buffered saline. After the addition of 4 μl trpysin/EDTA (Gibco BRL, Eggenstein, Germany), cells are incubated for 5 min in an incubator. Detachment of the cells from the bottom is suported by gentile shaking and is controlled under the microscope. When the cells are almost completely detached, the procedure is stopped by adding 8 ml serum-containing medium. The suspension is transferred into a 15 ml-reaction tube, and the cells are sedimented by centrifugation (5 min, 1,000 x g, RT). The supernatant is removed, the pellet is resuspended in 10 ml serum-containing medium and 1 to 3 ml of the cell suspension are transferred into a new tissue culture flask and supplemented with serum containing medium to give a final volume of 13 ml.

Drosophila S2 cells are cultered in serum-free media (Hi-Five plus glutamine, Invitrogen) in the absence of blasticidin as described (Fritzinger et al., 2009).

### 1.2. Transfection

*1.2.1. Transfection of mammalian cells.* For expression in mammalian systems, expression vector pcDNA3 (Invitrogen, Leek, the Netherlands) comprising the corresponding genes is introduced into the cells by the GenePorter reagent (PeqLab, Erlangen).

DNA (1 to 4 μg) and 10 to 15 μl GenePorter transfection reagent are diluted in 500 μl serum-free medium and pooled. The reaction mix is incubated for 45 min at room temperature. The cells which have been passaged in a 6-well plate (300-500 μl of cell suspension with 2 ml serum-containing medium per well; TC-plate, 6-well, Greiner Labortechnik, Frickenhausen, Germany), are washed with phosphate buffered saline, and then the DNA GenePorter mixture is carefully added drop-wise to the cells. After 3 hours in the incubator, the medium is replaced by 2 ml serum-containing medium, and the cells are kept for 2 to 3 days in the incubator for cell growth. When using Nutridoma HU (100x; Roche Diagnostics, Mannheim, Germany), the cells are added to Nutridoma HU after transfection in serum-free medium and kept growing in the incubator for 2 to 3 days. A reaction mixture without DNA is prepared as negative control. If applicable, a reaction mixture with 1 μg pEGFP-N1 is prepared as positive control. Plasmid pEGFP-N1 encodes the green fluorescence protein (GFP) and can be used for determining transfection efficiency since the GFP expressing cells can be detected using a fluorescence microscope. The supernatant of the pEGFP-N1 transfected cells is removed after 24 hours, cells are washed with 2 ml PBS, and 500 μl trypsin/EDTA is added. Detachment of cells is performed for 5 min in the incubator. Then, 2 ml phosphate-buffered saline is added and the cell suspension is transferred into a 50 ml reaction tube for centrifugation (5 min., 1,000 x g, RT). The supernatant is removed and the cell pellet is resuspended in 2 ml phosphate-buffered saline. 10 μl thereof are provided in a Neubauer-counting chamber, and the number of fluorescening cells and the total number of all cells are counted in the outer four squares.

Calculation of the cell number/ml:

$$\text{Cells/ml} = \frac{\text{Number of cells of all squares}}{4} \times 10^4$$

Calculation of transfection efficiency:

$$\text{Efficiency (\%)} = \frac{\text{number of fluorescent cells}}{\text{number of cells of all squares}} \times 100\%$$

*1.2.2. Transfection of insect cells.*

Transfection of *Drosophila* S2 cells is performed as described (Fritzinger et al., 2009). Briefly, *Drosophila* S2 cells are transfected with a mixture of the expression plasmid and pCoBlast (ratio of 19:1 w/w) using the calcium phosphate method.

**1.3. Expression under selection pressure**

In order to increase yields, stably expressing mammalian lines are kept in culture containing an antibiotic. Depending on their resistance, 10 μl/ml culture medium is added to an antibiotic stock solution (G418), or 5 μl/ml culture medium is added to zeocine.

**1.4. Expression in serum or protein-free medium**

For culturing in serum-free or protein-free medium, cells are step-wise adapted with SCF30- or Mampf3-medium (Promocell, Heidelberg, Germany) with 1 mM L-glutamine. The percentual portion of the serum-free or protein-free medium is increased by 25 % in every second passage.

**1.5. Monoclonalization**

In order to obtain a homogeneous cell population, monoclonalization is conducted, wherein the different cells are monoclonalized, expanded and subsequently examined with respect to their expression level by Western blotting and immuno printing.

For this purpose, all cells are incubated for 3 to 4 passages after transfection under selection pressure in order to guarantee that a large portion of cells contains the resistance and therefore expresses the target protein. This procedure allows to adjust the cell number per ml, which is counted thereafter using a Neubauer-counting-chamber, with the number of cells expressing the target protein. Subsequently, cell density is set to 1 cell per 100 μl medium with 10% FCS, and 100 μl of this dilution are introduced into each of the 48-96 wells of the 96 well plates (Greiner Labortechnik, Frickenhausen, Germany). After approximately one week, the medium is removed, and 100 μl medium with 10% fetal calf serum is added. After approximately 2 weeks, the wells are examined under the microscope for one colony per well. Some are selected, and the cells are detached with 25 μl trypsin for 5 min at 37°C and completely transferred into the well of a 24-well-plate (Greiner Labortechnik, Frickenhausen, Germany), which is completely filled with 500 μl medium containing 10 % fetal calf serum. After one week, this precedure is repeated with 100 μl trypsin, and all cells are placed into a well of a 6-wellplate. After another 3 to 4 days, 100 μl supernatant per well are taken and examined by Western blotting and subsequent immunoprinting. The cell population, which finally shows the strongest band, is further expressed under selection pressure and cryoconserved, if applicable.

EXAMPLE 2. Determination of recombinant protein concentrations 2.1 Densitometric determination

For densitometric determination of protein concentrations, 4-5 dilutions of known concentration of native CVF or human C3 (depending on the sample) are applied in addition to different volumina of the protein-containing sample to be determined. The protein concentration in the calibration series is adjusted to the expected concentration range of protein in the sample. The gel is subjected to wet-blotting or semidry-blotting procedures and the proteins are subsequently stained using immunoprinting. Then, the membrane is scanned and the concentration of the sample is determined using the program *Imagemaster* 1D *Elite Version 2.01* (Amersham Pharmacia Biotech, Freiburg, Germany).

## 2.2. Determination by ELISA

For the determination of recombinant proteins by ELISA, approximately 1 μg protein diluted in 100 μl incubation solution, is immobilized on the well of an ELISA plate overnight at 4°C. Subsequently, the wells of the plate are washed three times with washing buffer (200 μl). Then, the wells are blocked with 200 μl 5 % milk powder in phosphate-buffered saline for 5 hours at room temperature. After washing three times, 200 μl of the supernatant of transient expression or purified diluted proteins in phosphate-buffered saline, respectively, are introduced into the wells and incubated overnight under agitation at 4°C. After this, the samples are washed three times with washing buffer and incubated with 100 μl of a 1:1000 dilution of the respective antibody in 2,5 % milk powder in phosphate buffered saline at room temperature. Subsequently, the samples are washed three times and incubated for one hour with 100 μl of a 1:1000 dilution of an respective peroxidase-conjugate in a 2,5 % milk powder in PBS at room temperature. Finally, the samples are washed three times, and then 100 μl detection buffer (for POD) are added. After staining the wells, extinction at 404 nm is measured with a micro titer plate-photometer (ELISA-Reader, SLT-Instruments, Grödingen, Austria, Easy Reader EAR 400AT).

## 2.3. Determination by BCA assay

Quantification of purified recombinant proteins is performed with the bicinchoninc acid (BCA) assay using the BCA™ Protein Assay Kit (Pierce, USA).

## EXAMPLE 3. Analysis of C3 cleavage activity

This assay determines the activity of C3/C5 convertases formed by rhC3-derivatives or CVF (as control) to activate human C3 by cleaving off the C3a peptide. To analyze C3 cleaving activity, a C3 convertase is pre-formed by incubation of rhC3-derivatives at a concentration of 1 μM in the presence of a three-fold molar excess of Factor B, 0.5 μM Factor D and $MgCl_2$ at 37°C for up to 24 hours. Controls include CVF and EDTA (Vogel and Müller-Eberhard, 1982). The convertase formation is stopped by the addition of EDTA (final concentration 5 mM) und purified human C3 is added. The reaction mixture is incubated at 37°C for one hour or any other appropriate period of time. Aliquots are taken and immediately transferred into an ice water bath to stop further C3 activation. C3 cleavage is monitored by 7.5% (w/v) SDS polyacrylamide gel electrophoresis (SDS PAGE) under non-reducing conditions analyzing the disappearance of the C3 α-chain and appearance of the C3 α'-chain. Subsequent western blots may be performed to confirm the results obtained by SDS PAGE.

**EXAMPLE 4. Analysis of C5 cleavage activity**

**4.1. Bystander lysis assay**

This test for detecting hemolytic activity is based on fluid C5-activation and can be determined by lysis of non-sensitized guinea pig erythrocytes (Vogel et al., 1985). In this method, the extent of complement activation is determined by photometric measurement of released hemoglobin.

*Isolation of guinea pig erythrocytes*

Approximately one ml blood, obtained from isofluoran-narcotized guinea pigs by punction of the eyes, is taken and immediately transferred into a tube containing 1 ml ice-cold ACD solution. The ACD solution serves as anti-coagulant. The erythrocytes are separated by centrifugation (1,000 x g, 4 min, 4°C), resuspended in 14 ml GVBS$^{++}$ and again centrifuged (1,000 x g, 4 min., 4°C). This procedure is repeated 3 to 4 times until the supernatant remaines clear. Finally, the erythrocytes are resuspended in approximately 5 ml GVBS$^{++}$. The erythrocytes are diluted with GVBS$^{++}$ until 30 μl of the erythrocyte suspension in 1 ml $H_2O$ gives an absorption of 1.9 (5 x $10^8$ cells/ml) at 412 nm.

*Bystander lysis*

Different amounts of the protein to be analyzed are diluted in 20 μl GVBS$^{++}$ mixed with 20 μl guinea pig serum (Sigma, Taufkirchen, Germany) and 20 μl guinea pig erythrocytes (5 x $10^8$ cells/ml) in a 2 ml reaction tube and incubated for 3 hours at 37°C under agitation (Thermomixer 5436, Eppendorf, Hamburg, Germany). The reaction is stopped by adding 1 ml ice-cold VBS$^{++}$-buffer. The erythrocytes are centrifuged (2,000 x g, 4°C, 2 min) and released hemoglobin in the supernatant is determined by measurement of extinction at 412 nm. Reaction mixtures with 20 μl erythrocytes and 40 μl $H_2O$ (complete lysis) or 20 μl guinea pig serum, 20 μl erythrocytes and 20 μl GVBS$^{++}$ (serum control), respectively, serve as controls.

**4.2. Assay for C5 cleavage activity**

This assay performed as described by Petrella et al. (1987) determines the activity of C3/C5 convertases formed by rhC3-derivatives or CVF (as control) to activate human C5 by cleaving off the C5a peptide. The C5 convertase is pre-formed using a rhC3-derivative (3 μg) and human Factor B and Factor D as described in section 1.6. After convertase formation, the reaction is stopped by the addition of EDTA to a final concentration of 5 mM. Then 5 μl of this raction mixture is added to 25 μl containing 7 μg purified human C5 in phosphate-buffered saline. After incubation at 37°C for 24 hours, the reaction is stopped by the addition of 7 μl Laemmli gel loading buffer followed by boiling for 5 min. C5 cleavage is monitored by 7.5% (w/v) SDS polyacrylamide gel electrophoresis (SDS PAGE) under non-reducing conditions analyzing the disappearance of the C5 α-chain and appearance of the C5 α'-chain. Subsequent western blots may be performed to confirm the results obtained by SDS PAGE.

**EXAMPLE 5. Assay for Factor B cleavage activity**

To detect cleavage of Factor B into Ba and Bb, rhC3-derivatives are incubated at a concentration of 1 μM in the presence of a three-fold molar excess of Factor B, 0.5 μM Factor D and $MgCl_2$ at 37°C for up to 24 hours. Controls include CVF and EDTA (Vogel and Müller-Eberhard, 1982). The reaction mixtures are analyzed by 7.5% (w/v) SDS polyacrylamide gel electrophoresis (SDS PAGE) under non-reducing conditions to monitor the disappearance of Factor B and the appearance of the cleavage products Ba and Bb. Subsequent western blots may be performed to confirm the results obtained by SDS PAGE.

**EXAMPLE 6. Analysis of complement depletion**

**6.1. Complement consumption assay**

The complement consumption assay is based on the complement consuming effect of rhC3-derivatives or CVF. If a rhC3-derivative- or CVF-containing sample is incubated with human serum, the complement proteins are consumed depending on the activity of the rhC3-derivative or CVF. The remaining complement activity of the serum can be detected using sensitized sheep erythrocytes (Ballow and Cochrane, 1969; Cochrane et. al., 1970).

*Preparation of sensitized sheep erythrocytes.*

Sheep whole blood (1 ml, Behringwerke, Marburg) is resuspended in 13 ml cold $GVBS^{++}$ and centrifuged for 10 min at 1,000 x g (4°C). The supernatant is removed and the erythrocytes are again resuspended in 14 ml $GVBS^{++}$. This procedure is repeated until a clear supernatant is obtained. Subsequently, the erythrocytes are resuspenden in approx. 5 ml $GVBS^{++}$. Erythrocytes are adjusted by dilution with $GVBS^{++}$ to give an absorption of 1.9 at 412 nm for 30 $\mu$l erythrocyte suspension in 1 ml $H_2O$ corresponding to a density of 5 x $10^8$ cells/ml. After the addition of 2 $\mu$l antiserum against sheep erythrocytes (anti sheep red blood cell stroma, Sigma, Taufkirchen) per ml of the adjusted erythrocytes, sensitizing is performed for 1 h in a water bath at 37°C, while inverting the reaction tube regularly after 10 min. The sensitized erythrocytes are washed three times with 2 ml $GVBS^{++}$ and centrifuged for 3 min at 1,000 x g (4°C). The erythrocytes can be stored up to three days. Prior to each use, $OD_{412}$ is adjusted to 1.9.

*Complement consumption.*

First, quantification of human serum is performed by serum titration to achieve hemolysis of sheep erythrocytes by 70-90%. For this purpose, different serum concentrations (serum value) in 2 ml reaction tubes are filled up to 40 $\mu$l with $GVBS^{++}$. Additionally, controls are prepared, which contain 40 $\mu$l $GVBS^{++}$ (buffer control) only, or 40 $\mu$l $H_2O$ (complete lysis) only. All reaction mixtures are incubated for 30 min at 37°C under agitation (Thermomixer 5437, Eppendorf, Hamburg, Germany). Then, 100 $\mu$l cold $GVBS^{++}$ or 100 $\mu$l $H_2O$ (complete lysis) and 30 $\mu$l sensitized sheep erythrocytes are added and the reaction mixtures are incubated for 30 min as described above.

Subsequently, the samples are kept on ice and 850 $\mu$l cold $VBS^{++}$ or 850 $\mu$l $H_2O$ (complete lysis) are added. The supernatants are transferred into cuvettes, and optical density is measured at 412 nm. Hemolysis is calculated according to the following formula:

$$\%\text{hemolysis} = \frac{OD_{412}\ \text{serum value} - OD_{412}\ \text{buffer control}}{OD_{412}\ \text{complete lysis} - OD_{412}\ \text{buffer control}} \times 100\%$$

For complement consumption assay, the quantity of serum determined in prior tests and the samples (max. 20 $\mu$l) are supplemented with $GVBS^{++}$ to give 40 $\mu$l. Additionally, reaction mixtures of the following controls are prepared: determined quantity of serum, supplemented with $GVBS^{++}$ to give 40 $\mu$l (serum control, 4 to 5 samples); 40 $\mu$l $GVBS^{++}$ (buffer control) and 40 $\mu$l $H_2O$ (complete lysis). All reaction mixtures are incubated for 3 hours at 37°C under agitation. After the addition of 100 $\mu$l cold $GVBS^{++}$ or 100 $\mu$l $H_2O$ (complete lysis) and 30 $\mu$l adjusted sensitized sheep erythrocytes, the reaction mixtures are incubated for 30-40 min as described above. After 15 min, the serum control as well as the complete lysis control are measured according to the following principle. The samples are kept on ice, 850 $\mu$l cold $VBS^{++}$ or 850 $\mu$l $H_2O$ for complete lysis are added and centrifuged (4°C, 2,000 x g, 2 min.). Supernatants are transferred into cuvettes and the optical density is measured at 412 nm. Lysis is calculated according to the following formla:

$$\%\text{hemolysis} = \frac{OD_{412}\ \text{serum control} - OD_{412}\ \text{buffer control}}{OD_{412}\ \text{complete lysis} - OD_{412}\ \text{buffer control}} \times 100\%$$

In case the value for the serum control is clearly below 80% of the complete lysis value, a second serum control is taken after 10 further minutes of incubation and measured as described above. Once a value of 70-80 % hemolysis is obtained, all reaction mixes are measured and evaluated according to the same principle. In order to facilitate comparison of different series of measurements, values are referred to the corresponding serum control.

### 6.2. Solid phase complement consumption assay

For characterization of complement consuming activity of the recombinant strep-tagII-rhC3-derivatives, a solid phase assay is performed as described in US patent 7,553,931. The assay comprises immobilizing of the rhC3-derivatives as Strep-tagII fusion proteins to Strep-Tactin which is bound to the surface of ELISA plates. The subsequent addition of buffer and serum facilitates the conduction of the complement-consumption-assays in the ELISA plate. For immobilization, a Strep-tagII is selected which is a peptide consisting of 8 amino acids (WSHPQFEK).

In a first step, 3 μg strep-tactin (diluted in 40 μl incubation solution) is immobilized overnight at 4°C on the surface of the wells of an ELISA plate (Greiner, Frickenhausen, Germany). Then, the wells are washed 3 times with 200 μl washing buffer and subsequently blocked with 200 μl 3 % bovine serum albumin in phosphate-buffered saline for 5 hours at room temperature. After washing the wells again (3 times), different volumes of the supernatants or of the samples are provided in the wells. Protein concentration in the supernatants is determined densitometrically. After overnight incubation at 4°C under agitation, the wells are washed 3 times with washing buffer. Subsequently, $GVBS^{++}$ and the amounts of serum determined in serum titration are added, giving a volume of 60 μl. The ELISA plate is sealed with paraffin and fixed to the bottom of an incubation shaker. Subsequently, incubation is performed for 3 hours at 37°C, 150 rpm. Supernatants are transferred into 2 ml reaction tubes, and following addition of 100 μl $GVBS^{++}$ and 30 μl sensitized erythrocytes the complement consumption assay is conducted as described.

## EXAMPLE 7. Determination of the stability of C3 convertases

500 ng of each native CVF (nCVF), hC3, or rhC3-derivative are mixed with 950 ng of factor B and 8 ng of factor D in a volume of 60 μl VBS (2.5 mM Na-5,5-diethyl-barbituric acid, 143 mM NaCl, pH 7.4). After addition of $MgCl_2$ to a final concentration of 10 mM, the samples are incubated for 2 h at 37° C to allow for convertase formation. Subsequently, all samples are supplemented with EDTA to a final concentration of 10 mM to inhibit further formation of convertases. Thereafter, the samples are incubated at 37° C and after different periods of incubation 20 μl aliquots are added to 150 μM Boc-Leu-Gly-Arg-7-amido-4-methylcoumarin acetate (Sigma, Taufkirchen, Germany) in 180 μl VBS. The time-course of fluorophore release is determined in black FIA-Plates (96 K, Greiner Bio-One, Frickenhausen, Germany) using an excitation filter of 370 nm and an emission filter of 465 nm in a microplate reader (Genios, Tecan, Creilsheim, Germany). Values after 60 min of fluorophore release are defined as 100 %. The slope of the graph is used to determine the enzymatic activity of the sample.

## EXAMPLE 8. Expression and purification of CVF and human C3

For the expression of CVF plasmids pUC18CVF and pcDNA3CVF (both contain the cDNA of CVF; Genebank Accession No. U09969) were employed and for the expression of human C3 plasmids pUC18hC3 and pcDNA3hC3 according to US patent 7,553,931 B2. The expression of recombinant CVF and human C3 was performed as desribed in Example 1.

For analyses via solid-phase-assays a Strep-tagII sequence was fused to the N-terminus of both molecules. Using the CVF-cDNA two amplification products were generated in two PCR reactions and the amplification products were hybridized by PCR as described in US patent 7,553,931 B2. Furthermore, an enterokinase-cleavage site was inserted into the cDNA of CVF and human C3 between the signal sequence and the N-terminus of the Strep-tagII to allow for cleavage of the affinity tag.

For separation of low molecular components of the culture supernatants or column fractions, the supernatants or fractions were transferred into a dialysis tube (SpectraPor CE 100, MWCO 100 kDa, Roth, Karlsruhe, Germany) and dialysed overnight against phosphate-buffered saline. The dialysed samples were concentrated with Centricon-units (MWCO 100 kDa, Millipore, Eschborn, Germany). For this purpose, centrifugation was performed at 1,000 x g (4°C) until the desired final volume was reached.

For purification the supernatant (2.5 ml) from transient expression was loaded onto a PD-10-column according to the manufacturer's instructions (Amersham Pharmacia Biotech, Freiburg, Germany). PBS was used as a running buffer. The fractions were examined for recombinant protein by Western blotting and subsequent immunoprinting. Alternatively, 2 ml supernatant of the transient expression were loaded onto a 1 ml EconoPac-column (Biorad, Munich, Germany) using a Tris-buffer (50 mM, pH 7.5). The same buffer containing 500 mM NaCl was used for elution. The fractions were also examined for protein content and dialysed against PBS.

For control purposes, natural CVF was also purified from lyophilized Indian cobra (Naja kaouthia) venom as described (Janssen et al., 2009).

### EXAMPLE 9. Cloning of rhC3-derivative H5

The rhC3-derivative H5 shown in Fig. 1 was constructed as described in US patent 7,553,931. The derivative contains the human β-chain and additionally the humanized Factor B- and Factor H-binding sites as well as the cleavage sites for Protease Factor I. In addition to the α-chain, the γ-chain as well as the C3a and the C3d regions were humanized.

For cloning of rhC3-derivative H5, a fragment consisting of pUC18 and the 3'terminus of CVF was obtained from pUC18CVF utilizing Ecl136I and BglII restriction. Subsequently, said fragment was ligated with a fragment obtained from pcDNA3hC3 by EcoRI restriction, followed by mung bean nuclease digestion and BglII restriction. The latter fragment had a size of 1870 bp contained the 5' terminus of C3 cDNA. The vector ligated in this way (referred to as H2Δ2307 bp) contained 1800 bp of the C3 5' terminus and 1000 bp of the CVF 3' terminus.

In order to completely construct rhC3-derivative H5, vector H2Δ2307bp was digested with BglII. The middle region of the C3-cDNA was isolated from plasmid pUC18hC3 via its BglII restriction sites and inserted into the vector. The resulting rhC3-derivative H5 was then inserted into an analogously digested pcDNA3-vector via the EagI-restriction sites. Subsequently, a Strep-tag was inserted between the signal sequence and the N-terminus as described in US patent 7,553,931.

### EXAMPLE 10. Expression and purification of rhC3-derivative H5

Expression of rhC3-derivative H5 in CHO cells was confirmed by ELISA via Strep-Tactin, immunoblot analysis, and densitometry. All methods provided yields of 1-2 mg/l.

For quantification by immunoblot polyclonal serum against human C3 was employed. Since rhC3-derivative H5 has 90% identity compared to human C3, it was assumed that the polyclonal serum detects both proteins with a variance that is lower than the one of densitometric quantification.

Determination of the concentration was confirmed by a sandwich ELISA utilizing an immobilized monoclonal C3d-antibody or the immobilized antibody fragment C3-1. After incubation of the immobilized antibodies with the recombinant proteins, the detection was performed using a polyclonal C3-antiserum. In addition to the samples, various concentrations of human C3 were employed. The evaluation of the ELISA analysis confirmed the concentrations obtained from the densitometric quantification.

For purification, supernatant (500 ml) obtained from stably transfected cells was adjusted to pH 7.5, passed trough a 0.45 μm cellulose acetate membrane and loaded onto a Poros HQ/M anion exchange column equilibrated with 50 mM Tris, pH 7.5 using ÄKTA purifier (Amersham Bioscience, Freiburg, Germany). The recombinant protein was eluted using a linear (0-500 mM) NaCl gradient. Fractions (2 ml) were analyzed using 7.5% SDS-PAGE and western blotting, pooled and dialyzed against phosphate buffered saline (PBS). The pooled sample was diluted (1:9) in 50 mM sodium phosphate, 0.55 M sodium sulfate buffer, pH 7.0, filtered (0.2 μm) and applied to a thiophilic resin (1.5 ml, BD Bioscience, Heidelberg, Germany) equilibrated with 50 mM sodium phosphate, 0.5 M sodium sulfate buffer, pH 7.0. After extensive washing of non-adsorbed proteins with the equilibration buffer (> 30 column volumes), elution was performed using 50 mM sodium phosphate buffer, pH 7.0. Fractions (1.5 ml) were analyzed by 7.5% SDS-PAGE and western blotting. Fractions containing H5 were pooled, dialyzed against 100 mM Tris, 150 mM NaCl, pH 8.0 (buffer W), loaded onto Strep-Tactin sepharose (2 ml, IBA, Göttingen, Germany) equilibrated with buffer W, washed with 10 ml buffer W, and eluted with buffer W containing 2.5 mM desthiobiotin. Protein concentration and purity of the fractions were analyzed by 7.5% SDS-PAGE. Pooled fractions were dialyzed against PBS and employed for further characterization.

### EXAMPLE 11. Characterization of rhC3-derivative H5

Following successful transient expression, rhC3-derivative H5 was used in a solid phase-assay, where CVF and human C3 served as controls (Fig. 2). Hybrid H5 clearly showed complement-consuming activity which is comparable to CVF. Despite of its degree of 90% humanization, hybrid H5 completely retains complement-consuming activity.

To analyze the functional activity of rhC3-derivative H5 more in detail, we established a stably transfected CHO cell line and purified the protein using thiophilic and strep-tactin resins. A detailed analysis of the complement-consuming activity of H5 over a broad concentration range confirmed the CVF-like activity observed in analyses of transiently expressed rhC3-derivative H5. As evident from Fig. 3, rhC3-derivative H5 exhibits approx. 85% of the complement-consuming activity of purified native CVF.

Furthermore, we analyzed the formation and stability of the convertase generated by the rhC3-derivative H5 (for details, see Example 4. The recombinant protein activates factor B by producing Bb and Ba in the presence of factor D and $Mg^{2+}$ in an identical manner as $C3(H_2O)$ and CVF. The time-dependent reduction in the release of 7-amido-4-methylcoumarin from a fluorogenic substrate analogue by the action of the convertase revealed a half-life of the rhC3-derivative H5-dependent convertase of approx. 5-6 hours (Fig. 4), which is close to the reported 7 hour half-life of the CVF-dependent convertase (Vogel und Müller-Eberhard, 1982). In contrast, the C3b,Bb convertase complex exhibited no activity, thereby confirming the extremely short half-life in the range of 1 to 2 minutes (Medicus et al., 1976). These results confirm the fact that, in addition to the CVF-$\alpha$-chain, also the CVF-$\gamma$-chain as well as the C3a- and C3d-homologous regions can be substituted by human C3 without loss of complement-consuming activity.

**EXAMPLE 12. Cloning of rhC3-derivative H6**

The rhC3-derivative H6 shown in Fig. 5 provides 96.3% identity to human C3. For the construction of this derivative, the Bsp1407I-restriction site was used and the region upstream of the Bsp1407I-restriction site was humanized as described in US patent 7,553,931 B2. For cloning of rhC3-derivative H6, a 350 bp-fragment from pUC18CVF* containing the 3'-terminus of CVF was amplified by PCR. The amplification product and the vector pcDNASt-hC3 were digested with Bsp1407I and XbaI and ligated.

The rhC3-derivative H6 was purified according to established techniques using affinity-chromatographical methods on the basis of the His-tag-system. For this purpose, the insertion of the His-tag was performed in analogy to the insertion of a Strep-tag between the signal sequence and the N-terminus. Briefly, two amplification products were generated as described in US patent 7,553,931B2. The amplification products were hybridized by PCR followed by insertion via the restriction sites NotI and Bpu1102I into a vector digested in an analogous manner. Subsequently, a Strep-tag was inserted between the signal sequence and the N-terminus as described in US patent 7,553,931 B2.

**EXAMPLE 13. Expression and purification of rhC3-derivative H6**

The successful transient expression of rhC3-derivative His-H6 in CHO cells was verified in a sandwich-ELISA via Strep-Tactin and by immunoblot analysis employing polyclonal serum against human C3. Densitometric quantification of these immunoblot analyses showed yields of 1-2 mg/l.

For purification of rhC3-derivative H6, stably expressing CHO-cells were monoclonalized and expanded. Subsequently, imidazole was added to the supernatant of the CHO cells in a final concentration of 20 mM and the mixture was incubated with Ni-NTA-matrix. The elution fractions were then analyzed by immunoblot. The fractions were pooled, dialyzed and quantified in a Sandwich-ELISA. Protein concentrations of 3-4 $\mu$g/ml were determined. Densitometric determinations of immunoblot analyses confirmed the quantification by ELISA. Analysis by SDS-PAGE and silver staining revealed a strong protein background in the purified fractions and insufficient binding of rhC3-derivative H6 to the Ni-NTA-matrix.

**EXAMPLE 14. Characterization of rhC3-derivative H6**

Fig. 6 shows the complement-consuming activity of a supernatant containing Strep-tagII-rhC3-derivative H6 in a solid phase-assay. As evident from the analysis, the complement-consuming activity of rhC3-derivative H6 is retained but slightly lower than that of CVF.

For further characterization of rhC3-derivative H6, the C3-convertase activity was also determined in a complement consumption assay in solution (Fig. 7). The data of this analysis show that rhC3-derivative H6 has approximately 68% of the complement-consuming activity of CVF.

Subsequent analyses in a *Bystander Lysis-assay* (see Example 1.5.) revealed that rhC3-derivative H6 does not exert significant fluid phase C5-convertase activity as compared to CVF.

**EXAMPLE 15. Cloning of rhC3-derivative HC3-1496**

The rhC3-derivative HC3-1496 provides 94% identity to human C3. Cloning of this derivative is performed according to the method described in patents PCT/US2005/05119 and US2010/0179092. Briefly, two PCR reactions are performed using pBS-HuC3-2 in the first reaction as template and following oligonucleotides as primers: TCTGTGTGGCAGACCCCTTCGAGG (forward) and GAGAAGGCCTGTTCCTTTA-TCCGGATGGTAGAACCGGGTAC (revers). In the seceond reaction pCVF-FL3Δ is used as template and following oligonucleotides as primers: CCGGTTCTACCATCCGGATAAAGGAACAGGCCTTC (forward) and CATCCATGACATAGATA-TCATTACCATCTTG (revers). The resulting two PCR products are joined in a PCR reaction and digested with NspV after purification with a Quiagen PCR cleanup kit. Thereafter, the sequence is cloned into pHC3-1550(-sig) treated with NspV and calf intestine alkaline phosphatase. For expression the insert from the resulting plasmid, called pHC3-1496, is isolated and cloned into pMT-Bip/V5-HisA (Invitrogen).

**EXAMPLE 16. Expression of rhC3-derivative HC3-1496**

Expression of rhC3-derivative HC3-1496 is performed in the *Drosophila* S2 cell system as described in US2010/0179092. Briefly, *Drosophila* S2 cells are transfected with a mixture of the expression plasmid and pCoBlast (ratio of 19:1 w/w) using the calcium phosphate method. Thereafter, cells containing both plasmids are selected using blasticidin (25μ/ml). For expression, transfected cells are grown in serum-free medium (Hi-Five plus L-glutamine) in the absence of blasticidin. After reaching a density of 5 x $10^6$ cells/ml production of rhC3-derivative HC3-1496 is induced by the addition of $CuSO_4$ (final concentration 25 μM) and continued for 4-5 days.

Purification of rhC3-derivative HC3-1496 from the media is performed as described (Fritzinger et al., 2009). Cells from 1 L cultures are removed by centrifugation (5 min at 1000 x g), and the media filtered through a 0.45 μm filter to remove any cellular debris. The filtered media are diluted by the addition of an equal volume of 20 mM Tris-HCl, pH 8.0, and applied to a 20 ml ANX-FF-column, and protein is eluted with a linear gradient from 0 to 0.6 M NaCl in the same buffer. Fractions containing rhC3-derivative HC3-1496 are detected by a combination of SDS-PAGE and Western blot analysis, and are then concentrated on a 5 ml ANX-FF column using a 0- 0.6 M NaCl step gradient in the same buffer. The concentrated protein is applied to a Sephacryl S-300HR column (2.6 cm x 60 cm) equilibrated with 20 mM Tris-HCl, pH 8.0, 0.1 M NaCl. Elution is performed with the equilibrarion buffer. Fractions containing rhC3-derivative HC3-1496 are detected by a combination of SDS-PAGE and Western blot analysis. The combined fractions are diluted with sodium acetate buffer (pH 5.5) until the conductivity is below 6 mS, and loaded onto a 5 ml CM-FF column. After elution of the protein with a 0 to 0.6 M NaCl gradient in the same buffer, fractions were immediately neutralized by the addition of 1 M Tris-HCl, pH 8, and those fractions containing rhC3-derivative HC3-1496 are identified by a combination of SDS-PAGE and Western blot analysis.

**EXAMPLE 17. Characterization of rhC3-derivative HC3-1496**

Analysis of the stability of the convertase formed by purified rhC3-derivative HC3-1496 and its complement consuming activity, Factor B cleavage activity, and C3 and C5 cleavage activity is performed according the methods of the present patent application and US patent application 2010/0179092. According to US patent application 2010/0179092 rhC3-derivative HC3-1496 has exhibits about 20 to 50% complement consuming activity as compared to CVF, Factor B cleavage activity about equal to CVF, convertase stability about equal to CVF, C3 cleavage activity about five times better than CVF, and none detected C5 cleavage activity.

**EXAMPLE 18. Cloning, expression and purification of the murine IL-4 antagonist QY**

A major obstacle for testing the effects of human antagonistic IL-4 mutants in vivo is the species specificity of IL-4. Human IL-4 does not bind to the mouse receptor and vice versa.

Therefore, the murine IL-4 antagonist QY (Q116D/Y119D) in which the amino acids glutamine 116 and tyrosine 119 are mutated to aspartic acid, has been generated for the proof of concept. This murine IL-4 mutant is analogous to the R121D/Y124D double mutant of human Il-4, binds with high affinity to the murine IL-4Rα without inducing signal transduction, has no detectable activity upon proliferation or differentiation of murine cells, and an excess of the murine QY mutant has been shown to completely inhibit responses toward wild-type murine IL-4 (Grunewald *et al.,* 1997). Furthermore, the murine QY mutant is also a complete inhibitor for murine IL-4 in the absence of γ (Andersson *et al.,* 1997).

*Cloning of the murine IL-4 antagonist QY (Q116D/Y119D).*

The nucleic acid sequence of murine IL-4 (SEQ ID NO:1) coding for the murine IL-4 protein (SEQ ID NO:2) is modified to include a thrombin cleavable N-terminal 6xHis-tag and the QY (Q116D/Y119D) mutations (SEQ ID NO:4). For expression of the murine IL-4 antagonist QY (SEQ ID NO:4) in prokaryontic *E. coli* cells the nucleic acid sequence is optimized using codon optimization according to Puigbo *et al.* (2007) and Grote *et al.* (2005). The resulting sequence (SEQ ID NO:3) is synthesized and amplified with primers 1 (SEQ ID NO:5) and 3 (SEQ ID NO:6) using PCR with Pfu polymerase according to the manufacture and annealing temperatures rising from initial 5 cycles at 50°C to 60°C and 30 cycles total. The resulting PCF fragment is gel-purified in 1% agarose (GeneJET Gel Extraction Kit, Fermentas) and transferred to a restriction enzyme double digest containing *Nde I* and *Xho I* (Fermentas). The restriction digest is again gel-purified and the fragment is ligated into *Nde I* and *Xho I* cut pET-22b (Novagen) expression vector.

*Expression.*

Recombinant His-tagged murine IL-4 antagonist QY (Q116D/Y119D) is expressed in *E. coli* (BL21(DE3)pLysS; Novagen) at 37°C. Cultures are grown in 1-L batches in minimal medium containing 100 μg/ml ampicillin and 34 μg/ml

chloramphenicol. The minimal medium contains the following components: 0.1 M phosphate buffer, pH 7.0, 2 mM $MgSO_4$, 17 mM NaCl, 0.1 mM $CaCl_2$, 5 mg/L $FeSO_4$, 0.2 mg/L $(NH_4)$ $MO_7O_{24}$, 1 mg/L $H_3BO_3$, 0.2 mg/L $CoCl_2$, 0.2 mg/L $CuSO_4$, 2 mg/L $MnSO_4$, and 2 mg/L $ZnSO_4$, supplemented with thymidine, biotin, folic acid, pantothenic acid, niacinamide, pyroxidine phosphate, and thiamine (1 mg/L each). The expression is induced by the addition of isopropyl-D-thiogalactopyranoside to a final concentration of 1 mM after the culture reaches an $OD_{600}$ of approximately 0.6. The cells are allowed to grow for 4h and then harvested by centrifugation at 5000 x g for 10 min at 4°C. The pellet is stored at -20°C.

*Refolding from inclusion bodies.*

The insoluble His-tagged murine IL-4 antagonist QY (Q116D/Y119D) is refolded according to a published protocol (Razeghifard, 2004). The *E. coli* pellet containing the recombinant IL-4 antagonist QY is resuspended in 15 ml of 50 mM Tris-HCl, pH 8.0, and 5 mM EDTA (lysis buffer) containing 2 mg lysozyme and incubated at room temperature for 20 min. The cell lysate is diluted with 60 ml lysis buffer and sonicated. It is then centrifuged at 11,000 rpm for 30 min at 4°C to collect the pellet containing inclusion bodies. The pellet is washed twice with 60 ml with 2 M urea in lysis buffer and collected by centrifugation as described above. Finally, the pellet is resuspended in 25 ml of 6 M GdnHCl, 50 mM potassium phosphate, pH 8.0, and 1 mM reduced glutathione (extraction buffer) and stirred at room temperature for 10 min. The solution is centrifuged to remove the insoluble portion.

The guanidine-extracted IL-4 antagonist QY is loaded onto 15 ml of Ni-charged HisTrap™ FF columns (GE Healthcare) equilibrated with the extraction buffer. The beads are washed with 8 column volumes of 7 M urea and 50 mM potassium phosphate, pH 6.8, to remove the cellular proteins. The His-tagged IL-4 antagonist QY is refolded on the column by the gradual removal of urea through a linear gradient expanding from 100% unfolding buffer to 100% refolding buffer (380 ml at a flow rate of 1 ml/min) using a fast protein liquid chromatography AKTA™ system (Pharmacia). The unfolding buffer is composed of 7 M urea, 100 mM NaCl, 50 mM potassium phosphate, pH 6.8, 1 mM reduced glutathione, and 1 mM oxidized glutathione; the refolding buffer has the same composition but without urea. After washing the column with 40 ml refolding buffer, the refolded His-tagged IL-4 antagonist QY is eluted from the column with 100 mM EDTA and 10 mM Tris-HCl, pH 8.0. EDTA is used to chelate divalent cations that can potentially cause protein aggregation during dialysis. The His-tagged IL-4 antagonist QY is then dialyzed against 50 mM TrisHCl, pH 8.0, 5 mM $CaCl_2$, and 100 mM NaCl to remove EDTA. The protein is concentrated to approximately 2 mg/ml.

*Removal of the His-tag.*

The His-tag is cleaved overnight by incubation of each mg fusion protein with 10 U thrombin (Sigma) in PBS buffer. The mix is incubated at room temperature overnight. The reaction can be stopped with 1 mM PMSF. Cleavage products are separated by chromatography. Residual thrombin can be removed with pAminonezamidine-Agarose (Sigma) either by batch or chromatographic methods.

The cleavage specificity is confirmed by N-terminus sequencing of blotted IL-4 antagonist QY. The His-tag is removed by passing the cleaved protein through Ni-fractogel beads. The purified samples are dialyzed in a desired buffer and kept at 4°C for short term storage or stored at -70°C in the presence of 25% glycerol.

*Analysis of correct refolding.*

The structural integrity of the murine IL-4 antagonist QY is analyzed in a cellular binding assay of murine IL-4 and the murine IL-4 antagonist QY to the murine IL-4 receptor. The functionality of murine IL-4 and the murine IL-4 antagonist QY is determined in a T cell proliferation assay (for details see Example 19).

**EXAMPLE 19. *In vitro* assays for quantitative determinations of murine IL-4 and the murine IL-4 antagonist QY**

In vitro assays for murine IL-4 and the murine IL-4 antagonist QY include an ELISA-type assay and cellular assays. The ELISA-type assay provides information about the integrity of the murine Il-4 epitope recognized by the anti-murine IL-4 antibody. Information about the structural integrity of the murine IL-4 antagonist QY is obtained from a cellular binding assay analyzing binding of murine IL-4 and the murine IL-4 antagonist QY to the murine IL-4 receptor. The functionality of murine IL-4 and the murine IL-4 antagonist QY is determined in a T cell proliferation assay.

*ELISA analysis*

The concentration of murine IL-4 or murine IL-4 mutants is determined by ELISA (Mouse IL-4 ELISA MAX™ Deluxe, BioLegend GmbH, Fell) according to the manufacturers instructions.

*Cellular binding assay for murine IL-4 and murine IL-4 antagonist QY*

Binding of murine Il-4 or the murine IL-4 antagonist QY to IL-4 receptor molecules on the surface of murine CTLL-2 cells is detected by FACScan analysis using a biotinylated anti-IL-4 antibody and fluorescent-labeled streptavidin. Murine CTLL-2 cells are IL-2-dependent and typically express 2000-5000 Il-4 receptors per cell.

Murine CTLL-2 cells, obtained from ATCC (ATCC TIB 214), are cultured in RPMI 1640 containing 8% fetal calf serum, 100 units/ml penicillin and 100 μg/ml streptomycin, supplemented with 100 ng/ml IL-2. For the cellular binding assay 5 x $10^6$ cells are incubated for 30 min at 37°C in 150 μl of PBS (pH 7.4) containing 1% (w/v) BSA, 1 x $10^{-9}$ M recombinant murine (rmu) IL-4 or the murine IL-4 antagonist QY. The mixture is chilled to 4°C, washed once in a large volume of PBS (pH 7.4) containing 1% (w/v) BSA, resuspended in 150 μl of PBS (pH 7.4) and subjected to FACScan analysis.

*T cell proliferation assay*

Il-4 induced proliferation of CTLL-2 cells is determined by incorporation of [$^3$H] thymidine as described (Duschl et *al.,* 1992). The inhibitory effect of the murine IL-4 antagonist QY is determined by adding increasing amounts of the antagonist to IL-4.

CTLL-2 cells are incubated in 0.2 ml aliquots at a density of 5 x $10^5$/ml with log 2 dilutions of murine Il-4 for 3 days, before the amount of [$^3$H] thymidine incorporated during the final 4 hr is determined. The concentration of IL-4 yielding half-maximal response is used for determining the inhibitory activity of the murine IL-4 antagonist QY. In these experiments, log 2 dilutions of the murine IL-4 antagonist QY are added to wild-type murine IL-4.

## EXAMPLE 20. Synthesis and characterization of thermogelling PLGA-PEG-PLGA hydrogels

The biodegradable triblock polymer described in this example has a PLG/PEG weight ratio of 2.3 (70/30), and a lactide/glycolide molar ratio of approx. 15/1. Synthesis of the triblock copolymer is performed according to published protocols (Quiao et al., 2005; patent application WO 02/102309).

### 20.1. Copolymer synthesis

Polyethylene glycol (PEG 1000) is purchased from Fluka, poly(DL-lactide) from Sigma, glycolide (1,4-Dioxane-2,5-dione) from Sigma, and stannous 2-ethylhexanoate from Aldrich.

A total of 25 g of DL-lactide, glycolide and PEG are used for polymerization (16.6 g DL-lactide, 0.9 g glycolide, 7.5 g PEG 1000). Under nitrogen atmosphere, PEG 1000 is dried under vacuum and stirring at 120°C for 2 h in a vigorously dried Erlenmeyer reaction flask. Then the reaction flask is filled with dry argon. DL-lactide and gycolide monomers are added under stirring followed by the addition of Stannous 2-ethylhexanoate (0.2% w/w). Then the tube is sealed under argon. The sealed flask was immersed and kept in an oil bath thermostated at 150°C. After 8 h the flask was cooled to room temperature, and the product was dissolved in cold water. After completely dissolved, the copolymer solution is heated to 80°C to precipitate the copolymer and to remove the watersoluble low molecular weight copolymers and unreacted monomers. The supernatant is decanted, the precipitated copolymer is again dissolved in cold water followed by heating to induce precipitation. This process of dissolution followed by precipitation is repeated three times. Finally, the copolymer is dried under vacuum at room temperature until constant weight.

### 20.2. Molecular weight determination

The molecular weight of the copolymer is determined by gel permeation chromatography using polystyrene standards as described by Quiao et al. (2005).

### 20.3. Measurement of gelation temperature

The gelation temperature is determined as described by Quiao et al. (2005). A 2 ml transparent vial is filled with 200 μl water solution of the copolymer (20% w/w and 25% w/w), is placed in a water bath. The solution is heated in 1°C steps beginning at 26°C in a thermomixing device (Eppendorf). At each temperature step the gellation is checked by careful inversion of the tube. When the solution is not free-flowing, gelation of the solution occured, the temperature read from the thermometer is determined as gelation temperature.

**EXAMPLE 21. In vitro degradation of thermogelling PLGA-PEG-PLGA hydrogels**

The *in vitro* degradation behavior of the copolymer of Example 20 is evaluated by the mass loss and the molecular weight reduction with time upon incubation in phosphate-buffered saline.

Samples (0.5 ml) are incubated in phosphate-buffered saline pH 7.4 at 37°C under mild agitation in a water bath. The solid residues are removed from the incubation medium at scheduled time intervals and lyophilized. The samples are weighted and the weight loss is calculated. Then the solid residues are solved in cold water and analyzed by gel permeation chromatography using polystyrene standards as described by Quiao et al. (2005).

**EXAMPLE 22. Biodegradation of thermogelling PLGA-PEG-PLGA hydrogels**

To test the *in vivo* gel formation behavior and the degradation characteristics of PLGA-PEG-PLGA hydrogels, the hydrogel is injected subcutaneously into mice that have been anesthetized with ethyl ether. The resulting gel implants are then allowed to develop in vivo over the experimental period. At each of the post-injection sampling points, the mice are sacrificed, the gel implants are removed from the subcutaneous injection site, and the removed gel implants are analyzed as described in Example 21.

Two groups of wild-type BALB/c mice (each group: 16 mice) are analyzed for the in *vivo* gel formation behavior and the degradation characteristics: group 1: 20% (w/w) polymer; group 2: 25% (w/w) polymer. The analysis is performed according to following schedule: implantation of 200 µl polymer on day 0, analysis of size in two mice on days 1,2,3,4,5,10,20 and 30.

**EXAMPLE 23. *In vitro* release characteristics of PLGA-PEG-PLGA/protein-composits**

This example shows the in vitro release characteristics of PLGA-PEG-PLGA/protein composits using polyclonal anti-lysozyme antibodies as model protein.

The PLGA-PEG-PLGA triblock copolymer of Example 20 is dissolved at room temperature in phosphate buffered saline (PBS)at pH 7.4, containing 25% or 20% w/v polymer.

1 µl polyclonal rabbit anti-lysozyme serum (80 mg/ml) was added to 200 µl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released antibodies is determined by ELISA with lysozyme coated mircotiter plates (96 wells, Greiner) and anti-rabbit alkaline phosphate antibody conjugate (Sigma) using para-nitrophenyl phosphate (pNPP) as chromogenic enzyme substrate. The absorbtion at 405 nm correlates with the amount of the released protein.

**EXAMPLE 24. *In* vitro release of rhC3-derivative H5 from PLGA-PEG-PLGA/rhC3-derivative H5 composits**

The PLGA-PEG-PLGA triblock copolymer of Example 20 is dissolved at room temperature in PBS pH 7.4, containing different concentrations of rhC3-derivative H5 (0.2 mg/ml up to 2.0 mg/ml) to make a 20% w/w or 25% w/w solution. Then 200 µl of the formulation is placed in a 2 ml vial, incubated at 37°C for 2 min until gelling, and 1.8 ml of PBS pH 7.4 is added. The vial is incubated at 37°C. At specified sample collection times, a sample is withdrawn and replaced by an identical volume of PBS pH 7.4 to maintain release conditions.

The amount of released rhC3-derivative H5 is determined by bicinchoninc acid (BCA) assay using BCA™ Protein Assay Kit (Pierce, USA). The structural and functional integrity of released rhC3-derivative H5 is determined by a complement consumption assay as described in Example 6.1.

**EXAMPLE 25. *In vitro* release of the murine IL-4 antagonist QY from PLGA-PEG-PLGA/murine IL-4 antagonist QY composits**

The PLGA-PEG-PLGA triblock copolymer of Example 20 is dissolved at room temperature in PBS pH 7.4 containing different concentrations of the murine IL-4 antagonist QY (0.5 mg/ml up to 2.0 mg/ml) to make a 20% w/w or 25% w/w solution. Then 200 µl of the formulation is placed in a 2 ml vial, incubated at 37°C for 2 min until gelling, and 1.8 ml of PBS pH 7.4 is added. The vial is incubated at 37°C. At specified sample collection times, a sample is withdrawn and replaced by an identical volume of PBS pH 7.4 to maintain release conditions.

The amount of released murine IL-4 antagonist QY is determined by bicinchoninc acid (BCA) assay using BCA™ Protein Assay Kit (Pierce, USA) and by an ELISA-type assay as described in Example 19. The structural integrity of released murine IL-4 antagonist QY is determined by a cellular binding assay as described in Example 19. The functionality of released murine IL-4 antagonist QY is determined by inhibition of IL-4-induced T cell proliferation as described in Example 19.

**EXAMPLE 26. Biodegradation and release characteristics of PLGA-PEG-PLGA/CVF composits**

To test the *in vivo* gel formation behavior and the release characteristics of composits of PLGA-PEG-PLGA hydrogels containing CVF, the composits are injected subcutaneously into mice that have been anesthetized with ethyl ether. The resulting gel implants are then allowed to develop in vivo over the experimental period. At each of the post-injection sampling points, the mice are sacrificed, serum samples are taken for a complement consumption assay as described in Example 6.1., and for the determination of CVF by ELISA as described in Example 2.2. Thereafter, the gel implants are removed from the subcutaneous injection site. After determination of the volume of the removed gel implants, the remaining *in vitro* releasable amount of CVF is determined by ELISA and protein determination (bicinchoninc acid assay using BCA™ Protein Assay Kit of Pierce, USA). The structural and functional integrity of released CVF is determined by a complement consumption assay as described in Example 6.1.

Two groups of wild-type BALB/c mice are analyzed for the in vivo gel formation behavior and the release characteristics: group 1: 20% (w/w) polymer containing in 200 $\mu$l 0.2 mg CVF; group 2: 25% (w/w) polymer containing in 200 $\mu$l 0.2 mg CVF. The analysis is performed according to following schedule: implantation of 200 $\mu$l polymer composit on day 0, analysis of polymer size and CVF release 2 hours after injection, 4 hours after injection, 6 hours after injection and 1, 2, and 3 days after injection.

**EXAMPLE 27. Biodegradation and release characteristics of PLGA-PEG-PLGA/rhC3-derivative H5 composits**

To test the *in vivo* gel formation behavior and the release characteristics of composits of PLGA-PEG-PLGA hydrogels containing rhC3-derivative H5, the composits are injected subcutaneously into mice that have been anesthetized with ethyl ether. The resulting gel implants are then allowed to develop in vivo over the experimental period. At each of the post-injection sampling points, the mice are sacrificed, serum samples are taken for a complement consumption assay as described in Example 6.1., and for the determination of rhC3-derivative H5 by ELISA as described in Example 2.2. Thereafter, the gel implants are removed from the subcutaneous injection site. After determination of the volume of the removed gel implants, the remaining *in vitro* releasable amount of rhC3-derivative H5 is determined by ELISA and protein determination (bicinchoninc acid assay using BOA™ Protein Assay Kit of Pierce, USA). The structural and functional integrity of released rhC3-derivative H5 is determined by a complement consumption assay as described in Example 6.1.

Two groups of wild-type BALB/c mice are analyzed for the in *vivo* gel formation behavior and the release characteristics: group 1: 20% (w/w) polymer containing 200 $\mu$l 0.2 mg rhC3-derivative H5; group 2: 25% (w/w) polymer containing in 200 $\mu$l 0.2 mg rhC3-derivative H5. The analysis is performed according to following schedule: implantation of 200 $\mu$l polymer composit on day 0, analysis of polymer size and release of rhC3-derivative H5 2 hours after injection, 4 hours after injection, 6 hours after injection and 1, 2, and 3 days after injection.

**EXAMPLE 28. Biodegradation and release characteristics of PLGA-PEG-PLGA/murine IL-4 antagonist QY composits**

To test the *in vivo* gel formation behavior and the release characteristics of composits of PLGA-PEG-PLGA hydrogels containing the murine IL-4 antagonist QY, the composits are injected subcutaneously into mice that have been anesthetized with ethyl ether. The resulting gel implants are then allowed to develop in vivo over the experimental period. At each of the post-injection sampling points, the mice are sacrificed and the gel implants are removed from the subcutaneous injection site. After determination of the volume of the removed gel implant, the remaining in vitro releasable amount of the murine IL-4 antagonist QY is determined as described Example 25.

Two groups of wild-type BALB/c mice (each group: 16 mice) are analyzed for the *in vivo* gel formation behavior and the release characteristics: group 1: 20% (w/w) polymer containing 200 $\mu$g murine IL-4 antagonist QY; group 2: 25% (w/w) polymer containing 200 $\mu$g murine IL-4 antagonist QY. The analysis is performed according to following schedule: implantation of 200 $\mu$l polymer composit on day 0, analysis of size and residual release in two mice on days 1, 2, 3, 4, 5, and 10.

**EXAMPLE 29. Humoral immune response in mice after co-injection of polymer-embedded rhC3-derivative H5/IL-4 antagonist QY and alum-adsorbed ovalbumin**

This example shows the effect of subcutaneously injected polymer-embedded rhC3-derivative H5 and IL-4/IL-13 antagonist QY followed by the injection of alum-adsorbed ovalbumin (OVA) at the site of the gelled polymer composit, on the humoral immune response and development of allergic symptoms in mice.

Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgG1, and goat antisera against murine IgG2a, IgG2b, and IgG3 from BD Pharmigen.

*Preparation of alum-adsorbed ovalbumin (OVA).*

OVA (100 μg) is solved in 0.3 ml of PBS, pH 7.4, and mixed with 0.70 ml Imject Alum.

*Treatment procedure.*

Mice are injected subcutaneously 200 μl of the PLGA-PEG-PLGA triblock copolymer of Example 20 dissolved in distilled water containing increasing doses of the rhC3-derivative H5 (0.2 mg/ml up to 0.8 mg/ml), and of the murine IL-4 antagonist QY (0.1 mg/ml up to 0.4 mg/ml). The final concentration of the copolymer in these experiments is 20% w/w or 25% w/w. A control group receives the copolymer without embedded rhC3-derivative H5 and IL-4 antagonist QY. Before immunization with OVA, analyses of OVA-specific antibodies, cytokine levels in serum and FOXP3 and GATA3 mRNA expression are performed. Six hours later, 100 μl alum-adsorbed OVA (10 μg in 100 μl of PBS/Imject Alum) are injected subcutaneously at the site of the gelled polymer composit (50 μl at each side of the gelled polymer). At day 3 and day 6 after immunization with OVA, one group of mice (group 6) are injected again 100 μl of the PLGA-PEG-PLGA triblock copolymer of Example 20 containing the rhC3-derivative H5 and the murine IL-4 antagonist QY. At day 9 after immunization with OVA, serum levels of OVA-specific antibodies are analyzed. Furthermore, immediate hypersensitivity in skin responses upon challenge via intradermal injection of OVA (3 mice of each group) and the development of anaphylactic shock upon i.v. injection of OVA (3 mice of each group) is assessed. Inguinal lymphnodes are used for a detailed analysis of T cell phenotypes.

Six groups of wild-type BALB/c mice (each group: 6 mice) are analyzed: group 1 (control 1): non-loaded polymer, mock immunization; group 2 (control 2): non-loaded polymer, immunization with alum-OVA; group 3: one injection of loaded polymer (0.2 mg rhC3-derivative H5 and 100 μg IL-4/IL-13 antagonist QY), immunization with alum-OVA); group 4: one injection of loaded polymer (0.4 mg rhC3-derivative H5 and 200 μg IL-4/IL-13 antagonist QY), immunization with alum-OVA; group 5: one injection of loaded polymer (0.8 mg rhC3-derivative H5 and 400 μg IL-4/IL-13 antagonist QY), immunization with alum-OVA; and group 6: two injections of loaded polymer (0.4 mg rhC3-derivative H5 and 200 μg IL-4/IL-13 antagonist QY), immunization with alum-OVA.

*Analysis of serum levels of OVA-specific antibodies.*

Mice are bled at day 0 before immunization with OVA and 9 days later. Murine anti-OVA IgE and IgG subclasses are determined by ELISA. Plates are coated with 10 μg OVA in 100 μl 0.1 M $NaHCO_3$ for 6 h at 37°C, followed by blocking with 200 μl 3% BSA in PBS, pH 7.4, for 2 h at 37°C. After washing, 100 μl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgG1, IgG2a, IgG2b, IgG3). Analysis is performed at 405 nm in a microplate autoreader.

*Analysis of serum levels of cytokines.*

The cytokine supernatant is profiled using a panel of 27 cytokines including the Th2 cytokines IL-4, IL-5 and IL-13.

*Analysis of FOXP3 and GATA3 mRNA expression.*

The read-out for T cell differentiation is FOXP3 and GATA3 mRNA expression, which are inversely regulated. In addition the release is followed in realtime using STAT6 responsive Luciferase systems reporter systems.

*Analysis of immediate cutaneous hypersensitivity.*

Active cutaneous anaphylaxis is tested by skin test after i.v. injection of 200 μl of 0.5% Evans Blue dye in PBS, pH 7.4. Thereafter, the skin of the belly is shaved and four injection sites are marked with a felt tip pen on the skin. Two of the marked sites are injected intradermally with 50 μl PBS, pH 7.4, containing 50 μg OVA, and the other two sites with protein-free PBS, pH 7.4. After 15 min, the mice were killed by cervical dislocation and the skin is stripped off for inspection of the injection sites. The intensity of blue patch formation on the dorsal side of the skin, resulting from fluid extravasation into the injection site upon mast cell degranulation, is scored by two independent observers. Reactions are rated as positive when the diameter of the blue patch exceeds 5 mm, which is pre-marked on the mouse skin. The intensity of bluing is rated in the following manner: 0 = no blue patch formation; 1 = slight bluing; 2 = marked bluing; 3 = strong bluing.

*Analysis of anaphylactic shock symptoms.*

Mice are injected i.v. 500 μg OVA in 200 μl 0.5% Evans Blue solution. After 15 min, symptoms of an anaphylactic shock are assessed including bluing (no = 0; slight = 1; strong = 2), pilo erection (no = 0; slight = 1; strong = 2), spontaneous activity (running around = 0; sitting passively = 1; lying = 2), and responsiveness to external stimuli (running away upon touching = 0; slight reaction = 1; no reaction = 2). The animals are considered to be in a state of shock if at least three of the four indicated symptoms are observed by two independent observers who are unaware of the sensitization status of each animal.

*Analysis of T cell phenotype in inguinal lymphnodes.*

Right and left inguinal lymph nodes are removed and T cell phenotype is tested by *in vitro* re-stimulation with allergen (ELISPOT/Luminex).

**EXAMPLE 30: Specific immunotherapy of OVA-sensitized mice with alum-adsorbed OVA and composits of PLGA-PEG-PLGA/rhC3-derivative H5/murine IL-4 antagonist QY**

This example shows the efficacy of immunotherapy in OVA-sensitized mice using subcutaneously injected polymer-embedded rhC3-derivative H5 and IL-4/IL-13 antagonist QY and co-injected alum-adsorbed OVA at the site of the gelled polymer composit.

Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgG1, and goat antisera against murine IgG2a, IgG2b, and IgG3 from BD Pharmigen.

*Preparation of alum-adsorbed ovalbumin (OVA).* OVA (100 μg) is solved in 0.3 ml of PBS, pH 7.4, and mixed with 0.70 ml Imject Alum (Pierce/KMF).

*Allergic sensitization procedure.* Mice are immunized three times by i.p. injection with 10 μg OVA in 200 μl of PBS, pH 7.4, mixed with 70 μl Imject Alum as adjuvant. The second i.p. injection is performed 7 days after the first injection and the third injection 14 days after the first injection.

*Immunotherapy.* One week after the third immunization with OVA, mice are subjected to two different modalities of immunotherapy, one based on conventional treatment with increasing doses of alum-adsorbed OVA, the other based on treatment with increasing doses of alum-adsorbed OVA in the presence of polymer-embedded rhC3-derivative H5 and murine IL-4/IL-13 antagonist QY. Subcutaneous administration of increasing doses of alum-adsorbed OVA is performed at 9-day intervals. Subcutaneous administration of polymer-embedded rhC3-derivative H5 and murine IL-4/IL-13 antagonist QY is performed at day 0, day 3 and day 6 within the 9-day intervals.

For immunotherapy of mice with alum-adsorbed OVA only, 100 μl of PBS/Imject Alum containing increasing doses of OVA (7.5, 15, 30, 60, 90 μg) are injected subcutaneously at 9-day intervals.

For immunotherapy of mice with alum-adsorbed OVA in the presence of polymer-embedded rhC3-derivative H5 and murine IL-4 antagonist QY, 200 μl of distilled water containing the PLGA-PEG-PLGA triblock copolymer of Example 20 (20% w/w or 25% w/w), 0.4 mg rhC3-derivative H5 and 200 μg IL-4/IL-13 antagonist QY, are injected subcutaneously first. After 60 min, 100 μl of PBS/Imject Alum containing increasing doses of OVA (7.5, 15, 30, 60, 90 μg) are injected subcutaneously at the site of the gelled polymer composit (50 μl at each side of the gelled polymer).

At day 3 and day 6 within each 9-day interval, additional 100 μl of distilled water containing the PLGA-PEG-PLGA triblock copolymer of Example 20 (20% w/w or 25% w/w), 0.4 mg rhC3-derivative H5 and 200 μg IL-4/IL-13 antagonist QY, are injected subcutaneously at the site of the first injection. The injection procedure is repeated at a different subcutaneous site until the highest dose of alum-adsorbed OVA is injected.

Three groups of wild-type BALB/c mice (each group: 10 mice) are analyzed: group 1: immunization (3 x alum-OVA), therapy with non-loaded polymer and PBS; group 2: immunization (3 x alum-OVA), therapy with loaded polymer and alum-OVA; and group 3: immunization (3 x alum-OVA), therapy with alum-OVA.

Mice are bled at day 0 before immunization with OVA, at day 7 after immunization with OVA (before starting immunotherapy), and at day 9 after the last immunotherapeutic treatment. Analyses include the determination of serum levels of OVA-specific antibodies, immediate hypersensitivity in skin responses upon challenge via intradermal injection of OVA and the development of anaphylactic shock upon i.v. injection of OVA.

*Analysis of serum levels of OVA-specific antibodies.*

Murine anti-OVA IgE and IgG subclasses are determined by ELISA. Plates are coated with 10 μg OVA in 100 μl 0.1 M $NaHCO_3$ for 6 h at 37°C, followed by blocking with 200 μl 3% BSA in PBS, pH 7.4, for 2 h at 37°C. After washing, 100 μl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgG1, IgG2a, IgG2b, IgG3). Analysis is performed at 405 nm in a microplate autoreader.

*Analysis of serum levels of cytokines.*

The cytokine supernatant is profiled using a panel of 27 cytokines including the Th2 cytokines IL-4, IL-5 and IL-13.

*Analysis of FOXP3 and GATA3 mRNA expression.*

The read-out for T cell differentiation is FOXP3 and GATA3 mRNA expression, which are inversely regulated. In addition the release is followed in realtime using STAT6 responsive Luciferase systems reporter systems.

*Analysis of immediate cutaneous hypersensitivity.*

Active cutaneous anaphylaxis is tested by skin test after i.v. injection of 200 μl of 0.5% Evans Blue dye in PBS, pH 7.4. Thereafter, the skin of the belly is shaved and four injection sites are marked with a felt tip pen on the skin. Two of the marked sites are injected intradermally with 50 μl PBS, pH 7.4, containing 50 μg OVA, and the other two sites with protein-free PBS, pH 7.4. After 15 min, the mice were killed by cervical dislocation and the skin is stripped off for inspection of the injection sites. The intensity of blue patch formation on the dorsal side of the skin, resulting from fluid extravasation into the injection site upon mast cell degranulation, is scored by two independent observers. Reactions are rated as positive when the diameter of the blue patch exceeds 5 mm, which is pre-marked on the mouse skin. The intensity of bluing is rated in the following manner: 0 = no blue patch formation; 1 = slight bluing; 2 = marked bluing; 3 = strong bluing.

*Analysis of anaphylactic shock symptoms.*

Mice are injected i.v. 500 μg OVA in 200 μl 0.5% Evans Blue solution. After 15 min, symptoms of an anaphylactic shock are assessed including bluing (no = 0; slight = 1; strong = 2), pilo erection (no = 0; slight = 1; strong = 2), spontaneous activity (running around = 0; sitting passively = 1; lying = 2), and responsiveness to external stimuli (running away upon touching = 0; slight reaction = 1; no reaction = 2). The animals are considered to be in a state of shock if at least three of the four indicated symptoms are observed by two independent observers who are unaware of the sensitization status of each animal.

*Analysis of T cell phenotype in inguinal lymphnodes.*

Right and left inguinal lymph nodes are removed and T cell phenotype is tested by *in vitro* re-stimulation with allergen (ELISPOT/Luminex).

**EXAMPLE 31: Specific immunotherapy of mice with OVA-induced allergic airway inflammation**

This example shows the efficacy of immunotherapy in OVA-sensitized mice exhibiting allergic airway inflammation after inhaled OVA administration, using subcutaneously injected polymer-embedded rhC3-derivative H5 and IL-4/IL-13 antagonist QY and co-injected alum-adsorbed OVA at the site of the gelled polymer composit.

Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgG1, and goat antisera against murine IgG2a, IgG2b, and IgG3 from BD Pharmigen.

*Preparation of alum-adsorbed ovalbumin (OVA).* OVA (100 μg) is solved in 0.3 ml of PBS, pH 7.4, and mixed with 0.70 ml Imject Alum (Pierce/KMF).

*Allergic sensitization procedure.* Mice are immunized three times by i.p. injection with 10 μg OVA in 200 μl of PBS, pH 7.4, mixed with 70 μl Imject Alum as adjuvant. The second i.p. injection is performed 7 days after the first injection and the third injection 14 days after the first injection. One week after the last injection the mice are exposed in a Plexiglas chamber (approx. 10 x 15 x 25 cm) to 1% (wt/vol) aerosolized OVA in 0.9% saline (using a nebulizer with an airflow rate of 10L/min), 30 min/day for 10 days.

*Immunotherapy.* One week after the final aerosol exposure, mice are subjected to two different modalities of immunotherapy, one based on conventional treatment with increasing doses of alum-adsorbed OVA, the other based on treatment with increasing doses of alum-adsorbed OVA in the presence of polymer-embedded rhC3-derivative H5 and murine IL-4/IL-13 antagonist QY. Subcutaneous administration of increasing doses of alum-adsorbed OVA (7.5, 15, 30, 60, 90 μg) is performed at 9-day intervals. Subcutaneous administration of polymer-embedded rhC3-derivative H5 and murine IL-4/IL-13 antagonist QY is performed at day 0, day 3 and day 6 within each 9-day interval.

For immunotherapy of mice with alum-adsorbed OVA only, 100 μl of PBS/Imject Alum containing increasing doses of OVA (7.5, 15, 30, 60, 90 μg) are injected subcutaneously at 9-day intervals.

For immunotherapy of mice with alum-adsorbed OVA in the presence of polymer-embedded rhC3-derivative H5 and murine IL-4 antagonist QY, 200 μl of distilled water containing the PLGA-PEG-PLGA triblock copolymer of Example 20 (20% w/w or 25% w/w), 0.4 mg rhC3-derivative H5 and 200 μg IL-4/IL-13 antagonist QY, are injected subcutaneously first. After 60 min, 100 μl of PBS/Imject Alum containing increasing doses of OVA (7.5, 15, 30, 60, 90 μg) are injected subcutaneously at the site of the gelled polymer composit (50 μl at each side of the gelled polymer).

At day 3 and day 6 within each 9-day interval, additional 100 μl of distilled water containing the PLGA-PEG-PLGA triblock copolymer of Example 20 (20% w/w or 25% w/w), 0.4 mg rhC3-derivative H5 and 200 μg IL-4/IL-13 antagonist QY, are injected subcutaneously at the site of the first injection. The injection procedure is repeated at a different subcutaneous site until the highest dose of alum-adsorbed OVA is injected.

*Challenge procedure*

One week after completed immunotherapeutic treatment, mice are challenged with 1% aerosolized OVA (as described above), 30 min/day for 3 consecutive days.

*Experimental animal groups*

Six groups of wild-type BALB/c mice (each group: 10 mice) are analyzed:

| | | |
|---|---|---|
| Group 1: | - immunization | 3 x i.p. alum-OVA and 10 days exposure to aerosolized OVA |
| | - no therapy | |
| | - AHR analysis | 2 days after immunization |
| | - sacrifice | 3 days after immunization |
| Group 2: | - immunization | 3 x i.p. alum-OVA and 10 days exposure to aerosolized OVA |
| | - therapy | with non-loaded polymer and PBS |
| | - AHR analysis | 12 days after immunotherapy |
| | - sacrifice | 13 days after immunotherapy |
| Group 3: | - immunization | 3 x i.p. alum-OVA and 10 days exposure to aerosolized OVA |
| | - therapy | with loaded polymer and alum-OVA |
| | - AHR analysis | 12 days after immunotherapy |
| | - sacrifice | 13 days after immunotherapy |
| Group 4: | - immunization | 3 x i.p. alum-OVA and |
| | - therapy | 10 days exposure to aerosolized OVA with loaded polymer and alum-OVA |
| | - challenge | 3 days exposure to aerosolized OVA (start: 7 days after immunotherapy) |
| | - AHR analysis | 12 days after immunotherapy (2 days after challenge) |
| | - sacrifice | 3 days after challenge (= 13 days after immunotherapy) |
| Group 5: | - immunization | 3 x i.p. alum-OVA and 10 days exposure to aerosolized OVA |
| | - therapy | with loaded polymer and alum-OVA |
| | - AHR analysis | 12 days after immunotherapy |
| | - sacrifice | 13 days after immunotherapy |
| Group 6: | - immunization | 3 x i.p. alum-OVA and 10 days exposure to aerosolized OVA) |
| | - therapy | with alum-OVA |
| | - challenge | 3 days exposure to aerosolized OVA (start: 7 days after immunotherapy) |
| | - AHR analysis | 12 days after immunotherapy (2 days after challenge) |
| | - sacrifice | 3 days after challenge (= 13 days after immunotherapy) |

Mice are bled at day 0 before immunization with OVA, one week after immunization with OVA (before starting immunotherapy), and two weeks after the last immunotherapeutic treatment.

Analyses include the determination of serum levels of OVA-specific antibodies and cytokines.

Measurement of airway hyperreactivity (AHR), defined as bronchoconstriction in response to methacholine, is performed in two animals of each group: 2 days after completion of immunization in group 1, 12 days after completion of immunotherapy in group 2-6.

Three days after the final aerosol exposure, the mice of group 1 are sacrificed by exsanguination after i.p. injection of a ketamine/xylazine overdose, and analyses of bronchoalveolar lavage (BAL), lung tissue, and blood samples are performed. The mice of group 2, 3 and 5 are sacrificed 13 days after completion of immunotherapy by exsanguination after i.p. injection of a ketamine/xylazine overdose, and analyzed as described for group 1. Mice from group 4 and 6 are sacrificed 3 days after the final aerosol challenge (= 13 days after completion of immunotherapy) by exsanguination after i.p. injection of a ketamine/xylazine overdose, and analyzed as described for group 1.

*Measurement of AHR: Lung resistance*

Measurements of pulmonary resistance ($R_L$) are performed in anesthetized, mechanically ventilated mice (Yiamouyiannis et al., 1999). After anesthetizing the animals with pentobarbital (75 mg/kg i.p. injection), the abdominal inferior vena cava is cannulated, and a tracheostomy catheter is placed. The chest is opened by a small nanterior incision, and the animal is placed in a whole-body plethysmograph. Mechanical ventilation is established with a small rodent respirator delivering a 10 ml/kg tidal volume at 140 breaths/minute, with a positive end-expiratory pressure (PEEP) of 3 cm $H_2O$. Values for $R_L$ are calculated by analysis of electrical signals proportional to lung volume, airflow, and transpulmonary pressure. Changes in lung volume are determined from the measured changes in plethysmographic pressure and are differentiated over time to obtain flow measurements. Transpulmonary pressure is obtained from the difference between measured pressures at the airway opening and within the plethysmograph. After the establishment of baseline lung function, the animal receives sequentially increasing intravenous doses of methacholine (Sigma; 3 to 3000 μg/ml in 1 ml/kg body weight increments). Maximal $R_L$ responses are determined from measurements averaged over 6-second intervals. Pulmonary function is allowed to return to baseline values before each subsequent dose.

*Bronchoalveolar lavage (BAL) analysis*

The lungs from five animals of each group are lavaged *in situ* with five 1 ml aliquots of sterile saline, with 3 to 4 ml BAL fluid recovered from each animal. The BAL is centrifuged and resulting cell pellets are suspended in 250 μl saline. BAL protein concentrations are measured in the supernatants by the bicinchoninc acid (BCA) assay using the BCA™ Protein Assay Kit (Pierce, USA) and bovine serum albumin as standard. Total leukocytes are counted in a hemocytometer using trypan blue dye exclusion as a measure of viability. Cytospin slides are made and stained with May-Grunwald/Giemsa to determine the BAL cell differential. The remaining cells are analyzed by fluorescence flow cytometry. For these analyses, BAL samples are washed in phosphate-buffered saline (PBS) containing 0.2% bovine serum albumin and 0.1% $NaN_3$. Aliquots containing $10^4$ to $10^5$ cells are incubated with 100 μl of appropriately diluted antibodies for 30 min at 4°C. After staining, the cells are washed twice with the above PBS solution, and relative fluorescence intensities are determined on a 4-decade log scale by flow cytometric analysis using a FACScan (Becton Dickinson). Fluorescent monoclonal antibodies used for the fluorescence flow cytometric analyses are directed against B cell and T cell antigens including CD3ε, TCRβ, TCRδ, CD4, CD8, and CD45.

*Analysis of serum levels of OVA-specific antibodies.*

Murine anti-OVA IgE and IgG subclasses are determined by ELISA. Plates are coated with 10 μg OVA in 100 μl 0.1 M $NaHCO_3$ for 6 h at 37°C, followed by blocking with 200 μl 3% BSA in PBS, pH 7.4, for 2 h at 37°C. After washing, 100 μl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgG1, IgG2a, IgG2b, IgG3). Analysis is performed at 405 nm in a microplate autoreader.

*Analysis of serum levels of cytokines.*

The cytokine supernatant is profiled using a panel of 27 cytokines including the TH2 cytokines IL-4, IL-5 and IL-13.

*Tissue analysis*

For tissue immunofluorescence, unmanipulated lungs (not exposed to BAL or methacholine) are excised, cut into small pieces, and are rapidly frozen in optimal cutting temperature embedding media. The pieces are then cut into 5 μm frozen sections using a Hacker cryostat, mounted onto microscope slides, and stored at -20°C. For immunofluorescence staining, the slides are fixed in acetone (-20°C) for 5 minutes, dried, and blocked with 1% ChromPure IgG solution (Jackson ImmunoResearch) for 30 min at room temperature. After two washes with PBS containing 1% $NaN_3$, specific fluorescent monoclonal antibodies are added to the tissue and incubated for 60 min in a humidity chamber. Slides are then washed twice with PBS containing 1% $NaN_3$, and then analyzed by fluorescence microscopy.

For staining with hematoxylin and eosin (H&E), unmanipulated lungs (not exposed to BAL or methacholine) are excised, fixed with 10% buffered formalin, and stained with H&E according to standard protocols.

**1 SEQ ID NO:1**

Murine Interleukin-4, Pro-peptide sequence

Ref.: Noma et al 1986, Nature 319: 640-646; UniProtKB/Swiss-Prot: P07750
Genbank X03532.1, Mouse mRNA for IgG1 induction factor
>ENA|X03532|X03532.1 Mouse mRNA for IgG1 induction factor : Location:1..1000

```
atttgttagcatctcttgataaacttaattgtctctcgtcactgacggcacagagctattgatggg
tctcaacccccagctagttgtcatcctgctcttctttctcgaatgtaccaggagccatatccacgg
atgcgacaaaaatcacttgagagagatcatcggcattttgaacgaggtcacaggagaagggacgcc
atgcacggagatggatgtgccaaacgtcctcacagcaacgaagaacaccacagagagtgagctcgt
ctgtagggcttccaaggtgcttcgcatattttatttaaaacatgggaaaactccatgcttgaagaa
gaactctagtgttctcatggagctgcagagactctttcgggcttttcgatgcctggattcatcgat
aagctgcaccatgaatgagtccaagtccacatcactgaaagacttcctggaaagcctaaagagcat
catgcaaatggattactcgtagtactgagccaccatgctttaacttatgaattttaatggtttta
tttttaatatttatatatttataattcataaaataaaatatttgtataatgt
```

**SEQ ID NO:2**
Murine Interleukin-4, Pro-peptide Translated DNA sequence.
Signal peptide sequence is underlined

<u>MGLNPQLVVILLFFLECTRS</u>HIHGCDKNHLREIIGILNEVTGEGTPCTEMDVPNVLTATKNTTESE
LVCRASKVLRIFYLKHGKTPCLKKNSSVLMELQRLFRAFRCLDSSISCTMNESKSTSLKDFLESLK
SIMQMDYS

**SEQ ID NO:3**

Murine Interleukin-4 QY-Double mutant, His-tagged

N-terminal His-tagged mature peptide sequence, *E. coli* K12 optimized codon usage sequence

Ref.: Puigbo et al 2007, NAR 35:W126-W131; Grote et al 2005, NAR 33: W526-31.

ATGGGTTCTTCTCACCACCACCACCACCACTCTTCTGGTCTGGTTCCGCGTGGTTCTCACAT
GCACATCCACGGTTGCGACAAAAACCACCTGCGTGAAATCATCGGTATCCTGAACGAAGTTA
CCGGTGAAGGTACCCCGTGCACCGAAATGGACGTTCCGAACGTTCTGACCGCGACCAAAAAC
ACCACCGAATCTGAACTGGTTTGCCGTGCGTCTAAAGTTCTGCGTATCTTCTACCTGAAACA
CGGTAAAACCCCGTGCCTGAAAAAAAACTCTTCTGTTCTGATGGAACTGCAGCGTCTGTTCC
GTGCGTTCCGTTGCCTGGACTCTTCTATCTCTTGCACCATGAACGAATCTAAATCTACCTCT
CTGAAAGACTTCCTGGAATCTCTGAAATCTATCATGGACATGGACGACTCT

**SEQ ID NO:4**

Murine Interleukin-4, QY-Double mutant, His-tagged. N-terminal His-tagged mature peptide sequence,His-Tag with thrombin cleavage site and mutated sites are underlined.

Ref.: Grunewald et al 1997 JBC 272: 1480-1483

MGSSHHHHHHSSGLVPRGSHMHIHGCDKNHLREIIGILNEVTGEGTPCTEMDVPNVLTATKNTTES
ELVCRASKVLRIFYLKHGKTPCLKKNSSVLMELQRLFRAFRCLDSSISCTMNESKSTSLKDFLESL
KSIMDMDDS

**SEQ ID NO:5**

Primer 1 (IL4his-for) for expression subcloning.

5′ end of Mouse IL-4 (RY/QY mutant) N-terminal His-tagged mature peptide. Contains *Nde I* restriction site for pET-22b+ (underlined)

IL4his-for, 29 bp

5′-AAAAAACCATATGGGTTCTTCTCACCACC

initial annealing temp: 54,5°C

overall annealing temp: 59,2°C

**SEQ ID NO:6**

Primer 3 (MOIL4mut-back) for subcloning.

3' end of Murine IL-4 (QY mutant) N-terminal His-tagged mature peptide. Contains Stop codon (TAA) and *Xho I* restriction site for pET-22b+ (underlined)

MOIL4mut-back, 29 bp

5'-AAAACTCGAGTTAAGAGTCGTCCATGTCC

initial annealing temp: 50,5 C

overall annealing temp: 59,8 C

**SEQ ID NO:7**

Length: 1276

Type: PRT

Organism: Artificial sequence

Feature:

Other Information: Processed hybrid protein comprising the C3 beta-chain and the gamma- and beta-chain of CVF

SPMYSIITPNILRLESEETMVLEAHDAQGDVPVTVTVHDFPGKKLVLSSEKTVLTPATNHMG
NVTFTIPANREFKSEKGRNKFVTVQATFGTQVVEKVVLVSLQSGYLFIQTDKTIYTPGSTVL
YRIFTVNHKLLPVGRTVMVNIENPEGIPVKQDSLSSQNQLGVLPLSWDIPELVNMGQWKIRA
YYENSPQQVFSTEFEVKEYVLPSFEVIVEPTEKFYYIYNEKGLEVTITARFLYGKKVEGTAF
VIFGIQDGEQRISLPESLKRIPIEDGSGEVVLSRKVLLDGVQNPRAEDLVGKSLYVSATVIL
HSGSDMVQAERSGIPIVTSPYQIHFTKTPKYFKPGMPFDLMVFVTNPDGSPAYRVPVAVQGE
DTVQSLTQGDGVAKLSINTHPSQKPLSITVRTKKQELSEAEQATRTMQALPYSTVGNSNNYL
HLSVLRTELRPGETLNVNFLLRMDRAHEAKIRYYTYLIMNKGRLLKAGRQVREPGQDLVVLP
LSITTDFIPSFRLVAYYTLIGASGQREVVADSVWVDVKDSCVGSLVVKSGQSEDRQPVPGQQ
MTLKIEGDHGARVVLVAVDKGVFVLNKKNKLTQSKIWDVVEKADIGCTPGSGKDYAGVFSDA
GLTFTSSSGQQTAQRAELQCPQPAADNEDGFIADSDIISRSDFPKSWLWLTKDLTEEPNSQ
GISSKTMSFYLRDSITTWVVLAVSFTPTKGICVAEPYEIRVMKVFFIDLQMPYSVVKNEQVE
IRAILHNYVNEDIYVRVELLYNPAFCSASTKGQRYRQQFPIKALSSRAVPFVIVPLEQGLHD
VEIKASVQEALWSDGVRKKLKVVPEGVQKSIVTIVKLDPRAKGVGGTQLEVIKARKLDDRVP

DTEIETKIIIQGDPVAQIIENSIDGSKLNEIQMPTHKDLNLDITIELPDREVPIRYRINYEN
ALLARTVETKLNQDITVTASGDGKATMTILTFYNAQLQEKANVCNKFHLNVSVENIHLNAMG
AKGALMLKICTRYLGEVDSTMTIIDISMLTGFLPDAEDLTRLSKGVDRYISRYEVDNNMAQK
VAVIIYLNKVSHSEDECLHFKILKHFEVGFIQPGSVKVYSYYNLDEKCTKFYHPDKGTGLLN
KICIGNVCRCAGETCSSLNHQERIDVPLQIEKACETNVDYVYKTKLLRIEEQDGNDIYVMDV
LEVIKQGTDENPRAKTHQYISQRKCQEALNLKVNDDYLIWGSRSDLLPTKDKISYIITKNTW
IERWPHEDECQEEEFQKLCDDFAQFSYTLTEFGCPT

**SEQ ID NO:8**

Length: 1637

Type: PRT

Organism: Artificial sequence

Feature:

Other Information: Processed H6 construct

SPMYSIITPNILRLESEETMVLEAHDAQGDVPVTVTVHDFPGKKLVLSSEKTVLTPATNHMG
NVTFTIPANREFKSEKGRNKFVTVQATFGTQVVEKVVLVSLQSGYLFIQTDKTIYTPGSTVL
YRIFTVNHKLLPVGRTVMVNIENPEGIPVKQDSLSSQNQLGVLPLSWDIPELVNMGQWKIRA
YYENSPQQVFSTEFEVKEYVLPSFEVIVEPTEKFYYIYNEKGLEVTITARFLYGKKVEGTAF
VIFGIQDGEQRISLPESLKRIPIEDGSGEVVLSRKVLLDGVQNLRAEDLVGKSLYVSATVIL
HSGSDMVQAERSGIPIVTSPYQIHFTKTPKYFKPGMPFDLMVFVTNPDGSPAYRVPVAVQGE
DTVQSLTQGDGVAKLSINTHPSQKPLSITVRTKKQELSEAEQATRTMQALPYSTVGNSNNYL
HLSVLRTELRPGETLNVNFLLRMDRAHEAKIRYYTYLIMNKGRLLKAGRQVREPGQDLVVLP
LSITTDFIPSFRLVAYYTLIGASGQREVVADSVWVDVKDSCVGSLVVKSGQSEDRQPVPGQQ
MTLKIEGDHGARVVLVAVDKGVFVLNKKNKLTQSKIWDVVEKADIGCTPGSGKDYAGVFSDA
GLTFTSSSGQQTAQRAELQCPQPAASVQLTEKRMDKVGKYPKELRKCCEDGMRENPMRFSCQ
RRTRFISLGEACKKVFLDCCNYITELRRQHARASHLGLARSNLDEDIIAEENIVSRSEFPES
WLWNVEDLKEPPKNGISTKLMNIFLKDSITTWEILAVSMSDKKGICVADPFEVTVMQDFFID
LRLPYSVVRNEQVEIRAVLYNYRQNQELKVRVELLHNPAFCSLATTKRRHQQTVTIPPKSSL
SVPYVIVPLKTGLQEVEVKAAVYHHFISDGVRKSLKVVPEGIRMNKTVAVRTLDPERLGREG
VQKEDIPPADLSDQVPDTESETRILLQGTPVAQMTEDAVDAERLKHLIVTPSGCGEQNMIGM
TPTVIAVHYLDETEQWEKFGLEKRQGALELIKKGYTQQLAFRQPSSAFAAFVKRAPSTWLTA
YVVKVFSLAVNLIAIDSQVLCGAVKWLILEKQKPDGVFQEDAPVIHQEMIGGLRNNNEKDMA
LTAFVLISLQEAKDICEEQVNSLPGSITKAGDFLEANYMNLQRSYTVAIAGYALAQMGRLKG
PLLNKFLTTAKDKNRWEDPGKQLYNVEATSYALLALLQLKDFDFVPPVVRWLNEQRYYGGGY
GSTQATFMVFQALAQYQKDAPDHQELNLDVSLQLPSRSSKITHRIHWESASLLRSEETKENE
GFTVTAEGKGQGTLSVVTMYHAKAKDQLTCNKFDLKVTIKPAPETEKRPQDAKNTMILEICT
RYRGDQDATMSILDISMMTGFAPDTDDLKQLANGVDRYISKYELDKAFSDRNTLIIYLDKVS
HSEDDCLAFKVHQYFNVELIQPGAVKVYAYYNLEESCTRFYHPEKEDGKLNKLCRDELCRCA
EENCFIQKSDDKVTLEERLDKACEPGVDYVYKTKLLRIEEQDGNDIYVMDVLEVIKQGTDEN
PRAKTHQYISQRKCQEALNLKVNDDYLIWGSRSDLLPTKDKISYIITKNTWIERWPHEDECQ
EEEFQKLCDDFAQFSYTLTEFGCPT

**SEQ ID NO:9**

Length: 1637

Type: PRT

Organism: Artificial sequence

Feature:

Other Information: Processed H5 construct

SPMYSIITPNILRLESEETMVLEAHDAQGDVPVTVTVHDFPGKKLVLSSEKTVLTPATNHMG
NVTFTIPANREFKSEKGRNKFVTVQATFGTQVVEKVVLVSLQSGYLFIQTDKTIYTPGSTVL
YRIFTVNHKLLPVGRTVMVNIENPEGIPVKQDSLSSQNQLGVLPLSWDIPELVNMGQWKIRA
YYENSPQQVFSTEFEVKEYVLPSFEVIVEPTEKFYYIYNEKGLEVTITARFLYGKKVEGTAF
VIFGIQDGEQRISLPESLKRIPIEDGSGEVVLSRKVLLDGVQNLRAEDLVGKSLYVSATVIL
HSGSDMVQAERSGIPIVTSPYQIHFTKTPKYFKPGMPFDLMVFVTNPDGSPAYRVPVAVQGE
DTVQSLTQGDGVAKLSINTHPSQKPLSITVRTKKQELSEAEQATRTMQALPYSTVGNSNNYL
HLSVLRTELRPGETLNVNFLLRMDRAHEAKIRYYTYLIMNKGRLLKAGRQVREPGQDLVVLP
LSITTDFIPSFRLVAYYTLIGASGQREVVADSVWVDVKDSCVGSLVVKSGQSEDRQPVPGQQ
MTLKIEGDHGARVVLVAVDKGVFVLNKKNKLTQSKIWDVVEKADIGCTPGSGKDYAGVFSDA
GLTFTSSSGQQTAQRAELQCPQPAASVQLTEKRMDKVGKYPKELRKCCEDGMRENPMRFSCQ
RRTRFISLGEACKKVFLDCCNYITELRRQHARASHLGLARSNLDEDIIAEENIVSRSEFPES
WLWNVEDLKEPPKNGISTKLMNIFLKDSITTWEILAVSMSDKKGICVADPFEVTVMQDFFID
LRLPYSVVRNEQVEIRAVLYNYRQNQELKVRVELLHNPAFCSLATTKRRHQQTVTIPPKSSL
SVPYVIVPLKTGLQEVEVKAAVYHHFISDGVRKSLKVVPEGIRMNKTVAVRTLDPERLGREG
VQKEDIPPADLSDQVPDTESETRILLQGTPVAQMTEDAVDAERLKHLIVTPSGCGEQNMIGM
TPTVIAVHYLDETEQWEKFGLEKRQGALELIKKGYTQQLAFRQPSSAFAAFVKRAPSTWLTA
YVVKVFSLAVNLIAIDSQVLCGAVKWLILEKQKPDGVFQEDAPVIHQEMIGGLRNNNEKDMA
LTAFVLISLQEAKDICEEQVNSLPGSITKAGDFLEANYMNLQRSYTVAIAGYALAQMGRLKG
PLLNKFLTTAKDKNRWEDPGKQLYNVEATSYALLALLQLKDFDFVPPVVRWLNEQRYYGGGY
GSTQATFMVFQALAQYQKDAPDHQELNLDVSLQLPSRSSKITHRIHWESASLLRSEETKENE
GFTVTAEGKGQGTLSVVTMYHAKAKDQLTCNKFDLKVTIKPAPETEKRPQDAKNTMILEICT
RYLGEVDSTMTIIDISMLTGFLPDAEDLTRLSKGVDRYISRYEVDNNMAQKVAVIIYLNKVS
HSEDECLHFKILKHFEVGFIQPGSVKVYSYYNLDEKCTKFYHPDKGTGLLNKICIGNVCRCA
GETCSSLNHQERIDVPLQIEKACETNVDYVYKTKLLRIEEQDGNDIYVMDVLEVIKQGTDEN
PRAKTHQYISQRKCQEALNLKVNDDYLIWGSRSDLLPTKDKISYIITKNTWIERWPHEDECQ
EEEFQKLCDDFAQFSYTLTEFGCPT

## Claims

1. Pharmaceutical composition for the modulation of effector T cell responses made of one or more preparations and comprising a therapeutically effective dose of at least one recombinant human C3-derivative and of at least one antigen or allergen.

2. Composition of claim 1, comprising a therapeutically effective dose of at least one inhibitor of IL-4/IL-13-mediated effests.

3. Composition according to claim 2, wherein the at least one antigen and the recombinant human C3-derivative and, optionally, at least one inhibitor of IL-4/IL-13-mediated effects are coated or absorbed on or embedded in a matrix, wherein the matrix is selected as to enable sustained release of both the antigen and the recombinant human C3-derivative, and, optionally of the at least one inhibitor of IL-4/IL-13-mediated effects.

4. Composition according to claim 2, wherein the recombinant human C3-derivative and, optionally, at least one inhibitor of IL-4/IL-13-mediated effects are coated or absorbed on or embedded in a first matrix, wherein the first matrix is selected as to enable sustained release of the recombinant human C3-derivative, and wherein the allergen or antigen is coated or absorbed on or embedded in a second matrix different from the first matrix, wherein the second matrix is an adjuvant providing a depot effect for antigen or allergen presentation, wherein the first and the second matrices are provided in the separate preparations.

5. Composition according to any of the claims 1 to 4, wherein the recombinant human C3-derivative is selected from a group of C3 convertases capable of forming a physico-chemically stable convertase with an extended half-life of its decay-dissociation, providing resistance to the action of Factor H or the combined action of Factors H and I, and exerting little or negligible C5-cleaving activity, and/or wherein the recombinant human C3-derivative is selected from a group of C3 convertases providing a sequence identity with human C3 of at least 70%.

6. Composition according to any of the claims 2 to 5, wherein the inhibitor of IL-4/IL-13-mediated effects is a human IL-4 mutant, preferably with one to three point mutations, most preferaby in at least one of the positions R121, Y124, and S125.

7. Composition according to any of the claims 3 to 6, wherein the first and/or the second matrix is a biodegradable or biostable polymer, preferably biodegradable, more preferably thermogelling, even more preferably reverse thermogelling, in particular selected from the group consisting of polyethylene, polypropylene, polyethylene oxide (PEO), polypropylene oxide (PPO), polyurethane, polyurea, polyamides, polycarbonates, polyaldehydes, polyorthoesters, polyiminocarbonates, poly caprolactone (PCL), poly-D,L-lactic acid (PDLLA), poly-L-lactic acid (PLLA), lactides of said lactic acids, polyphosphazenes, polyglycolic acids, albumin, monomethoxypoly(ethylene glycol) (MPEG), trimethylated chitosan derivatives, or copolymers or mixtures of any of the above including poly(lactic-co-glycolic acid) (PLGA), copolymers of L-lactide and D,L-lactide, polyester copolymers, diblock copolymers consisting of MPEG and PCL, MPEG and PCL-ran-PLLA, MPEG and PLGA, PEO and PLLA, trimethylated chitosan and a,ß-glycerophosphate, triblock copolymers consisting of PEO and PLLA, PLGA-PEG-PLGA, PEG-PLGA-PEG, PEG-PCL-PEG, and PEO-PPO-PEO (Poloxamers), wherein the polymer is most preferably reverse thermogelling and wherein the gelling temperature is between 20°C and 40°C, preferably between 25°C and 35°C, and/or wherein the 90% degradation of the polymer weight in body environment and/or 90% release of the antigen and/or recombinant human C3-derivative from the polymer is completed within 1 to 10 days, preferably within 1 to 3 days.

8. Composition according to any of the claims 4 to 7, wherein the second matrix is selected from the group consisting of aluminium salts, oil in water or water in oil emulsions, e.g. FIA, Montanide, Adjuvant 65 and Lipovant, liposomes, polymeric microsphere adjuvants, and virosomes, preferably selected from aluminium phosphate or aluminium hydroxide gels.

9. Composition according to one of the claims 2 to 8, wherein all components are mixed as a single preparation, or wherein the first matrix and the rhC3-derivative and, optionally, the inhibitor of IL-4/IL-13-mediated effects are mixed as a first preparation and the antigen and the second matrix according are mixed as a second preparation and/or wherein the composition is galenically prepared for administration by injection or by implantation, subcutaneously, intradermally, intramuscularly, nasally, transbucally, transmucosally, sublingually, rectally, vaginally, intraocularly, intra tumor, or topically.

10. Use of a composition according to one of the claims 1 to 9 for local C3 depletion and local IL-4/IL-13 inhibition in an organism, preferably a human, in need thereof, in particular for the treatment of T-cell-mediated diseases, preferably selected from the group consisting of, but not limited to, allergy, allergic asthma, acute respiratory stress (ARDS), type 1 diabetes, systemic lupus erythemadodes, glomerulonephritis, rheumatoid arthritis, multiple sclerosis, dermatomyositis, nephritis, Parkinson's disease, Alzheimer's disease, pemphigoid, sepsis, myocardial ischemia, acute myocardial infarction (AMI), reperfusion, hemodialysis, paroxysmal nocturnal hemoglobinuria (PNH), age-related macula degeneration (AMD), cardiopulmonary bypass (CPB), angioplasty, hyperacute rejection, transplant rejection, stroke, burns, spinal cord injury, and traumatic brain injury (TBI), wherein preferred fields of application include allergy, allergic asthma, and type 1 diabetes, and wherein the pharmaceutical composition is administered in a therapeutically effective dose.

11. Method for manufacturing a pharmaceutical composition according to one of the claims 1 to 9, wherein the components are mixed with each other in a therapeutically effective quantity, wherein optionally galenic compounds are additionally admixed to one or all of the preparations.

12. Method for the modulation of effector T cell responses in patients with type 1 allergy and/or allergic asthma, wherein a composition according to one of the claims 1 to 9 is administered prior and/or during specific immunotherapy in a therapeutically effective dose, wherein at least one recombinant human C3 derivative according to claims is injected or implanted once or more at the site of allergen presentation, wherein the presented allergens are adsorbed onto the second matrix according to claim 11, preferably onto aluminium salts.

**Fig. 1**

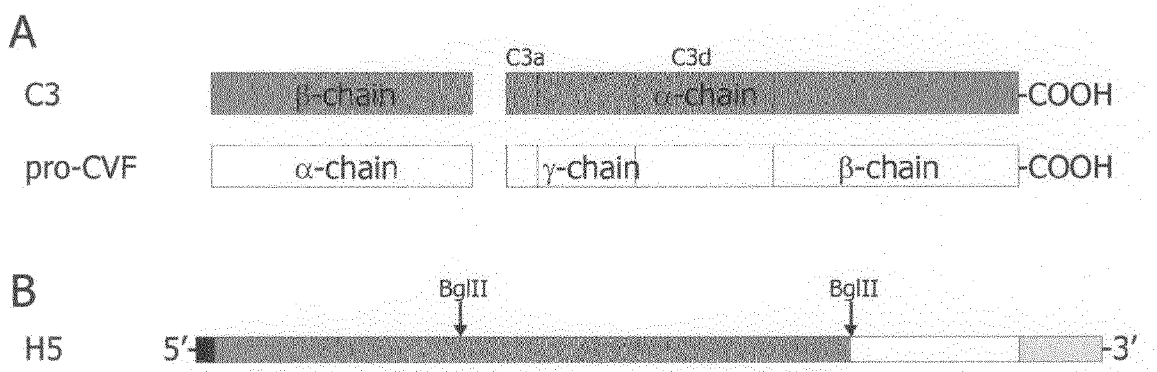
A
C3
C3a
C3d
β-chain
α-chain
-COOH
pro-CVF
α-chain
γ-chain
β-chain
-COOH
B
BglII
BglII
H5
5′
3′

Fig. 2

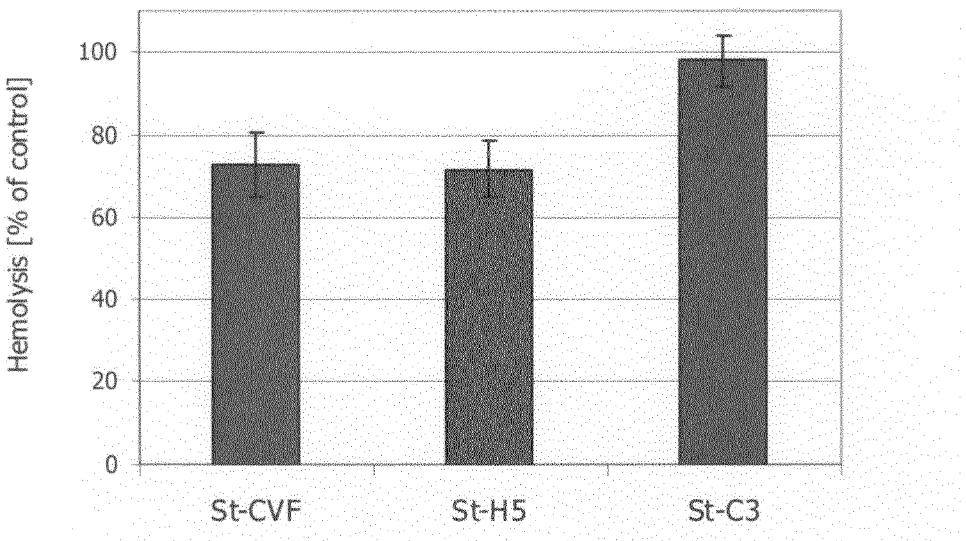

Fig. 3

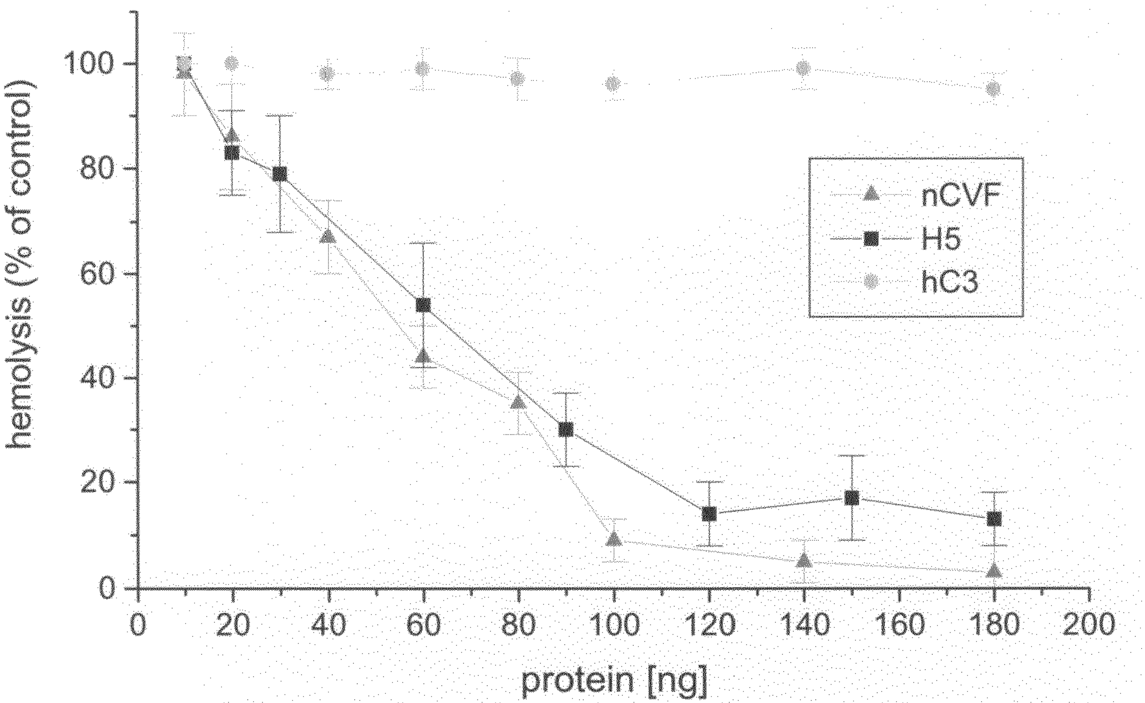
hemolysis (% of control)
100
80
60
40
20
0
0
20
40
60
80
100
120
140
160
180
200
protein [ng]
nCVF
H5
hC3

Fig. 4

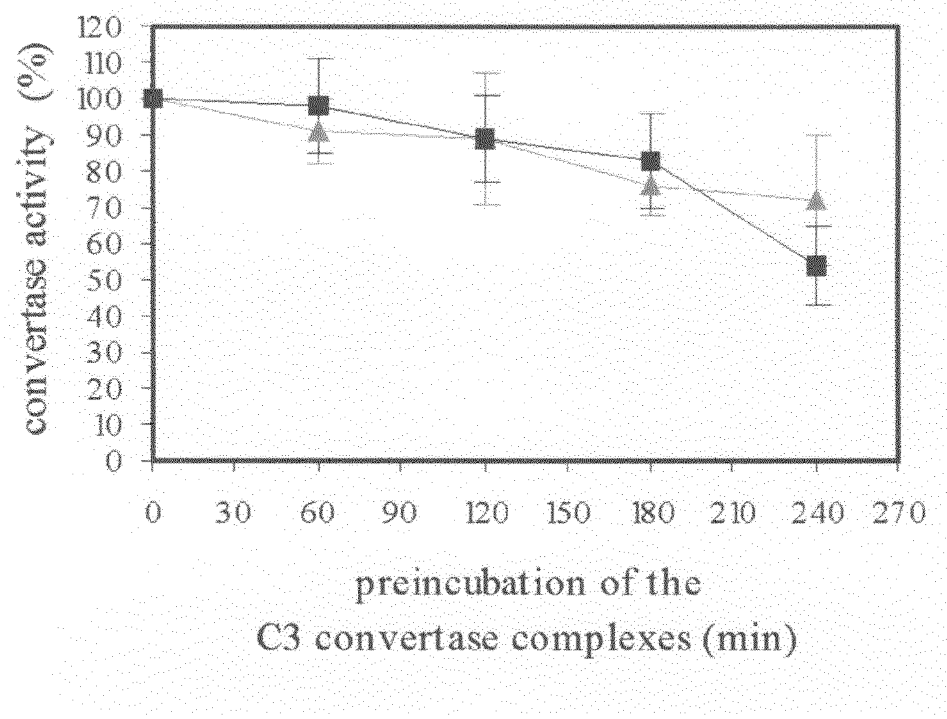

convertase activity (%)
120
110
100
90
80
70
60
50
40
30
20
10
0
0
30
60
90
120
150
180
210
240
270
preincubation of the
C3 convertase complexes (min)

**Fig. 5**

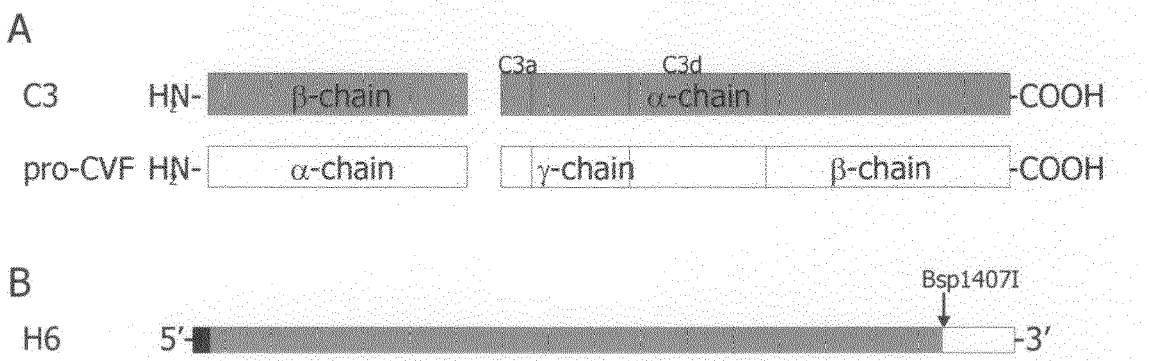
A
C3
$H_2N$-
β-chain
C3a
C3d
α-chain
-COOH
pro-CVF
$H_2N$-
α-chain
γ-chain
β-chain
-COOH
B
H6
5′-
Bsp1407I
-3′

**Fig. 6**

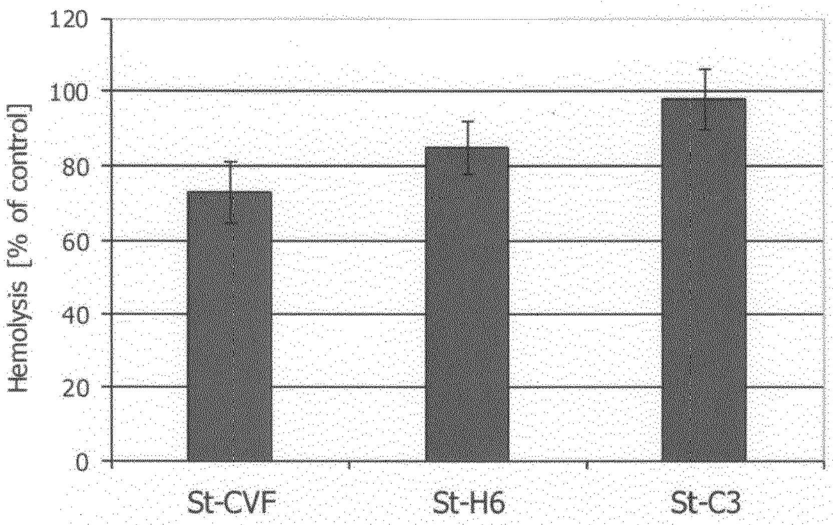

# Fig. 7

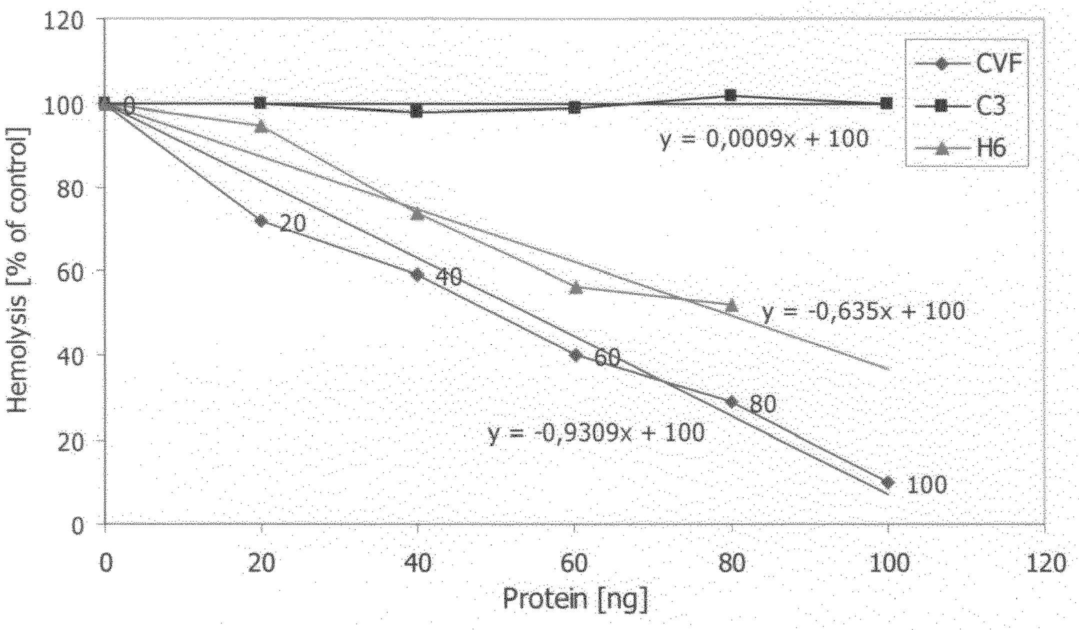
120
100
80
60
40
20
0
Hemolysis [% of control]
0
20
40
60
80
100
120
Protein [ng]
CVF
C3
H6
y = 0,0009x + 100
y = -0,635x + 100
y = -0,9309x + 100
0
20
40
60
80
100

**Fig. 8**

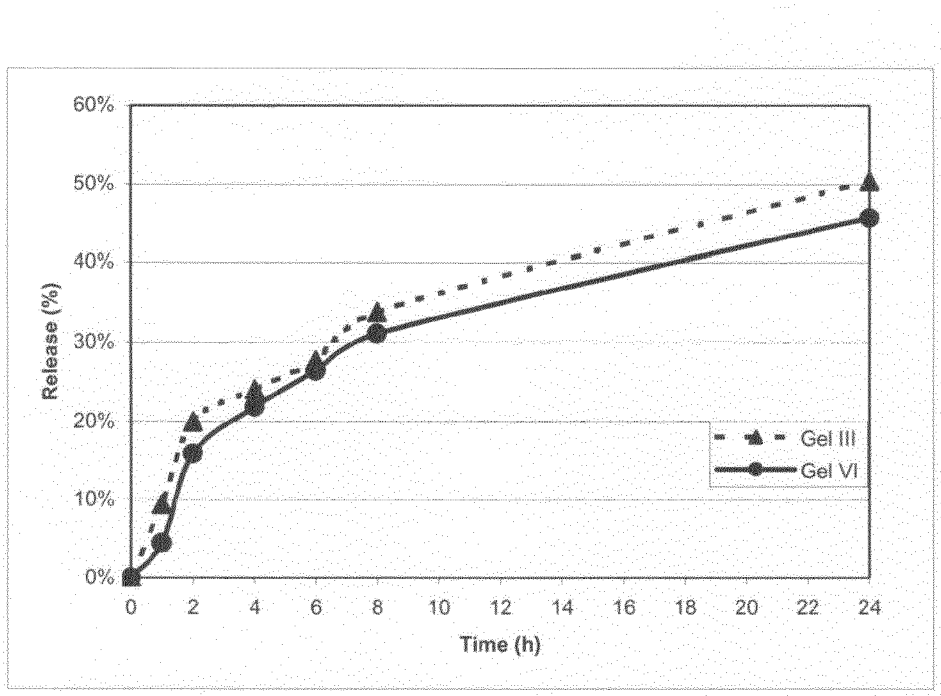
60%
50%
40%
30%
20%
10%
0%
Release (%)
0
2
4
6
8
10
12
14
16
18
20
22
24
Time (h)
Gel III
Gel VI

Europäisches Patentamt
European Patent Office
Office européen des brevets

# EUROPEAN SEARCH REPORT

Application Number
EP 12 07 5059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FRITZINGER DAVID C ET AL: "Derivatives of human complement component C3 for therapeutic complement depletion: a novel class of therapeutic agents.", ADVANCES IN EXPERIMENTAL MEDICINE AND BIOLOGY 2008, vol. 632, 2008, pages 293-307, XP002688644, ISSN: 0065-2598 | 1,5,9-11 | INV. A61K39/00 |
| Y | * figure 6 * | 2-4,6-8, 12 | |
| X | S.-Y. WANG ET AL: "Depletion of the C3 component of complement enhances the ability of rituximab-coated target cells to activate human NK cells and improves the efficacy of monoclonal antibody therapy in an in vivo model", BLOOD, vol. 114, no. 26, 17 December 2009 (2009-12-17), pages 5322-5330, XP055046784, ISSN: 0006-4971, DOI: 10.1182/blood-2009-01-200469 | 1,5,9,11 | TECHNICAL FIELDS SEARCHED (IPC) |
| Y | * the whole document * * in particular abstract, pg. 5323 figures * | 2-4,6-8, 10,12 | A61K |

-/--

The present search report has been drawn up for all claims

| Place of search | Date of completion of the search | Examiner |
|---|---|---|
| Munich | 11 December 2012 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 12 07 5059

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | ALI ET AL: "Regulation of human mast cell and basophil function by anaphylatoxins C3a and C5a", IMMUNOLOGY LETTERS, ELSEVIER BV, NL, vol. 128, no. 1, 18 January 2010 (2010-01-18), pages 36-45, XP026872660, ISSN: 0165-2478 [retrieved on 2009-11-04] * the whole document * * in particular pg. 37 references 36 and 38 * ----- | 10,12 | |
| Y | GRUNEWALD S M ET AL: "An Antagonistic IL-4 Mutant Prevents Type I Allergy in the Mouse: Inhibition of the IL-4/IL-13 Receptor System Completely Abrogates Humoral Immune Response to Allergen and Development of Allergic Symptoms In Vivo", THE JOURNAL OF IMMUNOLOGY, THE AMERICAN ASSOCIATION OF IMMUNOLOGISTS, US, vol. 160, no. 8, 15 April 1998 (1998-04-15), pages 4004-4009, XP002102659, ISSN: 0022-1767 * abstract * * page 4005, left-hand column * ----- -/-- | 2,6 | TECHNICAL FIELDS SEARCHED (IPC) |

2

The present search report has been drawn up for all claims

| Place of search | Date of completion of the search | Examiner |
|---|---|---|
| Munich | 11 December 2012 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 07 5059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | STEINKE J W: "Current Prospective of Anti-IL-4, -IL-9, and -IL-13 Therapies in Allergic Disease", RECENT PATENTS ON INFLAMMATION & ALLERGY DRUG DISCOVERY, BENTHAM SCIENCE, vol. 4, no. 3, 1 November 2010 (2010-11-01), pages 222-230, XP002674979, ISSN: 1872-213X * abstract * * tables 1-2 * ----- | 2,6 | |
| Y | ANIL MAHAPATRO ET AL: "Biodegradable nanoparticles are excellent vehicle for site directed in-vivo delivery of drugs and vaccines", JOURNAL OF NANOBIOTECHNOLOGY, vol. 9, no. 1, 1 January 2011 (2011-01-01), page 55, XP055045680, ISSN: 1477-3155, DOI: 10.1186/1477-3155-9-55 * the whole document * ----- | 3,4,7,8 | TECHNICAL FIELDS SEARCHED (IPC) |
| Y | PANYAM J ET AL: "Biodegradable nanoparticles for drug and gene delivery to cells and tissue", ADVANCED DRUG DELIVERY REVIEWS, ELSEVIER BV, AMSTERDAM, NL, vol. 55, no. 3, 24 February 2003 (2003-02-24), pages 329-347, XP008096954, ISSN: 0169-409X, DOI: 10.1016/S0169-409X(02)00228-4 * the whole document * ----- -/-- | 3,4,7,8 | |

The present search report has been drawn up for all claims

| Place of search | Date of completion of the search | Examiner |
|---|---|---|
| Munich | 11 December 2012 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 12 07 5059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | QIAO M ET AL: "Injectable biodegradable temperature-responsive PLGA-PEG-PLGA copolymers: Synthesis and effect of copolymer composition on the drug release from the copolymer-based hydrogels", INTERNATIONAL JOURNAL OF PHARMACEUTICS, ELSEVIER BV, NL, vol. 294, no. 1-2, 27 April 2005 (2005-04-27), pages 103-112, XP027624076, ISSN: 0378-5173 [retrieved on 2005-04-27] * the whole document * ----- | 3,4,7,8 | |
| Y | SPILLNER EDZARD ET AL: "Inactivation of complement by recombinant human C3 derivatives", ADVANCES IN EXPERIMENTAL MEDICINE AND BIOLOGY SPRINGER, 233 SPRING STREET, NEW YORK, NY 10013, UNITED STATES SERIES : ADVANCES IN EXPERIMENTAL MEDICINE AND BIOLOGY (ISSN 0065-2598(PRINT)), 2006, pages 347-360, XP009165449, & 3RD WORKSHOP ON COMPLEMENT ASSOCIATED DISEASES, ANIMAL MODELS AND THERAPEUTICS; RHODES, GREECE; JUNE 05 -10, 2005 * the whole document * * in particular figures 1-3 page 355 * ----- | 10,12 | TECHNICAL FIELDS SEARCHED (IPC) |

-/--

The present search report has been drawn up for all claims

| Place of search | Date of completion of the search | Examiner |
|---|---|---|
| Munich | 11 December 2012 | Bernhardt, Wiebke |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 07 5059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KRUG N ET AL: "Complement factors C3a and C5a are increased in bronchoalveolar lavage fluid after segmental allergen provocation in subjects with asthma", AMERICAN JOURNAL OF RESPIRATORY AND CRITICAL CARE MEDICINE, AMERICAN LUNG ASSOCIATION, NEW YORK, NY, US, vol. 164, no. 10 Part 1, 15 November 2001 (2001-11-15), pages 1841-1843, XP002363574, ISSN: 1073-449X * the whole document * ----- | 10,12 | |
| A | PENG QI ET AL: "Dendritic cell synthesis of C3 is required for full T cell activation and development of a Th1 phenotype", THE JOURNAL OF IMMUNOLOGY, THE AMERICAN ASSOCIATION OF IMMUNOLOGISTS, US, vol. 176, no. 6, 15 March 2006 (2006-03-15), pages 3330-3341, XP009165302, ISSN: 0022-1767 * the whole document * ----- | 1-12 | TECHNICAL FIELDS SEARCHED (IPC) |
| | -/-- | | |

The present search report has been drawn up for all claims

2

| Place of search | Date of completion of the search | Examiner |
|---|---|---|
| Munich | 11 December 2012 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 07 5059

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FRITZINGER D ET AL: "A novel concept for the treatment of paroxysmal nocturnal haemoglobinuria (PNH): Complement depletion with a human C3 derivative with cobra venom factor-like activity prevents lysis of PNH erythrocytes", MOLECULAR IMMUNOLOGY, PERGAMON, GB, vol. 45, no. 16, 1 October 2008 (2008-10-01), page 4177, XP025896416, ISSN: 0161-5890, DOI: 10.1016/J.MOLIMM.2008.08.245 [retrieved on 2008-10-01] * the whole document * ----- | 1-12 | |
| A | CARL-WILHELM VOGEL ET AL: "Cobra venom factor: Structure, function, and humanization for therapeutic complement depletion", TOXICON, vol. 56, no. 7, 1 December 2010 (2010-12-01), pages 1198-1222, XP055046258, ISSN: 0041-0101, DOI: 10.1016/j.toxicon.2010.04.007 * the whole document * ----- | 1-12 | TECHNICAL FIELDS SEARCHED (IPC) |
| A | WO 2005/003159 A1 (KOELN JOHANNA [DE]; BREDEHORST REINHARD [DE]; SPILLNER EDZARD [DE]) 13 January 2005 (2005-01-13) * the whole document * ----- | 1-12 | |

The present search report has been drawn up for all claims

2

| Place of search | Date of completion of the search | Examiner |
|---|---|---|
| Munich | 11 December 2012 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 07 5059

This annex lists the patent family members relating to the patent documents cited in the above-mentioned European search report.
The members are as contained in the European Patent Office EDP file on
The European Patent Office is in no way liable for these particulars which are merely given for the purpose of information.

11-12-2012

| Patent document cited in search report | Publication date | Patent family member(s) | Publication date |
|---|---|---|---|
| WO 2005003159 A1 | 13-01-2005 | EP 1644409 A1 | 12-04-2006 |
| | | US 2005079585 A1 | 14-04-2005 |
| | | US 2009270324 A1 | 29-10-2009 |
| | | WO 2005003159 A1 | 13-01-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7553931 B
- US 8119769 B
- US 20100179092 A1
- WO 9918142 A
- EP 11075261 A
- WO 2009081201 A2
- WO 0204009 A2
- US 20110002013 A1
- US 6028176 A
- US 5723118 A
- US 5599905 A
- EP 1785490 B1
- US 7553931 B2
- US 200505119 W
- US 20100179092 A
- WO 02102309 A, Quiao


### Non-patent literature cited in the description


- **LONG, A.A.** Monoclonal antibodies and other biologic agents in the treatment of asthma. *MAbs,* 2009, vol. 1 (3), 237-246
- **LETZELTER et al.** *Eur. J. Biochem.,* 1998, vol. 257, 11-20
- **KRUSE, N. et al.** *EMBO J.,* 1993, vol. 12, 5121-5129
- **WENZEL, S. et al.** *Lancet,* 2007, vol. 370, 1422-1431
- *J. Immunl.,* 1998, vol. 160, 404-4009
- **KIOI, M. et al.** *Cell. Immunol.,* 2004, vol. 229, 41-51
- **TONY, H.P. et al.** *Eur. J. Biochem.,* 1994, vol. 225, 659-665
- **IDZERDA et al.** *J. Exp. Med.,* 1990, vol. 171 (3), 861-873
- **MOSLEY, B. et al.** *Cell,* 1989, vol. 59, 335-348
- **BORISH, L.C. et al.** *J. Allergy Clin. Immunol.,* 2001, vol. 107, 963-970
- **ECONOMIDES, A.E. et al.** *Nature Med,* 2003, vol. 9, 47-52
- **ANDREWS, A.-L. et al.** *J. Immunol.,* 2006, vol. 176, 7456-7461
- **HOLGATE, S.T. et al.** *Nat. Rev. Immunol.,* 2008, vol. 8, 218-230
- **HART, T.K. et al.** *Clin. Exp. Immunol.,* 2002, vol. 130, 93-100
- **GAUVREAU, G.M. et al.** The effects of IL-13 blockade on allergen-induced airway responses in mild atopic asthma. *Am. J. Respir. Crit. Care Med.,* 05 November 2010
- **CORREN, J. et al.** *Am. J. Respir. Crit. Care Med.,* 2010, vol. 181, 788-796
- **FAMULOK, M. et al.** *Curr. Top. Microbiol. Immunol.,* 1999, vol. 243, 123-136
- **YAN, A.C. et al.** *Frontiers Biosci.,* 2005, vol. 10, 1802-1827
- **ABE, M. et al.** *J. Immunol.,* 2001, vol. 167, 4651-4660
- **AKBAR, A.N. et al.** *Immunology,* 2003, vol. 109, 319-325
- **ANDERSSON, A. et al.** *Eur. J. Immunol.,* 1997, vol. 27, 1762-1768
- **ANDRÄ, J. et al.** *Mol. Immunol.,* 2002, vol. 39, 357-365
- **AVNI, O. et al.** *Nat. Immunol.,* 2002, vol. 3, 643-651
- **BALLOW, M. ; COCHRANE, C. G.** *J. Immunol.,* 1969, vol. 103, 944-952
- **BANCHEREAU, J. et al.** *Annu. Rev. Immunol.,* 2000, vol. 18, 767-811
- **BAO, L. et al.** *J. Clin. Invest.,* 2009, vol. 119, 1264-1274
- **BAUTSCH, W. et al.** *J. Immunol.,* 2000, vol. 165, 5401-5405
- **BLUESTONE, J.A. et al.** *Nature,* 2010, vol. 464, 1293
- **CALABRESI, P.A. et al.** *J. Neuroimmunol.,* 2003, vol. 139, 58-65
- **CARDONE, J. et al.** *Nat. Immunol.,* 2010, vol. 11, 862-872
- **CARROL, M.C.** *Nat. Immunol.,* 2004, vol. 5, 981-986
- **CHOI, S. et al.** *Pharmaceut. Res.,* 2003, vol. 20, 2008-2010
- **COCHRANE, C. G. et al.** *J. Immunol.,* 1970, vol. 105, 55-69
- **COPPIETERS, K.T. et al.** *J. Exp. Med.,* 2012, vol. 209, 51-60

- **DROUIN, S.M. et al.** *J. Immunol.,* 2001, vol. 167, 4141-4145
- **DROUIN, S.M. et al.** *Am. J. Respir. Crit. Care Med.,* 2006, vol. 173, 852-857
- **DUNKELBERGER, J.R. ; SONG, W.-C.** *Cell Res.,* 2010, vol. 20, 34-50
- **DUSCHL, A. et al.** *Eur. Cytokine Netw.,* 1992, vol. 3, 97-102
- **EHIRCHIOU, D. et al.** *J. Exp. Med.,* 2007, vol. 204, 1510-1524
- **EZEKOWITZ, R.A. et al.** *J. Exp. Med.,* 1984, vol. 150, 244-260
- **FANG, C. et al.** *Blood,* 2009, vol. 114, 1005-1015
- **FARRAR, C.A. et al.** *FASEB J.,* 2006, vol. 20, 217-226
- **FONTENOT, J.D. et al.** *Nat. Immunol.,* 2003, vol. 4, 330-336
- **FRITZINGER, D.C. et al.** *Mol. Immunol.,* 2008, vol. 45, 4112
- **FRITZINGER, D.C. et al.** *Adv. Exp. Med. Biol.,* 2008, vol. 632, 293-307
- **FRITZINGER, D.C. et al.** *Dev. Comp. Immunol.,* 2009, vol. 33, 105-116
- **GERARD, PN.P. ; GERARD, C.** *Curr. Opin. Immunol.,* 2002, vol. 14, 705-708
- **GHANNAM, A. et al.** *J. Immunol.,* 2008, vol. 181, 5158-5166
- **GILBERT, J.C. et al.** *J. Control. Release,* 1987, vol. 5, 113-118
- **GONG, C.Y. et al.** *Int. J. Pharm.,* 2009, vol. 365, 89-99
- **GONG, C.Y. et al.** *BMC Biotechnol.,* 2009, vol. 9, 8
- **GROTE, A. et al.** *NAR,* 2005, vol. 33, W526-531
- **GRUNEWALD, S.M. et al.** *J. Biol. Chem.,* 1997, vol. 272, 1480-1483
- **GUELER, F. et al.** *J. Am. Soc. Nephrol.,* 2008, vol. 19, 2302-2312
- **HAGENAARS, N. et al.** *J. Control. Release,* 2010, vol. 144, 17-24
- **HASHIMOTO, M. et al.** *J. Exp. Med.,* 2010, vol. 207, 1135-1143
- **HAWLISCH, H. et al.** *Mol. Immunol.,* 2004, vol. 41, 123-131
- **HEEGER, P.S. ; KEMPER, C.** *Immunobiology,* 2012, vol. 217, 216-224
- **HERING, B.J. et al.** *Am. J. Transplant.,* 2004, vol. 4, 390-401
- **HEROLD, K.C. et al.** *N. Engl. J. Med.,* 2002, vol. 346, 1692-1698
- **HOMANN, D. ; VON HERRATH, M.** *Clin. Immunol.,* 2004, vol. 112, 202-209
- **HUMBLES, A.A. et al.** *Nature,* 2000, vol. 406, 998-1001
- **HYUN, H. et al.** *Biomacromolecules,* 2007, vol. 8, 1093-1100
- **JANSSEN, B.J.C. et al.** *EMBO J.,* 2009, vol. 28, 2469-2478
- **JEONG, B. et al.** *Nature,* 1997, vol. 388, 860-862
- **JUEDES, A.E. ; VON HERRATH, M.G.** *Diabetes Metab. Res. Rev.,* 2004, vol. 20, 446-451
- **KANG, Y.M. et al.** *Biomaterials,* 2010, vol. 31, 2453-2460
- **KARP, C.I. et al.** *Nat. Immunol.,* 2000, vol. 1, 221-226
- **KARSTEN, C.M. ; KÖHL, J.** *Nat. Immunol.,* 2010, vol. 11, 775-777
- **KAWAMOTO, S. et al.** *J. Clin. Invest.,* 2004, vol. 114, 399-407
- **KEMPER, C. ; ATKINSON, J.P.** *Nat. Rev. Immunol.,* 2007, vol. 7, 9-18
- **KEYMEULEN, B.** *N. Engl. J. Med.,* 2005, vol. 352, 2598-2608
- **KNIP, M. et al.** *Diabetes,* 2005, vol. 54, S125-S136
- **KÖHL, J. et al.** *J. Clin. Invest.,* 2006, vol. 116, 783-796
- **KÖLLN, J. et al.** *J. Immunol.,* 2004, vol. 173, 5540-5545
- **KÖLLN, J. et al.** *Immunol. Lett.,* 2005, vol. 98, 49-56
- **KRETSCHMER, K. et al.** *Nat. Immunol.,* 2005, vol. 6, 1219-1227
- **KUKREJA, A. et al.** *J. Clin. Invest.,* 2002, vol. 109, 131-140
- **KWAN, W.-H. et al.** *Immunol. Res.,* 2012
- **LI, K. et al.** *Blood,* 2008, vol. 112, 5084-5094
- **LI, Q. et al.** *J. Am. Soc. Nephrol.,* 2010, vol. 21, 1344-1353
- **LIN, M. et al.** *Diabetes,* 2010, vol. 59, 2247-2252
- **LIU, J. et al.** *J. Immunol.,* 2008, vol. 180, 5882-5889
- **LUDVIGSSON, J. et al.** *N. Engl. J. Med.,* 2008, vol. 359, 676-781
- *MacroGenics press release,* 20 October 2011
- **MATHEWS, K.P.** *Ann. Intern. Med.,* 1980, vol. 93, 443-445
- **MORGAN, B.P. ; GASQUE, P.** *Clin. Exp. Immunol.,* 1997, vol. 107, 1-7
- **MULLIGAN, M.S. et al.** *J. Clin. Invest.,* 1996, vol. 98, 503-512
- **MUNEGOWDA, M.A. et al.** *J. Clin. Immunol.,* 2011, vol. 31, 811-826
- **PAI, S.S. et al.** *AAPS J.,* 2009, vol. 11, 88-98
- **PAVLOV, V. et al.** *J. Immunol.,* 2008, vol. 181, 4580-4589
- **PENG, Q. et al.** *J. Immunol.,* 2006, vol. 176, 3330-3341
- **PENG, Q. et al.** *Blood,* 2008, vol. 111, 2452-2461
- **PENG, K.-T. et al.** *Biomaterials,* 2010, vol. 31, 5227-5236

- **PETRELLA, E.C. et al.** *J. Immunol. Meth.,* 1987, vol. 104, 159-172
- **PRATT, J.R. et al.** *Nat. Med.,* 2002, vol. 8, 582-587
- **PUIGBO P. et al.** *NAR,* 2007, vol. 35, W126-131
- **QIAO, M. et al.** *Int. J. Pharm.,* 2005, vol. 294, 103-112
- **RAEDLER, H. et al.** *Am. J. Transplant.,* 2009, vol. 9, 1784-1795
- **RAEDLER, H. et al.** *Am. J. Transplant.*
- **RAZEGHIFARD, M.R.** *Prot. Expr. Purif.,* 2004, vol. 37, 180-186
- **REIS ; SOUSA.** *Nat. Rev. Immunol.,* 2006, vol. 6, 476-483
- **REIS ; SOUSA et al.** *Immunobiology,* 2007, vol. 212, 151-157
- **SAKAGUCHI, S. et al.** *J. Exp. Med.,* 1985, vol. 161, 72-87
- **SANDOR, N. et al.** *Mol. Immunol.,* 2009, vol. 47, 438-448
- **SHORTMAN, K. ; LIU, Y.J.** *Nat. Rev. Immunol.,* 2002, vol. 2, 151-161
- **SINHA, V.R. et al.** *Int. J. Pharm.,* 2004, vol. 278, 1-23
- **SKYLER, J.S. et al.** *Diabetes Care,* 2005, vol. 28, 1068-1076
- **STÄGER, S. et al.** *Nat. Med.,* 2003, vol. 9, 1287-1292
- **STRAINIC, M.G. et al.** *Immunity,* 2008, vol. 28, 425-435
- **SURESH, M. et al.** *J. Immunol.,* 2003, vol. 170, 788-794
- **SZABO, S.J. et al.** *Immunity,* 1995, vol. 2, 665-675
- **TANIGUCHI, S. et al.** *Transplantation,* 1996, vol. 62, 678-681
- **TILL, G.O. et al.** *J. Clin. Invest.,* 1992, vol. 69, 1126-1135
- **TILL, G.O. et al.** *Am. J. Pathol.,* 1987, vol. 129, 44-53
- **VAN KOOTEN, C. et al.** *Mol. Immunol.,* 2008, vol. 45, 4064-4072
- **VERSCHOOR, A. et al.** *J. Immunol.,* 2003, vol. 171, 5363-5371
- **VIEYRA, M. et al.** *Am. J. Pathol.,* 2011, vol. 179, 766-744
- **VIGLIETTA, V. et al.** *J. Exp. Med.,* 2004, vol. 199, 971-979
- **VOGEL, C. W. ; MÜLLER-EBERHARD, H. J.** *J. Biol. Chem.,* 1982, vol. 257, 8292-8299
- **VOGEL, C. W. et al.** *Haematol. Blood Transfus,* 1985, vol. 29, 514-517
- **VOGEL, C.W. ; FRITZINGER, D.C.** *Curr. Pharm. Des.,* 2007, vol. 13, 2916-2926
- **VOGEL, C.W. ; FRITZINGER, D.C.** *Toxicon,* 2010, vol. 56, 1198-1222
- **WALKER, M.R. et al.** *J. Clin. Invest.,* 2003, vol. 112, 1437-1443
- **WILLEMSE, J.L. et al.** *Clin. Chim. Acta,* 2008, vol. 389, 181-182
- **WU, Y. et al.** *Biomaterials,* 2012, vol. 33, 2351-3260
- **YAMAZAKI, S. et al.** *J. Exp. Med.,* 2003, vol. 198, 235-247
- **YIAMOUYIANNIS, C.A. et al.** *Am. J. Pathol.,* 1999, vol. 154, 1911-1921
- **ZHANG, J. et al.** *Biomacromolecules,* 2006, vol. 7, 2492-2500
- **ZHOU, W. et al.** *Blood,* 2006, vol. 107, 2461-2469
- **ZHOU, W. et al.** *Mol. Immunol.,* 2007, vol. 44, 57-63
- **ZHOU, W.** *Immunobiology,* 2012, vol. 217, 225-234

SEQUENCE LISTING

<110> PLS-Design GmbH

<120> Modulation of effector T cell responce

<160> 9

<170> BiSSAP 1.0

<210> 1
<211> 580
<212> DNA
<213> Mus musculus musculus

<220>
<221> source
<222> 1..580
<223> /mol_type="DNA"
/organism="Mus musculus musculus"

<400> 1
```
atttgttagc atctcttgat aaacttaatt gtctctcgtc actgacggca cagagctatt      60

gatgggtctc aacccccagc tagttgtcat cctgctcttc tttctcgaat gtaccaggag     120

ccatatccac ggatgcgaca aaaatcactt gagagagatc atcggcattt tgaacgaggt     180

cacaggagaa gggacgccat gcacggagat ggatgtgcca aacgtcctca cagcaacgaa     240

gaacaccaca gagagtgagc tcgtctgtag ggcttccaag gtgcttcgca tattttattt     300

aaaacatggg aaaactccat gcttgaagaa gaactctagt gttctcatgg agctgcagag     360

actctttcgg gcttttcgat gcctggattc atcgataagc tgcaccatga atgagtccaa     420

gtccacatca ctgaaagact tcctggaaag cctaaagagc atcatgcaaa tggattactc     480

gtagtactga gccaccatgc tttaacttat gaatttttaa tggttttatt tttaatattt     540

atatatttat aattcataaa ataaaatatt tgtataatgt                           580
```

<210> 2
<211> 140
<212> PRT
<213> Mus musculus musculus

<220>
<221> SOURCE
<222> 1..140
<223> /mol_type="protein"
/note="1-20 signal peptide"
/organism="Mus musculus musculus"

<400> 2
```
Met Gly Leu Asn Pro Gln Leu Val Val Ile Leu Leu Phe Phe Leu Glu
1               5                   10                  15
Cys Thr Arg Ser His Ile His Gly Cys Asp Lys Asn His Leu Arg Glu
            20                  25                  30
Ile Ile Gly Ile Leu Asn Glu Val Thr Gly Glu Gly Thr Pro Cys Thr
        35                  40                  45
Glu Met Asp Val Pro Asn Val Leu Thr Ala Thr Lys Asn Thr Thr Glu
    50                  55                  60
Ser Glu Leu Val Cys Arg Ala Ser Lys Val Leu Arg Ile Phe Tyr Leu
65                  70                  75                  80
Lys His Gly Lys Thr Pro Cys Leu Lys Lys Asn Ser Ser Val Leu Met
                85                  90                  95
```

```
Glu Leu Gln Arg Leu Phe Arg Ala Phe Arg Cys Leu Asp Ser Ser Ile
            100                 105                 110
Ser Cys Thr Met Asn Glu Ser Lys Ser Thr Ser Leu Lys Asp Phe Leu
        115                 120                 125
Glu Ser Leu Lys Ser Ile Met Gln Met Asp Tyr Ser
    130                 135                 140

<210> 3
<211> 423
<212> DNA
<213> Artificial Sequence

<220>
<221> source
<222> 1..423
<223> /mol_type="DNA"
      /note="Ref.: Puigbo et al 2007, NAR 35:W126-131; Grote et al 2005
      , NAR 33:W525-531"
      /organism="Artificial Sequence"

<400> 3
atgggttctt ctcaccacca ccaccaccac tcttctggtc tggttccgcg tggttctcac      60

atgcacatcc acggttgcga caaaaaccac ctgcgtgaaa tcatcggtat cctgaacgaa     120

gttaccggtg aaggtacccc gtgcaccgaa atggacgttc cgaacgttct gaccgcgacc     180

aaaaacacca ccgaatctga actggtttgc cgtgcgtcta aagttctgcg tatcttctac     240

ctgaaacacg gtaaaacccc gtgcctgaaa aaaaactctt ctgttctgat ggaactgcag     300

cgtctgttcc gtgcgttccg ttgcctggac tcttctatct cttgcaccat gaacgaatct     360

aaatctacct ctctgaaaga cttcctggaa tctctgaaat ctatcatgga catggacgac     420

tct                                                                   423


<210> 4
<211> 141
<212> PRT
<213> Artificial Sequence

<220>
<221> SOURCE
<222> 1..141
<223> /mol_type="protein"
      /note="N-terminal His-Tag and thrombin cleavage site"
      /organism="Artificial Sequence"

<400> 4
Met Gly Ser Ser His His His His His His Ser Ser Gly Leu Val Pro
1               5                   10                  15
Arg Gly Ser His Met His Ile His Gly Cys Asp Lys Asn His Leu Arg
            20                  25                  30
Glu Ile Ile Gly Ile Leu Asn Glu Val Thr Gly Glu Gly Thr Pro Cys
        35                  40                  45
Thr Glu Met Asp Val Pro Asn Val Leu Thr Ala Thr Lys Asn Thr Thr
    50                  55                  60
Glu Ser Glu Leu Val Cys Arg Ala Ser Lys Val Leu Arg Ile Phe Tyr
65                  70                  75                  80
Leu Lys His Gly Lys Thr Pro Cys Leu Lys Lys Asn Ser Ser Val Leu
                85                  90                  95
Met Glu Leu Gln Arg Leu Phe Arg Ala Phe Arg Cys Leu Asp Ser Ser
            100                 105                 110
Ile Ser Cys Thr Met Asn Glu Ser Lys Ser Thr Ser Leu Lys Asp Phe
        115                 120                 125
Leu Glu Ser Leu Lys Ser Ile Met Asp Met Asp Asp Ser
```

130 135 140

<210> 5
<211> 29
<212> DNA
<213> Artificial Sequence

<220>
<221> source
<222> 1..29
<223> /mol_type="DNA"
/note="Primer 1 for subcloning"
/organism="Artificial Sequence"

<400> 5
aaaaaaccat atgggttctt ctcaccacc 29

<210> 6
<211> 29
<212> DNA
<213> Artificial Sequence

<220>
<221> source
<222> 1..29
<223> /mol_type="DNA"
/note="Primer 3 for subcloning"
/organism="Artificial Sequence"

<400> 6
aaaactcgag ttaagagtcg tccatgtcc 29

<210> 7
<211> 1276
<212> PRT
<213> Artificial Sequence

<220>
<221> SOURCE
<222> 1..1276
<223> /mol_type="protein"
/note="Processed protein comprising C3 beta-, CVF gamma-, and CVF beta-chains"
/organism="Artificial Sequence"

<400> 7
Ser Pro Met Tyr Ser Ile Ile Thr Pro Asn Ile Leu Arg Leu Glu Ser
1 5 10 15
Glu Glu Thr Met Val Leu Glu Ala His Asp Ala Gln Gly Asp Val Pro
20 25 30
Val Thr Val Thr Val His Asp Phe Pro Gly Lys Lys Leu Val Leu Ser
35 40 45
Ser Glu Lys Thr Val Leu Thr Pro Ala Thr Asn His Met Gly Asn Val
50 55 60
Thr Phe Thr Ile Pro Ala Asn Arg Glu Phe Lys Ser Glu Lys Gly Arg
65 70 75 80
Asn Lys Phe Val Thr Val Gln Ala Thr Phe Gly Thr Gln Val Val Glu
85 90 95
Lys Val Val Leu Val Ser Leu Gln Ser Gly Tyr Leu Phe Ile Gln Thr
100 105 110
Asp Lys Thr Ile Tyr Thr Pro Gly Ser Thr Val Leu Tyr Arg Ile Phe
115 120 125
Thr Val Asn His Lys Leu Leu Pro Val Gly Arg Thr Val Met Val Asn
130 135 140
Ile Glu Asn Pro Glu Gly Ile Pro Val Lys Gln Asp Ser Leu Ser Ser
145 150 155 160

Gln Asn Gln Leu Gly Val Leu Pro Leu Ser Trp Asp Ile Pro Glu Leu
165 170 175

Val Asn Met Gly Gln Trp Lys Ile Arg Ala Tyr Tyr Glu Asn Ser Pro
180 185 190

Gln Gln Val Phe Ser Thr Glu Phe Glu Val Lys Glu Tyr Val Leu Pro
195 200 205

Ser Phe Glu Val Ile Val Glu Pro Thr Glu Lys Phe Tyr Tyr Ile Tyr
210 215 220

Asn Glu Lys Gly Leu Glu Val Thr Ile Thr Ala Arg Phe Leu Tyr Gly
225 230 235 240

Lys Lys Val Glu Gly Thr Ala Phe Val Ile Phe Gly Ile Gln Asp Gly
245 250 255

Glu Gln Arg Ile Ser Leu Pro Glu Ser Leu Lys Arg Ile Pro Ile Glu
260 265 270

Asp Gly Ser Gly Glu Val Val Leu Ser Arg Lys Val Leu Leu Asp Gly
275 280 285

Val Gln Asn Pro Arg Ala Glu Asp Leu Val Gly Lys Ser Leu Tyr Val
290 295 300

Ser Ala Thr Val Ile Leu His Ser Gly Ser Asp Met Val Gln Ala Glu
305 310 315 320

Arg Ser Gly Ile Pro Ile Val Thr Ser Pro Tyr Gln Ile His Phe Thr
325 330 335

Lys Thr Pro Lys Tyr Phe Lys Pro Gly Met Pro Phe Asp Leu Met Val
340 345 350

Phe Val Thr Asn Pro Asp Gly Ser Pro Ala Tyr Arg Val Pro Val Ala
355 360 365

Val Gln Gly Glu Asp Thr Val Gln Ser Leu Thr Gln Gly Asp Gly Val
370 375 380

Ala Lys Leu Ser Ile Asn Thr His Pro Ser Gln Lys Pro Leu Ser Ile
385 390 395 400

Thr Val Arg Thr Lys Lys Gln Glu Leu Ser Glu Ala Glu Gln Ala Thr
405 410 415

Arg Thr Met Gln Ala Leu Pro Tyr Ser Thr Val Gly Asn Ser Asn Asn
420 425 430

Tyr Leu His Leu Ser Val Leu Arg Thr Glu Leu Arg Pro Gly Glu Thr
435 440 445

Leu Asn Val Asn Phe Leu Leu Arg Met Asp Arg Ala His Glu Ala Lys
450 455 460

Ile Arg Tyr Tyr Thr Tyr Leu Ile Met Asn Lys Gly Arg Leu Leu Lys
465 470 475 480

Ala Gly Arg Gln Val Arg Glu Pro Gly Gln Asp Leu Val Val Leu Pro
485 490 495

Leu Ser Ile Thr Thr Asp Phe Ile Pro Ser Phe Arg Leu Val Ala Tyr
500 505 510

Tyr Thr Leu Ile Gly Ala Ser Gly Gln Arg Glu Val Val Ala Asp Ser
515 520 525

Val Trp Val Asp Val Lys Asp Ser Cys Val Gly Ser Leu Val Val Lys
530 535 540

Ser Gly Gln Ser Glu Asp Arg Gln Pro Val Pro Gly Gln Gln Met Thr
545 550 555 560

Leu Lys Ile Glu Gly Asp His Gly Ala Arg Val Val Leu Val Ala Val
565 570 575

Asp Lys Gly Val Phe Val Leu Asn Lys Lys Asn Lys Leu Thr Gln Ser
580 585 590

Lys Ile Trp Asp Val Val Glu Lys Ala Asp Ile Gly Cys Thr Pro Gly
595 600 605

Ser Gly Lys Asp Tyr Ala Gly Val Phe Ser Asp Ala Gly Leu Thr Phe
610 615 620

Thr Ser Ser Ser Gly Gln Gln Thr Ala Gln Arg Ala Glu Leu Gln Cys
625 630 635 640

Pro Gln Pro Ala Ala Asp Asp Asn Glu Asp Gly Phe Ile Ala Asp Ser
645 650 655

Asp Ile Ile Ser Arg Ser Asp Phe Pro Lys Ser Trp Leu Trp Leu Thr
660 665 670

Lys Asp Leu Thr Glu Glu Pro Asn Ser Gln Gly Ile Ser Ser Lys Thr
675 680 685

Met Ser Phe Tyr Leu Arg Asp Ser Ile Thr Thr Trp Val Val Leu Ala
690 695 700

Val Ser Phe Thr Pro Thr Lys Gly Ile Cys Val Ala Glu Pro Tyr Glu
705                 710                 715                 720
Ile Arg Val Met Lys Val Phe Phe Ile Asp Leu Gln Met Pro Tyr Ser
                725                 730                 735
Val Val Lys Asn Glu Gln Val Glu Ile Arg Ala Ile Leu His Asn Tyr
            740                 745                 750
Val Asn Glu Asp Ile Tyr Val Arg Val Glu Leu Leu Tyr Asn Pro Ala
        755                 760                 765
Phe Cys Ser Ala Ser Thr Lys Gly Gln Arg Tyr Arg Gln Gln Phe Pro
    770                 775                 780
Ile Lys Ala Leu Ser Ser Arg Ala Val Pro Phe Val Ile Val Pro Leu
785                 790                 795                 800
Glu Gln Gly Leu His Asp Val Glu Ile Lys Ala Ser Val Gln Glu Ala
                805                 810                 815
Leu Trp Ser Asp Gly Val Arg Lys Lys Leu Lys Val Val Pro Glu Gly
            820                 825                 830
Val Gln Lys Ser Ile Val Thr Ile Val Lys Leu Asp Pro Arg Ala Lys
        835                 840                 845
Gly Val Gly Gly Thr Gln Leu Glu Val Ile Lys Ala Arg Lys Leu Asp
    850                 855                 860
Asp Arg Val Pro Asp Thr Glu Ile Glu Thr Lys Ile Ile Ile Gln Gly
865                 870                 875                 880
Asp Pro Val Ala Gln Ile Ile Glu Asn Ser Ile Asp Gly Ser Lys Leu
                885                 890                 895
Asn Glu Ile Gln Met Pro Thr His Lys Asp Leu Asn Leu Asp Ile Thr
            900                 905                 910
Ile Glu Leu Pro Asp Arg Glu Val Pro Ile Arg Tyr Arg Ile Asn Tyr
        915                 920                 925
Glu Asn Ala Leu Leu Ala Arg Thr Val Glu Thr Lys Leu Asn Gln Asp
    930                 935                 940
Ile Thr Val Thr Ala Ser Gly Asp Gly Lys Ala Thr Met Thr Ile Leu
945                 950                 955                 960
Thr Phe Tyr Asn Ala Gln Leu Gln Glu Lys Ala Asn Val Cys Asn Lys
                965                 970                 975
Phe His Leu Asn Val Ser Val Glu Asn Ile His Leu Asn Ala Met Gly
            980                 985                 990
Ala Lys Gly Ala Leu Met Leu Lys Ile Cys Thr Arg Tyr Leu Gly Glu
        995                 1000                1005
Val Asp Ser Thr Met Thr Ile Ile Asp Ile Ser Met Leu Thr Gly Phe
    1010                1015                1020
Leu Pro Asp Ala Glu Asp Leu Thr Arg Leu Ser Lys Gly Val Asp Arg
1025                1030                1035                1040
Tyr Ile Ser Arg Tyr Glu Val Asp Asn Asn Met Ala Gln Lys Val Ala
                1045                1050                1055
Val Ile Ile Tyr Leu Asn Lys Val Ser His Ser Glu Asp Glu Cys Leu
            1060                1065                1070
His Phe Lys Ile Leu Lys His Phe Glu Val Gly Phe Ile Gln Pro Gly
        1075                1080                1085
Ser Val Lys Val Tyr Ser Tyr Tyr Asn Leu Asp Glu Lys Cys Thr Lys
    1090                1095                1100
Phe Tyr His Pro Asp Lys Gly Thr Gly Leu Leu Asn Lys Ile Cys Ile
1105                1110                1115                1120
Gly Asn Val Cys Arg Cys Ala Gly Glu Thr Cys Ser Ser Leu Asn His
                1125                1130                1135
Gln Glu Arg Ile Asp Val Pro Leu Gln Ile Glu Lys Ala Cys Glu Thr
            1140                1145                1150
Asn Val Asp Tyr Val Tyr Lys Thr Lys Leu Leu Arg Ile Glu Glu Gln
        1155                1160                1165
Asp Gly Asn Asp Ile Tyr Val Met Asp Val Leu Glu Val Ile Lys Gln
    1170                1175                1180
Gly Thr Asp Glu Asn Pro Arg Ala Lys Thr His Gln Tyr Ile Ser Gln
1185                1190                1195                1200
Arg Lys Cys Gln Glu Ala Leu Asn Leu Lys Val Asn Asp Asp Tyr Leu
                1205                1210                1215
Ile Trp Gly Ser Arg Ser Asp Leu Leu Pro Thr Lys Asp Lys Ile Ser
            1220                1225                1230
Tyr Ile Ile Thr Lys Asn Thr Trp Ile Glu Arg Trp Pro His Glu Asp
        1235                1240                1245

```
Glu Cys Gln Glu Glu Glu Phe Gln Lys Leu Cys Asp Asp Phe Ala Gln
    1250                1255                1260
Phe Ser Tyr Thr Leu Thr Glu Phe Gly Cys Pro Thr
1265                1270                1275
```

<210> 8
<211> 1637
<212> PRT
<213> Artificial Sequence

<220>
<221> SOURCE
<222> 1..1637
<223> /mol_type="protein"
/note="Processed H6 construct"
/organism="Artificial Sequence"

<400> 8

```
Ser Pro Met Tyr Ser Ile Ile Thr Pro Asn Ile Leu Arg Leu Glu Ser
1               5                   10                  15
Glu Glu Thr Met Val Leu Glu Ala His Asp Ala Gln Gly Asp Val Pro
            20                  25                  30
Val Thr Val Thr Val His Asp Phe Pro Gly Lys Lys Leu Val Leu Ser
        35                  40                  45
Ser Glu Lys Thr Val Leu Thr Pro Ala Thr Asn His Met Gly Asn Val
    50                  55                  60
Thr Phe Thr Ile Pro Ala Asn Arg Glu Phe Lys Ser Glu Lys Gly Arg
65                  70                  75                  80
Asn Lys Phe Val Thr Val Gln Ala Thr Phe Gly Thr Gln Val Val Glu
                85                  90                  95
Lys Val Val Leu Val Ser Leu Gln Ser Gly Tyr Leu Phe Ile Gln Thr
            100                 105                 110
Asp Lys Thr Ile Tyr Thr Pro Gly Ser Thr Val Leu Tyr Arg Ile Phe
        115                 120                 125
Thr Val Asn His Lys Leu Leu Pro Val Gly Arg Thr Val Met Val Asn
    130                 135                 140
Ile Glu Asn Pro Glu Gly Ile Pro Val Lys Gln Asp Ser Leu Ser Ser
145                 150                 155                 160
Gln Asn Gln Leu Gly Val Leu Pro Leu Ser Trp Asp Ile Pro Glu Leu
                165                 170                 175
Val Asn Met Gly Gln Trp Lys Ile Arg Ala Tyr Tyr Glu Asn Ser Pro
            180                 185                 190
Gln Gln Val Phe Ser Thr Glu Phe Glu Val Lys Glu Tyr Val Leu Pro
        195                 200                 205
Ser Phe Glu Val Ile Val Glu Pro Thr Glu Lys Phe Tyr Tyr Ile Tyr
    210                 215                 220
Asn Glu Lys Gly Leu Glu Val Thr Ile Thr Ala Arg Phe Leu Tyr Gly
225                 230                 235                 240
Lys Lys Val Glu Gly Thr Ala Phe Val Ile Phe Gly Ile Gln Asp Gly
                245                 250                 255
Glu Gln Arg Ile Ser Leu Pro Glu Ser Leu Lys Arg Ile Pro Ile Glu
            260                 265                 270
Asp Gly Ser Gly Glu Val Val Leu Ser Arg Lys Val Leu Leu Asp Gly
        275                 280                 285
Val Gln Asn Leu Arg Ala Glu Asp Leu Val Gly Lys Ser Leu Tyr Val
    290                 295                 300
Ser Ala Thr Val Ile Leu His Ser Gly Ser Asp Met Val Gln Ala Glu
305                 310                 315                 320
Arg Ser Gly Ile Pro Ile Val Thr Ser Pro Tyr Gln Ile His Phe Thr
                325                 330                 335
Lys Thr Pro Lys Tyr Phe Lys Pro Gly Met Pro Phe Asp Leu Met Val
            340                 345                 350
Phe Val Thr Asn Pro Asp Gly Ser Pro Ala Tyr Arg Val Pro Val Ala
        355                 360                 365
Val Gln Gly Glu Asp Thr Val Gln Ser Leu Thr Gln Gly Asp Gly Val
    370                 375                 380
Ala Lys Leu Ser Ile Asn Thr His Pro Ser Gln Lys Pro Leu Ser Ile
385                 390                 395                 400
```

```
Thr Val Arg Thr Lys Lys Gln Glu Leu Ser Glu Ala Glu Gln Ala Thr
                405                 410                 415
Arg Thr Met Gln Ala Leu Pro Tyr Ser Thr Val Gly Asn Ser Asn Asn
            420                 425                 430
Tyr Leu His Leu Ser Val Leu Arg Thr Glu Leu Arg Pro Gly Glu Thr
        435                 440                 445
Leu Asn Val Asn Phe Leu Leu Arg Met Asp Arg Ala His Glu Ala Lys
    450                 455                 460
Ile Arg Tyr Tyr Thr Tyr Leu Ile Met Asn Lys Gly Arg Leu Leu Lys
465                 470                 475                 480
Ala Gly Arg Gln Val Arg Glu Pro Gly Gln Asp Leu Val Val Leu Pro
                485                 490                 495
Leu Ser Ile Thr Thr Asp Phe Ile Pro Ser Phe Arg Leu Val Ala Tyr
            500                 505                 510
Tyr Thr Leu Ile Gly Ala Ser Gly Gln Arg Glu Val Val Ala Asp Ser
        515                 520                 525
Val Trp Val Asp Val Lys Asp Ser Cys Val Gly Ser Leu Val Val Lys
    530                 535                 540
Ser Gly Gln Ser Glu Asp Arg Gln Pro Val Pro Gly Gln Gln Met Thr
545                 550                 555                 560
Leu Lys Ile Glu Gly Asp His Gly Ala Arg Val Val Leu Val Ala Val
                565                 570                 575
Asp Lys Gly Val Phe Val Leu Asn Lys Lys Asn Lys Leu Thr Gln Ser
            580                 585                 590
Lys Ile Trp Asp Val Val Glu Lys Ala Asp Ile Gly Cys Thr Pro Gly
        595                 600                 605
Ser Gly Lys Asp Tyr Ala Gly Val Phe Ser Asp Ala Gly Leu Thr Phe
    610                 615                 620
Thr Ser Ser Ser Gly Gln Gln Thr Ala Gln Arg Ala Glu Leu Gln Cys
625                 630                 635                 640
Pro Gln Pro Ala Ala Ser Val Gln Leu Thr Glu Lys Arg Met Asp Lys
                645                 650                 655
Val Gly Lys Tyr Pro Lys Glu Leu Arg Lys Cys Cys Glu Asp Gly Met
            660                 665                 670
Arg Glu Asn Pro Met Arg Phe Ser Cys Gln Arg Arg Thr Arg Phe Ile
        675                 680                 685
Ser Leu Gly Glu Ala Cys Lys Lys Val Phe Leu Asp Cys Cys Asn Tyr
    690                 695                 700
Ile Thr Glu Leu Arg Arg Gln His Ala Arg Ala Ser His Leu Gly Leu
705                 710                 715                 720
Ala Arg Ser Asn Leu Asp Glu Asp Ile Ile Ala Glu Glu Asn Ile Val
                725                 730                 735
Ser Arg Ser Glu Phe Pro Glu Ser Trp Leu Trp Asn Val Glu Asp Leu
            740                 745                 750
Lys Glu Pro Pro Lys Asn Gly Ile Ser Thr Lys Leu Met Asn Ile Phe
        755                 760                 765
Leu Lys Asp Ser Ile Thr Thr Trp Glu Ile Leu Ala Val Ser Met Ser
    770                 775                 780
Asp Lys Lys Gly Ile Cys Val Ala Asp Pro Phe Glu Val Thr Val Met
785                 790                 795                 800
Gln Asp Phe Phe Ile Asp Leu Arg Leu Pro Tyr Ser Val Val Arg Asn
                805                 810                 815
Glu Gln Val Glu Ile Arg Ala Val Leu Tyr Asn Tyr Arg Gln Asn Gln
            820                 825                 830
Glu Leu Lys Val Arg Val Glu Leu Leu His Asn Pro Ala Phe Cys Ser
        835                 840                 845
Leu Ala Thr Thr Lys Arg Arg His Gln Gln Thr Val Thr Ile Pro Pro
    850                 855                 860
Lys Ser Ser Leu Ser Val Pro Tyr Val Ile Val Pro Leu Lys Thr Gly
865                 870                 875                 880
Leu Gln Glu Val Glu Val Lys Ala Ala Val Tyr His His Phe Ile Ser
                885                 890                 895
Asp Gly Val Arg Lys Ser Leu Lys Val Val Pro Glu Gly Ile Arg Met
            900                 905                 910
Asn Lys Thr Val Ala Val Arg Thr Leu Asp Pro Glu Arg Leu Gly Arg
        915                 920                 925
Glu Gly Val Gln Lys Glu Asp Ile Pro Pro Ala Asp Leu Ser Asp Gln
    930                 935                 940
```

Val Pro Asp Thr Glu Ser Glu Thr Arg Ile Leu Leu Gln Gly Thr Pro
945 950 955 960
Val Ala Gln Met Thr Glu Asp Ala Val Asp Ala Glu Arg Leu Lys His
965 970 975
Leu Ile Val Thr Pro Ser Gly Cys Gly Glu Gln Asn Met Ile Gly Met
980 985 990
Thr Pro Thr Val Ile Ala Val His Tyr Leu Asp Glu Thr Glu Gln Trp
995 1000 1005
Glu Lys Phe Gly Leu Glu Lys Arg Gln Gly Ala Leu Glu Leu Ile Lys
1010 1015 1020
Lys Gly Tyr Thr Gln Gln Leu Ala Phe Arg Gln Pro Ser Ser Ala Phe
1025 1030 1035 1040
Ala Ala Phe Val Lys Arg Ala Pro Ser Thr Trp Leu Thr Ala Tyr Val
1045 1050 1055
Val Lys Val Phe Ser Leu Ala Val Asn Leu Ile Ala Ile Asp Ser Gln
1060 1065 1070
Val Leu Cys Gly Ala Val Lys Trp Leu Ile Leu Glu Lys Gln Lys Pro
1075 1080 1085
Asp Gly Val Phe Gln Glu Asp Ala Pro Val Ile His Gln Glu Met Ile
1090 1095 1100
Gly Gly Leu Arg Asn Asn Asn Glu Lys Asp Met Ala Leu Thr Ala Phe
1105 1110 1115 1120
Val Leu Ile Ser Leu Gln Glu Ala Lys Asp Ile Cys Glu Glu Gln Val
1125 1130 1135
Asn Ser Leu Pro Gly Ser Ile Thr Lys Ala Gly Asp Phe Leu Glu Ala
1140 1145 1150
Asn Tyr Met Asn Leu Gln Arg Ser Tyr Thr Val Ala Ile Ala Gly Tyr
1155 1160 1165
Ala Leu Ala Gln Met Gly Arg Leu Lys Gly Pro Leu Leu Asn Lys Phe
1170 1175 1180
Leu Thr Thr Ala Lys Asp Lys Asn Arg Trp Glu Asp Pro Gly Lys Gln
1185 1190 1195 1200
Leu Tyr Asn Val Glu Ala Thr Ser Tyr Ala Leu Leu Ala Leu Leu Gln
1205 1210 1215
Leu Lys Asp Phe Asp Phe Val Pro Pro Val Val Arg Trp Leu Asn Glu
1220 1225 1230
Gln Arg Tyr Tyr Gly Gly Gly Tyr Gly Ser Thr Gln Ala Thr Phe Met
1235 1240 1245
Val Phe Gln Ala Leu Ala Gln Tyr Gln Lys Asp Ala Pro Asp His Gln
1250 1255 1260
Glu Leu Asn Leu Asp Val Ser Leu Gln Leu Pro Ser Arg Ser Ser Lys
1265 1270 1275 1280
Ile Thr His Arg Ile His Trp Glu Ser Ala Ser Leu Leu Arg Ser Glu
1285 1290 1295
Glu Thr Lys Glu Asn Glu Gly Phe Thr Val Thr Ala Glu Gly Lys Gly
1300 1305 1310
Gln Gly Thr Leu Ser Val Val Thr Met Tyr His Ala Lys Ala Lys Asp
1315 1320 1325
Gln Leu Thr Cys Asn Lys Phe Asp Leu Lys Val Thr Ile Lys Pro Ala
1330 1335 1340
Pro Glu Thr Glu Lys Arg Pro Gln Asp Ala Lys Asn Thr Met Ile Leu
1345 1350 1355 1360
Glu Ile Cys Thr Arg Tyr Arg Gly Asp Gln Asp Ala Thr Met Ser Ile
1365 1370 1375
Leu Asp Ile Ser Met Met Thr Gly Phe Ala Pro Asp Thr Asp Asp Leu
1380 1385 1390
Lys Gln Leu Ala Asn Gly Val Asp Arg Tyr Ile Ser Lys Tyr Glu Leu
1395 1400 1405
Asp Lys Ala Phe Ser Asp Arg Asn Thr Leu Ile Ile Tyr Leu Asp Lys
1410 1415 1420
Val Ser His Ser Glu Asp Asp Cys Leu Ala Phe Lys Val His Gln Tyr
1425 1430 1435 1440
Phe Asn Val Glu Leu Ile Gln Pro Gly Ala Val Lys Val Tyr Ala Tyr
1445 1450 1455
Tyr Asn Leu Glu Glu Ser Cys Thr Arg Phe Tyr His Pro Glu Lys Glu
1460 1465 1470
Asp Gly Lys Leu Asn Lys Leu Cys Arg Asp Glu Leu Cys Arg Cys Ala
1475 1480 1485

Glu Glu Asn Cys Phe Ile Gln Lys Ser Asp Asp Lys Val Thr Leu Glu
    1490                1495                1500
Glu Arg Leu Asp Lys Ala Cys Glu Pro Gly Val Asp Tyr Val Tyr Lys
1505                1510                1515                1520
Thr Lys Leu Leu Arg Ile Glu Glu Gln Asp Gly Asn Asp Ile Tyr Val
                1525                1530                1535
Met Asp Val Leu Glu Val Ile Lys Gln Gly Thr Asp Glu Asn Pro Arg
            1540                1545                1550
Ala Lys Thr His Gln Tyr Ile Ser Gln Arg Lys Cys Gln Glu Ala Leu
        1555                1560                1565
Asn Leu Lys Val Asn Asp Asp Tyr Leu Ile Trp Gly Ser Arg Ser Asp
    1570                1575                1580
Leu Leu Pro Thr Lys Asp Lys Ile Ser Tyr Ile Ile Thr Lys Asn Thr
1585                1590                1595                1600
Trp Ile Glu Arg Trp Pro His Glu Asp Glu Cys Gln Glu Glu Glu Phe
                1605                1610                1615
Gln Lys Leu Cys Asp Asp Phe Ala Gln Phe Ser Tyr Thr Leu Thr Glu
            1620                1625                1630
Phe Gly Cys Pro Thr
        1635

<210> 9
<211> 1637
<212> PRT
<213> Artificial Sequence

<220>
<221> SOURCE
<222> 1..1637
<223> /mol_type="protein"
      /note="Processed H5 construct"
      /organism="Artificial Sequence"

<400> 9
Ser Pro Met Tyr Ser Ile Ile Thr Pro Asn Ile Leu Arg Leu Glu Ser
1               5                   10                  15
Glu Glu Thr Met Val Leu Glu Ala His Asp Ala Gln Gly Asp Val Pro
            20                  25                  30
Val Thr Val Thr Val His Asp Phe Pro Gly Lys Lys Leu Val Leu Ser
        35                  40                  45
Ser Glu Lys Thr Val Leu Thr Pro Ala Thr Asn His Met Gly Asn Val
    50                  55                  60
Thr Phe Thr Ile Pro Ala Asn Arg Glu Phe Lys Ser Glu Lys Gly Arg
65                  70                  75                  80
Asn Lys Phe Val Thr Val Gln Ala Thr Phe Gly Thr Gln Val Val Glu
                85                  90                  95
Lys Val Val Leu Val Ser Leu Gln Ser Gly Tyr Leu Phe Ile Gln Thr
            100                 105                 110
Asp Lys Thr Ile Tyr Thr Pro Gly Ser Thr Val Leu Tyr Arg Ile Phe
        115                 120                 125
Thr Val Asn His Lys Leu Leu Pro Val Gly Arg Thr Val Met Val Asn
    130                 135                 140
Ile Glu Asn Pro Glu Gly Ile Pro Val Lys Gln Asp Ser Leu Ser Ser
145                 150                 155                 160
Gln Asn Gln Leu Gly Val Leu Pro Leu Ser Trp Asp Ile Pro Glu Leu
                165                 170                 175
Val Asn Met Gly Gln Trp Lys Ile Arg Ala Tyr Tyr Glu Asn Ser Pro
            180                 185                 190
Gln Gln Val Phe Ser Thr Glu Phe Glu Val Lys Glu Tyr Val Leu Pro
        195                 200                 205
Ser Phe Glu Val Ile Val Glu Pro Thr Glu Lys Phe Tyr Tyr Ile Tyr
    210                 215                 220
Asn Glu Lys Gly Leu Glu Val Thr Ile Thr Ala Arg Phe Leu Tyr Gly
225                 230                 235                 240
Lys Lys Val Glu Gly Thr Ala Phe Val Ile Phe Gly Ile Gln Asp Gly
                245                 250                 255
Glu Gln Arg Ile Ser Leu Pro Glu Ser Leu Lys Arg Ile Pro Ile Glu
            260                 265                 270

```
Asp Gly Ser Gly Glu Val Val Leu Ser Arg Lys Val Leu Leu Asp Gly
        275                 280                 285
Val Gln Asn Leu Arg Ala Glu Asp Leu Val Gly Lys Ser Leu Tyr Val
    290                 295                 300
Ser Ala Thr Val Ile Leu His Ser Gly Ser Asp Met Val Gln Ala Glu
305                 310                 315                 320
Arg Ser Gly Ile Pro Ile Val Thr Ser Pro Tyr Gln Ile His Phe Thr
                325                 330                 335
Lys Thr Pro Lys Tyr Phe Lys Pro Gly Met Pro Phe Asp Leu Met Val
            340                 345                 350
Phe Val Thr Asn Pro Asp Gly Ser Pro Ala Tyr Arg Val Pro Val Ala
        355                 360                 365
Val Gln Gly Glu Asp Thr Val Gln Ser Leu Thr Gln Gly Asp Gly Val
    370                 375                 380
Ala Lys Leu Ser Ile Asn Thr His Pro Ser Gln Lys Pro Leu Ser Ile
385                 390                 395                 400
Thr Val Arg Thr Lys Lys Gln Glu Leu Ser Glu Ala Glu Gln Ala Thr
                405                 410                 415
Arg Thr Met Gln Ala Leu Pro Tyr Ser Thr Val Gly Asn Ser Asn Asn
            420                 425                 430
Tyr Leu His Leu Ser Val Leu Arg Thr Glu Leu Arg Pro Gly Glu Thr
        435                 440                 445
Leu Asn Val Asn Phe Leu Leu Arg Met Asp Arg Ala His Glu Ala Lys
    450                 455                 460
Ile Arg Tyr Tyr Thr Tyr Leu Ile Met Asn Lys Gly Arg Leu Leu Lys
465                 470                 475                 480
Ala Gly Arg Gln Val Arg Glu Pro Gly Gln Asp Leu Val Val Leu Pro
                485                 490                 495
Leu Ser Ile Thr Thr Asp Phe Ile Pro Ser Phe Arg Leu Val Ala Tyr
            500                 505                 510
Tyr Thr Leu Ile Gly Ala Ser Gly Gln Arg Glu Val Val Ala Asp Ser
        515                 520                 525
Val Trp Val Asp Val Lys Asp Ser Cys Val Gly Ser Leu Val Val Lys
    530                 535                 540
Ser Gly Gln Ser Glu Asp Arg Gln Pro Val Pro Gly Gln Gln Met Thr
545                 550                 555                 560
Leu Lys Ile Glu Gly Asp His Gly Ala Arg Val Val Leu Val Ala Val
                565                 570                 575
Asp Lys Gly Val Phe Val Leu Asn Lys Lys Asn Lys Leu Thr Gln Ser
            580                 585                 590
Lys Ile Trp Asp Val Val Glu Lys Ala Asp Ile Gly Cys Thr Pro Gly
        595                 600                 605
Ser Gly Lys Asp Tyr Ala Gly Val Phe Ser Asp Ala Gly Leu Thr Phe
    610                 615                 620
Thr Ser Ser Ser Gly Gln Gln Thr Ala Gln Arg Ala Glu Leu Gln Cys
625                 630                 635                 640
Pro Gln Pro Ala Ala Ser Val Gln Leu Thr Glu Lys Arg Met Asp Lys
                645                 650                 655
Val Gly Lys Tyr Pro Lys Glu Leu Arg Lys Cys Cys Glu Asp Gly Met
            660                 665                 670
Arg Glu Asn Pro Met Arg Phe Ser Cys Gln Arg Arg Thr Arg Phe Ile
        675                 680                 685
Ser Leu Gly Glu Ala Cys Lys Lys Val Phe Leu Asp Cys Cys Asn Tyr
    690                 695                 700
Ile Thr Glu Leu Arg Arg Gln His Ala Arg Ala Ser His Leu Gly Leu
705                 710                 715                 720
Ala Arg Ser Asn Leu Asp Glu Asp Ile Ile Ala Glu Glu Asn Ile Val
                725                 730                 735
Ser Arg Ser Glu Phe Pro Glu Ser Trp Leu Trp Asn Val Glu Asp Leu
            740                 745                 750
Lys Glu Pro Pro Lys Asn Gly Ile Ser Thr Lys Leu Met Asn Ile Phe
        755                 760                 765
Leu Lys Asp Ser Ile Thr Thr Trp Glu Ile Leu Ala Val Ser Met Ser
    770                 775                 780
Asp Lys Lys Gly Ile Cys Val Ala Asp Pro Phe Glu Val Thr Val Met
785                 790                 795                 800
Gln Asp Phe Phe Ile Asp Leu Arg Leu Pro Tyr Ser Val Val Arg Asn
                805                 810                 815
```

```
Glu Gln Val Glu Ile Arg Ala Val Leu Tyr Asn Tyr Arg Gln Asn Gln
            820                 825                 830
Glu Leu Lys Val Arg Val Glu Leu Leu His Asn Pro Ala Phe Cys Ser
        835                 840                 845
Leu Ala Thr Thr Lys Arg Arg His Gln Gln Thr Val Thr Ile Pro Pro
    850                 855                 860
Lys Ser Ser Leu Ser Val Pro Tyr Val Ile Val Pro Leu Lys Thr Gly
865                 870                 875                 880
Leu Gln Glu Val Glu Val Lys Ala Ala Val Tyr His His Phe Ile Ser
                885                 890                 895
Asp Gly Val Arg Lys Ser Leu Lys Val Val Pro Glu Gly Ile Arg Met
            900                 905                 910
Asn Lys Thr Val Ala Val Arg Thr Leu Asp Pro Glu Arg Leu Gly Arg
        915                 920                 925
Glu Gly Val Gln Lys Glu Asp Ile Pro Pro Ala Asp Leu Ser Asp Gln
    930                 935                 940
Val Pro Asp Thr Glu Ser Glu Thr Arg Ile Leu Leu Gln Gly Thr Pro
945                 950                 955                 960
Val Ala Gln Met Thr Glu Asp Ala Val Asp Ala Glu Arg Leu Lys His
                965                 970                 975
Leu Ile Val Thr Pro Ser Gly Cys Gly Glu Gln Asn Met Ile Gly Met
            980                 985                 990
Thr Pro Thr Val Ile Ala Val His Tyr Leu Asp Glu Thr Glu Gln Trp
        995                 1000                1005
Glu Lys Phe Gly Leu Glu Lys Arg Gln Gly Ala Leu Glu Leu Ile Lys
    1010                1015                1020
Lys Gly Tyr Thr Gln Gln Leu Ala Phe Arg Gln Pro Ser Ser Ala Phe
1025                1030                1035                1040
Ala Ala Phe Val Lys Arg Ala Pro Ser Thr Trp Leu Thr Ala Tyr Val
                1045                1050                1055
Val Lys Val Phe Ser Leu Ala Val Asn Leu Ile Ala Ile Asp Ser Gln
            1060                1065                1070
Val Leu Cys Gly Ala Val Lys Trp Leu Ile Leu Glu Lys Gln Lys Pro
        1075                1080                1085
Asp Gly Val Phe Gln Glu Asp Ala Pro Val Ile His Gln Glu Met Ile
    1090                1095                1100
Gly Gly Leu Arg Asn Asn Asn Glu Lys Asp Met Ala Leu Thr Ala Phe
1105                1110                1115                1120
Val Leu Ile Ser Leu Gln Glu Ala Lys Asp Ile Cys Glu Glu Gln Val
                1125                1130                1135
Asn Ser Leu Pro Gly Ser Ile Thr Lys Ala Gly Asp Phe Leu Glu Ala
            1140                1145                1150
Asn Tyr Met Asn Leu Gln Arg Ser Tyr Thr Val Ala Ile Ala Gly Tyr
        1155                1160                1165
Ala Leu Ala Gln Met Gly Arg Leu Lys Gly Pro Leu Leu Asn Lys Phe
    1170                1175                1180
Leu Thr Thr Ala Lys Asp Lys Asn Arg Trp Glu Asp Pro Gly Lys Gln
1185                1190                1195                1200
Leu Tyr Asn Val Glu Ala Thr Ser Tyr Ala Leu Leu Ala Leu Leu Gln
                1205                1210                1215
Leu Lys Asp Phe Asp Phe Val Pro Pro Val Val Arg Trp Leu Asn Glu
            1220                1225                1230
Gln Arg Tyr Tyr Gly Gly Gly Tyr Gly Ser Thr Gln Ala Thr Phe Met
        1235                1240                1245
Val Phe Gln Ala Leu Ala Gln Tyr Gln Lys Asp Ala Pro Asp His Gln
    1250                1255                1260
Glu Leu Asn Leu Asp Val Ser Leu Gln Leu Pro Ser Arg Ser Ser Lys
1265                1270                1275                1280
Ile Thr His Arg Ile His Trp Glu Ser Ala Ser Leu Leu Arg Ser Glu
                1285                1290                1295
Glu Thr Lys Glu Asn Glu Gly Phe Thr Val Thr Ala Glu Gly Lys Gly
            1300                1305                1310
Gln Gly Thr Leu Ser Val Val Thr Met Tyr His Ala Lys Ala Lys Asp
        1315                1320                1325
Gln Leu Thr Cys Asn Lys Phe Asp Leu Lys Val Thr Ile Lys Pro Ala
    1330                1335                1340
Pro Glu Thr Glu Lys Arg Pro Gln Asp Ala Lys Asn Thr Met Ile Leu
1345                1350                1355                1360
```

```
Glu Ile Cys Thr Arg Tyr Leu Gly Glu Val Asp Ser Thr Met Thr Ile
                1365                1370                1375
Ile Asp Ile Ser Met Leu Thr Gly Phe Leu Pro Asp Ala Glu Asp Leu
            1380                1385                1390
Thr Arg Leu Ser Lys Gly Val Asp Arg Tyr Ile Ser Arg Tyr Glu Val
        1395                1400                1405
Asp Asn Asn Met Ala Gln Lys Val Ala Val Ile Ile Tyr Leu Asn Lys
    1410                1415                1420
Val Ser His Ser Glu Asp Glu Cys Leu His Phe Lys Ile Leu Lys His
1425                1430                1435                1440
Phe Glu Val Gly Phe Ile Gln Pro Gly Ser Val Lys Val Tyr Ser Tyr
                1445                1450                1455
Tyr Asn Leu Asp Glu Lys Cys Thr Lys Phe Tyr His Pro Asp Lys Gly
            1460                1465                1470
Thr Gly Leu Leu Asn Lys Ile Cys Ile Gly Asn Val Cys Arg Cys Ala
        1475                1480                1485
Gly Glu Thr Cys Ser Ser Leu Asn His Gln Glu Arg Ile Asp Val Pro
    1490                1495                1500
Leu Gln Ile Glu Lys Ala Cys Glu Thr Asn Val Asp Tyr Val Tyr Lys
1505                1510                1515                1520
Thr Lys Leu Leu Arg Ile Glu Glu Gln Asp Gly Asn Asp Ile Tyr Val
                1525                1530                1535
Met Asp Val Leu Glu Val Ile Lys Gln Gly Thr Asp Glu Asn Pro Arg
            1540                1545                1550
Ala Lys Thr His Gln Tyr Ile Ser Gln Arg Lys Cys Gln Glu Ala Leu
        1555                1560                1565
Asn Leu Lys Val Asn Asp Asp Tyr Leu Ile Trp Gly Ser Arg Ser Asp
    1570                1575                1580
Leu Leu Pro Thr Lys Asp Lys Ile Ser Tyr Ile Ile Thr Lys Asn Thr
1585                1590                1595                1600
Trp Ile Glu Arg Trp Pro His Glu Asp Glu Cys Gln Glu Glu Glu Phe
                1605                1610                1615
Gln Lys Leu Cys Asp Asp Phe Ala Gln Phe Ser Tyr Thr Leu Thr Glu
            1620                1625                1630
Phe Gly Cys Pro Thr
        1635
```